# EUROPEAN PATENT SPECIFICATION

(11) **EP 3 027 603 B1**
(45) Date of publication and mention of the grant of the patent: **18.07.2018**
(21) Application number: 14767126.7
(22) Date of filing: 24.07.2014
(51) Int. Cl.: C07D 401/14, C07D 413/14, A61K 31/454, A61K 31/497, A61K 31/506, A61K 31/519, A61K 31/5355, C07D 401/04, C07D 407/14, C07D 413/04, C07D 417/14, C07D 471/04, C07D 487/04, A61P 29/02, A61P 37/08

(54) **HETEROBICYCLOARYL RORC2 INHIBITORS AND METHODS OF USE THEREOF**
HETEROBICYCLOARYL-RORC2-INHIBITOREN UND VERFAHREN ZUR VERWENDUNG DAVON
INHIBITEURS DE RORC2 MÉTHODES D'UTILISATION ASSOCIÉES

(30) Priority: 02.08.2013 US 201361861709 P
(43) Date of publication of application: 08.06.2016
(73) Proprietor: Pfizer Inc., New York, NY 10017 (US)
(72) Inventor: BLINN, James Robert, O'Fallon, Missouri 63368 (US); FLICK, Andrew Christopher, Westbrook, Connecticut 06498 (US); WENNERSTÅL, Göran Mattias, SE-129 44 Hägersten (SE); JONES, Peter, Arlington, Massachusetts 02474 (US); KAILA, Neelu, Lexington, Massachusetts 02421 (US); KIEFER, James Richard Jr., Belmont, California 94002 (US); KURUMBAIL, Ravi G., East Lyme, Connecticut 06333 (US); MENTE, Scot Richard, Arlington, Massachusetts 02476 (US); MEYERS, Marvin Jay, Wentzville, Missouri 63385 (US); SCHNUTE, Mark Edward, Acton, Massachusetts 01720 (US); THORARENSEN, Atli, Stow, Massachusetts 01775 (US); XING, Li, Lexington, Massachusetts 02421 (US); ZAMARATSKI, Edouard, SE-753 13 Uppsala (SE); ZAPF, Christoph Wolfgang, Marlborough, Massachusetts 01752 (US)
(74) Representative: Pfizer
(86) International application number: PCT/IB2014/063383
(87) International publication number: WO 2015/015378

(56) References cited:
- EP-A1- 0 842 934
- EP-A2- 0 832 650
- WO-A1-2012/139775
- WO-A1-2013/064231
- WO-A1-2013/178362
- WO-A1-2014/026328
- WO-A2-2012/064744
- WO-A2-2013/171729
- US-A- 5 708 008
- US-A1- 2003 191 124
- SANDRA A FILLA ET AL: "Novel Potent 5-HT1F Receptor Agonists: Structure-Activity Studies of a Series of Substituted N [3-(1-Methyl-4-piperidinyl)-1H pyrrolo[3,2,b]pyridin-5-yl]amides", JOURNAL OF MEDICINAL CHEMISTRY, AMERICAN CHEMICAL SOCIETY, US, vol. 46, no. 14, 3 June 2003 (2003-06-03), pages 3060-3071, XP008134552, ISSN: 0022-2623, DOI: 10.1021/JM030020M [retrieved on 2003-06-03]
- FORBES I T ET AL: "CCR2B receptor antagonists: conversion of a weak HTS hit to a potent lead compound", BIOORGANIC & MEDICINAL CHEMISTRY LETTERS, PERGAMON, AMSTERDAM, NL, vol. 10, no. 16, 21 August 2000 (2000-08-21), pages 1803-1806, XP004216003, ISSN: 0960-894X, DOI: 10.1016/S0960-894X(00)00347-4
- ZHANG D ET AL: "Design, synthesis and evaluation of bicyclic benzamides as novel 5-HT1F receptor agonists", BIOORGANIC & MEDICINAL CHEMISTRY LETTERS, PERGAMON, AMSTERDAM, NL, vol. 14, no. 24, 20 December 2004 (2004-12-20), pages 6011-6016, XP004645145, ISSN: 0960-894X, DOI: 10.1016/J.BMCL.2004.09.079
- JENNIE A. HUTTON ET AL: "Structure-Based Design of Potent and Selective Leishmania N -Myristoyltransferase Inhibitors", JOURNAL OF MEDICINAL CHEMISTRY, vol. 57, no. 20, 23 October 2014 (2014-10-23), pages 8664-8670, XP055169348, ISSN: 0022-2623, DOI: 10.1021/jm5011397

## Description

### BACKGROUND OF THE INVENTION

Retinoid-related orphan receptors (ROR) are reported to have an important role in numerous biological processes. Scientific investigations relating to each of retinoid-related orphan receptors RORα, RORβ, and RORγ have been described in the literature. Continuing research in this field is spurred by the promise of developing new therapeutic agents to treat medical disorders associated with retinoid-related orphan receptor activity.

RORγ has been reported to be expressed in high concentration in various tissues, such as thymus, kidney, liver, muscle, and certain fat tissue. Two isoforms of RORγ have been identified and are referred to as γ1 and γ2 (also referred to as RORγt). Expression of the γ2 isoform has been reported to appear in, for example, double-positive thymocytes. Compounds capable of modulating RORyt activity are contemplated to provide a therapeutic benefit in the treatment of multiple medical disorders, including immune and inflammatory disorders.

Numerous immune and inflammatory disorders continue to afflict millions of patients worldwide. Significant advances have been made in treating these disorders. However, current therapies do not provide satisfactory results for all patients due to, for example, detrimental side effects or insufficient efficacy. Treatments for immune and inflammatory disorders vary depending on the particular medical disorder, and often involve use of immunosuppressive drugs. Surgery (*e.g.,* splenectomy), plasmapheresis, or radiation can be used in certain instances.

One exemplary immune disorder in need of better therapy is psoriasis. Psoriasis is a T cell-mediated inflammatory disease that affects approximately 2% to 3% of adults and has a substantial adverse impact on the quality of life for patients suffering from this disorder. Plaques resulting from psoriasis can be painful and are visually unappealing. Various therapeutics have been developed in an attempt to treat psoriasis. However, the traditional therapies for psoriasis often have toxic adverse effects.

WO2012/139775 discloses certain pyrrolo sulphonamides as modulators for the orphan nuclear receptor RORγ. WO2012064744 discloses certain heterocyclyl sulphonamides as inhibitors of RORγ.

Accordingly, a need exists for improved treatments for psoriasis as well as other immune and inflammatory disorders. The present invention addresses this need and provides other related advantages.

### SUMMARY

The present invention provides compounds and pharmaceutical compositions. There are also disclosed methods of inhibiting RORγ activity and/or reducing the amount of IL-17 in a subject, and methods of treating various medical disorders using such compounds. In particular, one aspect of the invention relates to compounds represented by Formula II: 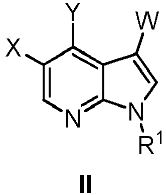 and pharmaceutically acceptable salts, and pharmaceutically acceptable solvates thereof; wherein R¹, X, Y and W are as defined in the Detailed Description.

There is also disclosed a method of treating a subject suffering from a medical disorder. The method comprises administering to the subject a therapeutically effective amount of a compound of Formula II or a pharmaceutically acceptable salt or solvate thereof, as described in the Detailed Description. A large number of disorders can be treated using the compounds described herein. For example, the compounds described herein can be used to treat an immune disorder or inflammatory disorder, such as rheumatoid arthritis, psoriasis, chronic graft- versus-host disease, acute graft-versus-host disease, Crohn's disease, inflammatory bowel disease, multiple sclerosis, systemic lupus erythematosus, Celiac Sprue, idiopathic thrombocytopenic thrombotic purpura, myasthenia gravis, Sjogren's syndrome, scleroderma, ulcerative colitis, asthma, epidermal hyperplasia, and other medical disorders described herein.

### DETAILED DESCRIPTION

The invention provides compounds, pharmaceutical compositions, and therapeutic uses of said compounds and pharmaceutical compositions. Also disclosed are methods of inhibiting RORγ activity and/or reducing the amount of IL-17 in a subject. The practice of the present invention employs, unless otherwise indicated, conventional techniques of organic chemistry, pharmacology, molecular biology (including recombinant techniques), cell biology, biochemistry, and immunology. Such techniques are explained in the literature, such as in "Comprehensive Organic Synthesis" (B.M. Trost & I. Fleming, eds., 1991-1992); "Handbook of experimental immunology" (D.M. Weir & C.C. Blackwell, eds.); "Current protocols in molecular biology" (F.M. Ausubel et al., eds., 1987, and periodic updates); and "Current protocols in immunology" (J.E. Coligan et al., eds., 1991).

Various aspects of the invention are set forth below in sections; however, aspects of the invention described in one particular section are not to be limited to any particular section. Further, when a variable is not accompanied by a definition, the previous definition of the variable controls.

### Definitions

It is to be understood that the foregoing general description and the following detailed description are exemplary and explanatory only and are not restrictive of any subject matter claimed. In this application, the use of the singular includes the plural unless specifically stated otherwise. It must be noted that, as used in the specification and the appended claims, the singular forms "a," "an" and "the" include plural referents unless the context clearly dictates otherwise. In this application, the use of "or" means "and/or" unless stated otherwise. Furthermore, use of the term "including" as well as other forms, such as "include", "includes," and "included," is not limiting.

It is to be understood that the methods and compositions described herein are not limited to the particular methodology, protocols, cell lines, constructs, and reagents described herein and as such may vary. It is also to be understood that the terminology used herein is for the purpose of describing particular embodiments only, and is not intended to limit the scope of the methods and compositions described herein, which will be limited only by the appended claims.

Chemical names, common names, and chemical structures may be used interchangeably to describe the same structure. If a chemical compound is referred to using both a chemical structure and a chemical name, and an ambiguity exists between the structure and the name, the structure predominates.

"ROR" stands for Retinoic acid receptor-Related Orphan Receptor. There are three forms of ROR, ROR-α, -β, and -γ and each is encoded by a separate gene (RORA, RORB, and RORC respectively). There are two subtypes of RORC: 1 and 2. Subtype 2 is also called "t". The human RORC gene is also called TOR; RORG; RZRG; NRIF3; and RZR-GAMMA. The human protein RORC is also called nuclear receptor ROR-gamma; nuclear receptor RZR-gamma; retinoic acid-binding receptor gamma; retinoid-related orphan receptor gamma; RAR-related orphan receptor C, isoform a; RAR-related orphan nuclear receptor variant 2; nuclear receptor subfamily 1 group F member 3. As used herein, "RORγ" and "RORC2" are used interchangeably to refer to a protein from a RORC subtype 2 gene.

As used herein, the term "modulator" refers to a compound that alters an activity of a molecule. For example, a modulator can cause an increase or decrease in the magnitude of a certain activity of a molecule compared to the magnitude of the activity in the absence of the modulator. In certain embodiments, a modulator is an inhibitor, which decreases the magnitude of one or more activities of a molecule. In certain embodiments, an inhibitor completely prevents one or more activities of a molecule. In certain embodiments, a modulator is an activator, which increases the magnitude of at least one activity of a molecule. In certain embodiments the presence of a modulator results in an activity that does not occur in the absence of the modulator.

The term "heteroatom" refers to an atom other than carbon or hydrogen. Heteroatoms are typically independently selected from among oxygen, sulfur, nitrogen, silicon and phosphorus, but are not limited to these atoms. In embodiments in which two or more heteroatoms are present, the two or more heteroatoms can all be the same as one another, or some or all of the two or more heteroatoms can each be different from the others.

The term "alkyl" refers to a linear or branched-chain saturated hydrocarbyl substituent (i.e., a substituent obtained from a hydrocarbon by removal of a hydrogen) containing from one to ten carbon atoms. Examples of such substituents include methyl, ethyl, propyl (including n-propyl and isopropyl), butyl (including n-butyl, isobutyl, sec-butyl and tert-butyl), pentyl, isoamyl, hexyl and the like. The terms "haloalkyl" and "haloalkoxy" include alkyl, and alkoxy structures, respectively, in which at least one hydrogen is replaced with a halogen atom. In certain embodiments in which two or more hydrogen atoms are replaced with halogen atoms, the halogen atoms are all the same as one another. In other embodiments in which two or more hydrogen atoms are replaced with halogen atoms, the halogen atoms are not all the same as one another.

The term "fluoroalkyl," as used herein, refers to alkyl group in which at least one hydrogen is replaced with a fluorine atom. Examples of fluoroalkyl groups include, but are not limited to, -CF₃, -CH₂CF₃, -CF₂CF₃, -CH₂CH₂CF₃ and the like.

The term "cycloalkyl" refers to a carbocyclic substituent obtained by removing a hydrogen from a saturated carbocyclic molecule and having three to ten carbon atoms. Cycloalkyl may be a single ring, which typically contains from 3 to 6 ring atoms. Examples of cycloalkyl include cyclopropyl, cyclobutyl, cyclopentyl and cyclohexyl. Alternatively, cycloalkyl may be 2 or 3 rings fused together, such as bicyclo[4.2.0]octane and decalinyl and may also be referred to as "bicycloalkyl".

The term "aryl" refers to an aromatic substituent containing one ring or two or three fused rings. The aryl substituent may have six to eighteen carbon atoms. As an example, the aryl substituent may have six to fourteen carbon atoms. The term "aryl" may refer to substituents such as phenyl, naphthalenyl, anthracenyl and phenanthrenyl.

In some instances, the number of atoms in a cyclic substituent containing one or more heteroatoms (i.e., heteroaryl or heterocycloalkyl) is indicated by the prefix "A-B membered", wherein A is the minimum and B is the maximum number of atoms forming the cyclic moiety of the substituent. Thus, for example, 5- to 8-membered heterocycloalkyl refers to a heterocycloalkyl containing from 5 to 8 atoms, including one or more heteroatoms, in the cyclic moiety of the heterocycloalkyl.

The term "hydroxy" or "hydroxyl" refers to OH.

The term "cyano" (also referred to as "nitrile") means CN.

The term "thio" means S.

The terms "halogen" and "halo" refer to fluorine (which may be depicted as F), chlorine (which may be depicted as Cl), bromine (which may be depicted as Br), or iodine (which may be depicted as I). In one embodiment, the halogen is chlorine. In another embodiment, the halogen is fluorine. In another embodiment, the halogen is bromine.

The terms "heterocycloalkyl" and "heterocyclyl" are used interchangeably and refer to a substituent obtained by removing a hydrogen from a saturated or partially saturated ring structure containing a total of 4 to 14 ring atoms in which at least one of the ring atoms is a heteroatom (i.e., oxygen, nitrogen, or sulfur), with the remaining ring atoms being independently selected from the group consisting of carbon, oxygen, nitrogen, and sulfur. For example, as used herein, the term "4- to 10-membered heterocycloalkyl" means the substituent is a single ring with 4 to 10 total members. A heterocycloalkyl alternatively may comprise 2 or 3 rings fused together, wherein at least one such ring contains a heteroatom as a ring atom (i.e., nitrogen, oxygen, or sulfur). In a group that has a heterocycloalkyl substituent, the ring atom of the heterocycloalkyl substituent that is bound to the group may be one of the heteroatoms, or it may be a ring carbon atom, where the ring carbon atom may be in the same ring as the heteroatom(s) or where the ring carbon atom may be in a different ring from the heteroatom(s). Similarly, if the heterocycloalkyl substituent is in turn substituted with a group or substituent, the group or substituent may be bound to the heteroatom(s), or it may be bound to a ring carbon atom, where the ring carbon atom may be in the same ring as the at least one heteroatom or where the ring carbon atom may be in a different ring from the heteroatom(s).

The term "heteroaryl" refers to a substituent obtained by removing a hydrogen from an aromatic ring structure containing from 5 to 14 ring atoms in which at least one of the ring atoms is a heteroatom (i.e., oxygen, nitrogen, or sulfur), with the remaining ring atoms being independently selected from the group consisting of carbon, oxygen, nitrogen, and sulfur. A heteroaryl may be a single ring or 2 or 3 fused rings. Examples of heteroaryl substituents include but are not limited to: 6-membered ring substituents such as pyridyl, pyrazyl, pyrimidinyl, and pyridazinyl; 5-membered ring substituents such as triazolyl, imidazolyl, furanyl, thiophenyl, pyrazolyl, oxazolyl, isoxazolyl, thiazolyl, 1,2,3-, 1,2,4-, 1,2,5-, or 1,3,4-oxadiazolyl and isothiazolyl; 6/5-membered fused ring substituents such as benzothiofuranyl, isobenzothiofuranyl, benzisoxazolyl, benzoxazolyl, purinyl, and anthranilyl; and 6/6-membered fused ring substituents such as quinolinyl, isoquinolinyl, cinnolinyl, quinazolinyl, and 1,4-benzoxazinyl. In a group that has a heteroaryl substituent, the ring atom of the heteroaryl substituent that is bound to the group may be the at least one heteroatom, or it may be a ring carbon atom, where the ring carbon atom may be in the same ring as the at least one heteroatom or where the ring carbon atom may be in a different ring from the at least one heteroatom. Similarly, if the heteroaryl substituent is in turn substituted with a group or substituent, the group or substituent may be bound to the heteroatom, or it may be bound to a ring carbon atom, where the ring carbon atom may be in the same ring as the heteroatom(s) or where the ring carbon atom may be in a different ring from the heteroatom(s). The term "heteroaryl" also includes pyridyl N-oxides and groups containing a pyridine N-oxide ring.

This specification uses the terms "substituent," "radical," and "group" interchangeably.

If a group of substituents are collectively described as being optionally substituted by one or more of a list of substituents, the group may include: (1) unsubstitutable substituents, (2) substitutable substituents that are not substituted by the optional substituents, and/or (3) substitutable substituents that are substituted by one or more of the optional substituents.

If a substituent is described such that it "may be substituted" or as being optionally substituted with up to a particular number of non-hydrogen substituents, that substituent may be either (1) not substituted; or (2) substituted by up to that particular number of non-hydrogen substituents or by up to the maximum number of substitutable positions on the substituent, whichever is less. Thus, for example, if a substituent is described as a heteroaryl optionally substituted with up to 3 non-hydrogen substituents, then any heteroaryl with less than 3 substitutable positions would be optionally substituted by up to only as many non-hydrogen substituents as the heteroaryl has substitutable positions. To illustrate, tetrazolyl (which has only one substitutable position) would be optionally substituted with up to one non-hydrogen substituent. To illustrate further, if an amino nitrogen is described as being optionally substituted with up to 2 non-hydrogen substituents, then the nitrogen will be optionally substituted with up to 2 non-hydrogen substituents if the amino nitrogen is a primary nitrogen, whereas the amino nitrogen will be optionally substituted with up to only 1 non-hydrogen substituent if the amino nitrogen is a secondary nitrogen.

A prefix attached to a multi-moiety substituent only applies to the first moiety. To illustrate, the term "alkylcycloalkyl" contains two moieties: alkyl and cycloalkyl. Thus, a (C₁-C₆) prefix on (C₁-C₆)alkyl(C₃-C₁₀)cycloalkyl means that the alkyl moiety of the alkylcycloalkyl contains from 1 to 6 carbon atoms; the (C₁-C₆)-prefix does not describe the cycloalkyl moiety. To illustrate further, the prefix "halo" on haloalkoxyalkyl indicates that only the alkoxy moiety of the alkoxyalkyl substituent is substituted with one or more halogen substituents. If the halogen substitution only occurs on the alkyl moiety, the substituent would be described as "alkoxyhaloalkyl." If the halogen substitution occurs on both the alkyl moiety and the alkoxy moiety, the substituent would be described as "haloalkoxyhaloalkyl."

As used herein compounds of Formula II may be referred to as a "compound(s) of the invention." Such terms are also defined to include all forms of the Formula II including hydrates, solvates, isomers, crystalline and non-crystalline forms, isomorphs, polymorphs, and metabolites thereof. For example, the compounds of Formula II and pharmaceutically acceptable salts thereof, may exist in unsolvated and solvated forms. When the solvent or water is tightly bound, the complex will have a well-defined stoichiometry independent of humidity. When, however, the solvent or water is weakly bound, as in channel solvates and hygroscopic compounds, the water/solvent content will be dependent on humidity and drying conditions. In such cases, non-stoichiometry will be the norm.

A "metabolite" of a compound disclosed herein is a derivative of that compound that is formed when the compound is metabolized. The term "active metabolite" refers to a biologically active derivative of a compound that is formed when the compound is metabolized. The term "metabolized," as used herein, refers to the sum of the processes (including, but not limited to, hydrolysis reactions and reactions catalyzed by enzymes, such as, oxidation reactions) by which a particular substance is changed by an organism. Thus, enzymes may produce specific structural alterations to a compound. For example, cytochrome P450 catalyzes a variety of oxidative and reductive reactions while uridine diphosphate glucuronyl transferases catalyze the transfer of an activated glucuronic-acid molecule to aromatic alcohols, aliphatic alcohols, carboxylic acids, amines and free sulfhydryl groups. Further information on metabolism may be obtained from The Pharmacological Basis of Therapeutics, 9th Edition, McGraw-Hill (1996). Metabolites of the compounds disclosed herein can be identified either by administration of compounds to a host and analysis of tissue samples from the host, or by incubation of compounds with hepatic cells in vitro and analysis of the resulting compounds. Both methods are well known in the art. Metabolites of a compound may be formed by oxidative processes and correspond to the corresponding hydroxy-containing compound. Also disclosed is a compound that is metabolized to pharmacologically active metabolites.

Also disclosed are compounds described herein that could be prepared as prodrugs. A "prodrug" refers to an agent that is converted into the parent drug in vivo. Prodrugs are often useful because, in some situations, they may be easier to administer than the parent drug. They may, for instance, be bioavailable by oral administration whereas the parent is not. The prodrug may also have improved solubility in pharmaceutical compositions over the parent drug. An example, without limitation, of a prodrug would be a compound described herein, which is administered as an ester (the "prodrug") to facilitate transmittal across a cell membrane where water solubility is detrimental to mobility but which then is metabolically hydrolyzed to the carboxylic acid, the active entity, once inside the cell where water-solubility is beneficial. A further example of a prodrug might be a short peptide (polyaminoacid) bonded to an acid group where the peptide is metabolized to reveal the active moiety. Also disclosed is, upon in vivo administration, a prodrug that is chemically converted to the biologically, pharmaceutically or therapeutically active form of the compound. Also disclosed is a prodrug that is enzymatically metabolized by one or more steps or processes to the biologically, pharmaceutically or therapeutically active form of the compound. To produce a prodrug, a pharmaceutically active compound is modified such that the active compound will be regenerated upon in vivo administration. The prodrug can be designed to alter the metabolic stability or the transport characteristics of a drug, to mask side effects or toxicity, to improve the flavor of a drug or to alter other characteristics or properties of a drug. By virtue of knowledge of pharmacodynamic processes and drug metabolism in vivo, those of skill in this art, once a pharmaceutically active compound is known, can design prodrugs of the compound. (see, for example, Nogrady (1985) Medicinal Chemistry A Biochemical Approach, Oxford University Press, New York, pages 388-392; Silverman (1992), The Organic Chemistry of Drug Design and Drug Action, Academic Press, Inc., San Diego, pages 352-401, Saulnier et al., (1994), Bioorganic and Medicinal Chemistry Letters, Vol. 4, p. 1985).

Prodrug forms of the herein described compounds, wherein the prodrug is metabolized *in vivo* to produce a derivative as set forth herein, are also disclosed. In some cases, some of the herein-described compounds may be a prodrug for another derivative or active compound.

Prodrugs are often useful because, in some situations, they may be easier to administer than the parent drug. They may, for instance, be bioavailable by oral administration whereas the parent is not. The prodrug may also have improved solubility in pharmaceutical compositions over the parent drug. Prodrugs may be designed as reversible drug derivatives, for use as modifiers to enhance drug transport to site-specific tissues. The design of a prodrug may increase the effective water solubility. See, e.g., Fedorak et al., Am. J. Physiol., 269:G210-218 (1995); McLoed et al., Gastroenterol, 106:405-413 (1994); Hochhaus et al., Biomed. Chrom., 6:283-286 (1992); J. Larsen and H. Bundgaard, Int. J. Pharmaceutics, 37, 87 (1987); J. Larsen et al., Int. J. Pharmaceutics, 47, 103 (1988); Sinkula et al., J. Pharm. Sci., 64:181-210 (1975); T. Higuchi and V. Stella, Prodrugs as Novel Delivery Systems, Vol. 14 of the A.C.S. Symposium Series; and Edward B. Roche, Bioreversible Carriers in Drug Design, American Pharmaceutical Association and Pergamon Press, 1987.

The compounds of the invention may have asymmetric carbon atoms. The carbon-carbon bonds of the compounds of the invention may be depicted herein using a solid line, a solid wedge or a dotted wedge. The use of a solid line to depict bonds to asymmetric carbon atoms is meant to indicate that all possible stereoisomers (e.g. specific enantiomers, racemic mixtures, etc.) at that carbon atom are included. The use of either a solid or dotted wedge to depict bonds to asymmetric carbon atoms is meant to indicate that only the stereoisomer shown is meant to be included. It is possible that compounds of the invention may contain more than one asymmetric carbon atom. In those compounds, the use of a solid line to depict bonds to asymmetric carbon atoms is meant to indicate that all possible stereoisomers are meant to be included. For example, unless stated otherwise, it is intended that the compounds of the invention can exist as enantiomers and diastereomers or as racemates and mixtures thereof. The use of a solid line to depict bonds to one or more asymmetric carbon atoms in a compound of the invention and the use of a solid or dotted wedge to depict bonds to other asymmetric carbon atoms in the same compound is meant to indicate that a mixture of diastereomers is present.

Stereoisomers of compounds of the invention include cis and trans isomers, optical isomers such as R and S enantiomers, diastereomers, geometric isomers, rotational isomers, conformational isomers, and tautomers of the compounds of the invention, including compounds exhibiting more than one type of isomerism; and mixtures thereof (such as racemates and diastereomeric pairs). Also included are acid addition or base addition salts wherein the counterion is optically active, for example, D-lactate or L-lysine, or racemic, for example, DL-tartrate or DL-arginine.

When any racemate crystallizes, crystals of two different types are possible. The first type is the racemic compound (true racemate) referred to above wherein one homogeneous form of crystal is produced containing both enantiomers in equimolar amounts. The second type is the racemic mixture or conglomerate wherein two forms of crystal are produced in equimolar amounts each comprising a single enantiomer.

The present invention also includes isotopically-labeled compounds, which are identical to those recited in Formula II herein, but for the fact that one or more atoms are replaced by an atom having an atomic mass or mass number different from the atomic mass or mass number usually found in nature. Examples of isotopes that may be incorporated into compounds of the invention include isotopes of hydrogen, carbon, nitrogen, oxygen, phosphorus, fluorine and chlorine, such as, but not limited to, ²H, ³H, ¹³C, ¹⁴C, ¹⁵N, ¹⁸O ¹⁷O ³¹P, ³²P, ³⁵S, ¹⁸F, and ³⁶Cl. Certain isotopically-labeled compounds of Formula (II), for example those into which radioactive isotopes such as ³H and ¹⁴O are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased in vivo half-life or reduced dosage requirements and, hence, may be preferred in some circumstances. Isotopically-labeled compounds of the invention may generally be prepared by carrying out the procedures disclosed in the Schemes and/or in the Examples and Preparations below, by substituting an isotopically-labeled reagent for a non-isotopically-labeled reagent.

The compounds of this invention may be used in the form of salts derived from inorganic or organic acids. Depending on the particular compound, a salt of the compound may be advantageous due to one or more of the salt's physical properties, such as enhanced pharmaceutical stability in differing temperatures and humidities, or a desirable solubility in water or oil. In some instances, a salt of a compound also may be used as an aid in the isolation, purification, and/or resolution of the compound.

Where a salt is intended to be administered to a patient (as opposed to, for example, being used in an *in vitro* context), the salt preferably is pharmaceutically acceptable. The term "pharmaceutically acceptable salt" refers to a salt prepared by combining a compound of Formula (II) with an acid whose anion, or a base whose cation, is generally considered suitable for human consumption. Pharmaceutically acceptable salts are particularly useful as products of the methods of the present invention because of their greater aqueous solubility relative to the parent compound. For use in medicine, the salts of the compounds of this invention are non-toxic "pharmaceutically acceptable salts." Salts encompassed within the term "pharmaceutically acceptable salts" refer to non-toxic salts of the compounds of this invention which are generally prepared by reacting the free base with a suitable organic or inorganic acid.

Suitable pharmaceutically acceptable acid addition salts of the compounds of the present invention when possible include those derived from inorganic acids, such as hydrochloric, hydrobromic, hydrofluoric, boric, fluoroboric, phosphoric, metaphosphoric, nitric, carbonic, sulfonic, and sulfuric acids, and organic acids such as acetic, benzenesulfonic, benzoic, citric, ethanesulfonic, fumaric, gluconic, glycolic, isothionic, lactic, lactobionic, maleic, malic, methanesulfonic, trifluoromethanesulfonic, succinic, toluenesulfonic, tartaric, and trifluoroacetic acids. Suitable organic acids generally include but are not limited to aliphatic, cycloaliphatic, aromatic, araliphatic, heterocyclic, carboxylic, and sulfonic classes of organic acids.

Specific examples of suitable organic acids include but are not limited to acetate, trifluoroacetate, formate, propionate, succinate, glycolate, gluconate, digluconate, lactate, malate, tartaric acid, citrate, ascorbate, glucuronate, maleate, fumarate, pyruvate, aspartate, glutamate, benzoate, anthranilic acid, stearate, salicylate, p-hydroxybenzoate, phenylacetate, mandelate, embonate (pamoate), methanesulfonate, ethanesulfonate, benzenesulfonate, pantothenate, toluenesulfonate, 2-hydroxyethanesulfonate, sufanilate, cyclohexylaminosulfonate, algenic acid, β-hydroxybutyric acid, galactarate, galacturonate, adipate, alginate, butyrate, camphorate, camphorsulfonate, cyclopentanepropionate, dodecylsulfate, glycoheptanoate, glycerophosphate, heptanoate, hexanoate, nicotinate, 2-naphthalesulfonate, oxalate, palmoate, pectinate, 3-phenylpropionate, picrate, pivalate, thiocyanate, and undecanoate.

Furthermore, where the compounds of the invention carry an acidic moiety, suitable pharmaceutically acceptable salts thereof may include alkali metal salts, i.e., sodium or potassium salts; alkaline earth metal salts, e.g., calcium or magnesium salts; and salts formed with suitable organic ligands, e.g., quaternary ammonium salts. In another embodiment, base salts are formed from bases which form non-toxic salts, including aluminum, arginine, benzathine, choline, diethylamine, diolamine, glycine, lysine, meglumine, olamine, tromethamine and zinc salts.

Organic salts may be made from secondary, tertiary or quaternary amine salts, such as tromethamine, diethylamine, N,N'-benzylethylenediamine, chloroprocaine, choline, diethanolamine, ethylenediamine, meglumine (N-methylglucamine), and procaine. Basic nitrogen-containing groups may be quaternized with agents such as lower alkyl (C₁-C₆) halides (e.g., methyl, ethyl, propyl, and butyl chlorides, bromides, and iodides), dialkyl sulfates (i.e., dimethyl, diethyl, dibutyl, and diamyl sulfates), long chain halides (i.e., decyl, lauryl, myristyl, and stearyl chlorides, bromides, and iodides), arylalkyl halides (i.e., benzyl and phenethyl bromides), and others.

In one embodiment, hemisalts of acids and bases may also be formed, for example, hemisulphate and hemicalcium salts.

### Compounds

In the following description of compounds suitable for use in the methods described herein, definitions of referred-to standard chemistry terms may be found in reference works (if not otherwise defined herein), including Carey and Sundberg "Advanced Organic Chemistry 4th Ed." Vols. A (2000) and B (2001), Plenum Press, New York. Unless otherwise indicated, conventional methods of mass spectroscopy, NMR, HPLC, protein chemistry, biochemistry, recombinant DNA techniques and pharmacology, within the ordinary skill of the art are employed. Unless specific definitions are provided, the nomenclature employed in connection with, and the laboratory procedures and techniques of, analytical chemistry, synthetic organic chemistry, and medicinal and pharmaceutical chemistry described herein are those known in the art. Standard techniques can be used for chemical syntheses, chemical analyses, pharmaceutical preparation, formulation, and delivery, and treatment of patients.

In certain embodiments, the compounds of the invention described herein are selective for RORγ over RORα and/or RORβ.

Generally, an inhibitor compound of RORγ used in the methods described herein is identified or characterized in an *in vitro* assay, e.g., an acellular biochemical assay or a cellular functional assay. Such assays are useful to determine an in vitro IC₅₀ for said compounds. In some embodiments, the RORγ inhibitor compound used for the methods described herein inhibits RORγ activity with an *in vitro* IC₅₀ of less than 25 µM (e.g., less than 20 µM, less than 10 µM, less than 1 µM, less than 0.5 µM, less than 0.4 µM, less than 0.3 µM, less than 0.1, less than 0.08 µM, less than 0.06 µM, less than 0.05 µM, less than 0.04 µM, less than 0.03 µM, less than less than 0.02 µM, less than 0.01, less than 0.008 µM, less than 0.006 µM, less than 0.005 µM, less than 0.004 µM, less than 0.003 µM, less than less than 0.002 µM, less than 0.001, less than 0.00099 µM, less than 0.00098 µM, less than 0.00097 µM, less than 0.00096 µM, less than 0.00095 µM, less than 0.00094 µM, less than 0.00093 µM, less than 0.00092, or less than 0.00090 µM). In some embodiments, the RORγ inhibitor compound is a compound described in the Exemplification.

Described herein are compounds of Formula II. Also described herein are pharmaceutically acceptable salts, pharmaceutically acceptable solvates, pharmaceutically active metabolites, and pharmaceutically acceptable prodrugs of such compounds. Pharmaceutical compositions that include at least one such compound or a pharmaceutically acceptable salt, pharmaceutically acceptable solvate, pharmaceutically active metabolite or pharmaceutically acceptable prodrug of such compound, are provided. In some embodiments, when compounds disclosed herein contain an oxidizable nitrogen atom, the nitrogen atom can be converted to an N-oxide by methods well known in the art. In certain embodiments, isomers of compounds having a structure represented by Formula II are also provided. Also provided are chemically protected forms of compounds having a structure represented by Formulae II.

One aspect of the invention relates to a compound of Formula II: 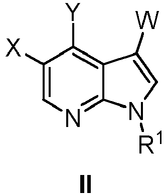 or a pharmaceutically acceptable salt, or pharmaceutically acceptable solvate thereof, wherein,
Y is hydrogen, halo, cyano, (C₁-C₃)alkyl, (C₁-C₃)hydroxyalkyl, (C₁-C₅)alkenyl, (C₁-C₃)haloalkyl, (C₁-C₃)hydroxyhaloalkyl, (C₃-C₆)cycloalkyl, (C₁-C₃)alkoxy or (C₁-C₃)haloalkoxy;
R¹ is hydrogen, (C₁-C₆)alkyl, (C₁-C₆)hydroxyalkyl or (C₁-C₆)haloalkyl;
X is 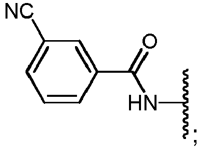
W is 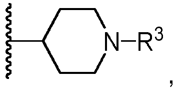 optionally substituted with one, two, three, four or five substitutents independently selected for each occurrence from the group consisting of halo, (C₁-C₆)alkyl, (C₁-C₆)hydroxyalkyl and (C₁-C₆)haloalkyl;
R³ is (C₁-C₆)alkyl, (C₃-C₈)cycloalkyl, (C₃-C₈)heterocycloalkyl, aryl, heteroaryl, -C(=O)R⁴, -C(=O)OR⁴, -C(=O)N(R⁵)₂ or -S(=O)₂R⁴, optionally substituted with one, two, three, four or five substitutents independently selected for each occurrence from the group consisting of halo, cyano, (C₁-C₄)alkyl, (C₁-C₄)haloalkyl, (C₁-C₄)cyanoalkyl, hydroxyl, (C₁-C₄)hydroxyalkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio, (C₁-C₄)haloalkylthio, -C(=O)R, -C(=O)OR, -OC(=O)R, -C(=O)NR₂, -N(R)C(=O)R, -CH₂C(=O )R, -CH₂C(=O)OR, -CH₂OC(=O)R, -CH₂C(=O)NR₂, -CH₂N(R)C(=O)R, -S(=O)₂R, -S(=O)₂N R₂, -N(R)S(=O)₂R, -A and -CH₂A;
R⁴ is hydrogen, (C₁-C₆)alkyl, (C₃-C₈)cycloalkyl, (C₃-C₈)heterocycloalkyl, (C₃-C₈)cycloalkyl(C₁-C₆)alkyl, (C₃-C₈)heterocycloalkyl(C₁-C₆)alkyl, aryl or heteroaryl, optionally substituted with one, two, three, four or five substitutents independently selected for each occurrence from the group consisting of halo, cyano, (C₁-C₄)alkyl, (C₁-C₄)haloalkyl, (C₁-C₄)cyanoalkyl, hydroxyl, (C₁-C₄)hydroxyalkyl, (C₁-C₄)alkoxy, (C₁-C₆)alkoxy(C₁-C₆)alkyl, (C₁-C₆)haloalkoxy, -NR₂, (R₂N)(C₁-C₆)alkyl, (C₁-C₆)alkylthio, (C₁-C₆)haloalkylthio, -C(=O)R, -C(=O)OR, -OC(=O)R, -C(=O)NR₂, -N(R)C(=O)R, -CH₂C(=O)R, -CH₂C(=O)OR, -CH₂OC(=O)R, -CH₂C(=O)NR₂, -CH₂N(R)C(=O)R, -S(=O)₂R, -S(=O)₂NR₂-, -N(R)S(=O)₂R, -A and -CH₂A;
R⁵ is independently selected for each occurrence from the group consisting of hydrogen, (C₁-C₆)alkyl, (C₃-C₈)cycloalkyl, (C₃-C₈)heterocycloalkyl, (C₃-C₈)cycloalkyl(C₁-C₆)alkyl, (C₃-C₈)heterocycloalkyl(C₁-C₆)alkyl, aryl and heteroaryl, optionally substituted with one, two, three, four or five substitutents independently selected for each occurrence from the group consisting of halo, cyano, (C₁-C₄)alkyl, (C₁-C₄)haloalkyl, (C₁-C₄)cyanoalkyl, hydroxyl, (C₁-C₄)hydroxyalkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio, (C₁-C₄)haloalkylthio, -C(=O)R, -C(=O)OR, -OC(=O)R, -C(=O)NR₂, -N(R)C(=O)R, -CH₂C(=O)R, -CH₂C(=O)OR, -CH₂OC(=O)R, -CH₂C(=O)NR₂, -CH₂N(R)C(=O)R, -S(=O)₂R, -S(=O)₂NR₂-, -N(R)S(=O)₂R, -A and -CH₂A; or where a nitrogen is substituted with two R⁵ groups may be taken together with the nitrogen atom to which they are attached to form a 4-, 5-, 6- or 7-membered saturated C₃₋₈heterocycloalkyl which, when so formed, may be optionally substituted with one, two or three substituents independently selected from the group consisting of (C₁-C₄)alkyl, halo, hydroxyl, (C₁-C₄)hydroxyalkyl, (C₁-C₄)haloalkyl and =O.
R is independently selected for each occurrence from the group consisting of hydrogen, (C₁-C₄)alkyl, (C₁-C₄)haloalkyl, (C₁-C₄)cyanoalkyl, (C₁-C₄)hydroxyalkyl, (C₃-C₈)cycloalkyl or (C₃-C₈)heterocycloalkyl; or where a nitrogen is substituted with two R groups they may be taken together with the nitrogen atom to which they are attached to form a 4-, 5-, 6- or 7-membered saturated (C₃-C₈)heterocycloalkyl which, when so formed, may be optionally substituted with one, two or three substituents independently selected from the group consisting of (C₁-C₄)alkyl, halo, hydroxyl, (C₁-C₄)hydroxyalkyl, (C₁-C₄)cyanoalkyl, (C₁-C₄)haloalkyl and =O; and
A is independently selected for each occurrence from the group consisting of (C₃-C₈)cycloalkyl, (C₃-C₈)heterocycloalkyl, aryl and heteroaryl, optionally substituted with one, two, three, four or five substitutents independently selected for each occurrence from the group consisting of halo, cyano, (C₁-C₄)alkyl, (C₁-C₄)haloalkyl, (C₁-C₄)cyanoalkyl, hydroxyl, (C₁-C₄)hydroxyalkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio, (C₁-C₄)haloalkylthio, -C(=O)R, -C(=O)OR, -OC(=O)R, -C(=O)NR₂, -N(R)C(=O)R, -CH₂C(=O)R, -CH₂C(=O)OR, -CH₂OC(=O)R, -CH₂C(=O)NR₂, -CH₂N(R)C(=O)R, -S(=O)₂R, -S(=O)₂NR₂ and -N(R)S(=O)₂R.

Also disclosed is a pharmaceutically active metabolite or pharmaceutically acceptable prodrug of a compound of Formula II.

In certain embodiments, the present invention relates to any of the aforementioned compounds, wherein R¹ is hydrogen. In certain embodiments, the present invention relates to any of the aforementioned compounds, wherein R¹ is (C₁-C₆)alkyl. In certain embodiments, the present invention relates to any of the aforementioned compounds, wherein R¹ is methyl.

In certain embodiments, the present invention relates to any of the aforementioned compounds, wherein Y is hydrogen. In certain embodiments, the present invention relates to any of the aforementioned compounds, wherein Y is (C₁-C₃)alkyl. In certain embodiments, the present invention relates to any of the aforementioned compounds, wherein Y is methyl.

In certain embodiments, the present invention relates to any of the aforementioned compounds, wherein W is 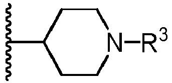 substituted with methyl. In certain embodiments, the present invention relates to any of the aforementioned compounds, wherein W is

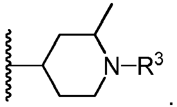

In certain embodiments, the present invention relates to any of the aforementioned compounds, wherein W is 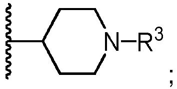 and R³ is -C(=O)R⁴. In certain embodiments, the present invention relates to any of the aforementioned compounds, wherein W is selected from the group consisting of 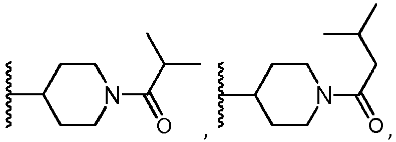 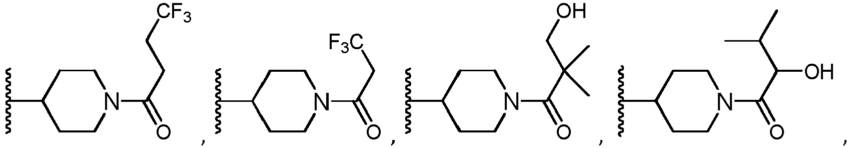 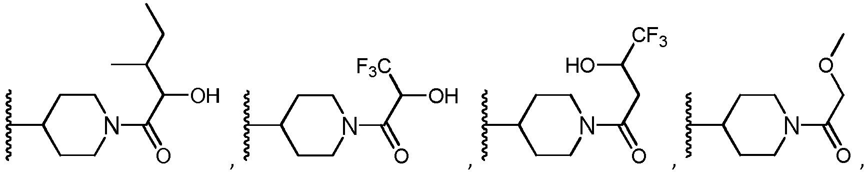 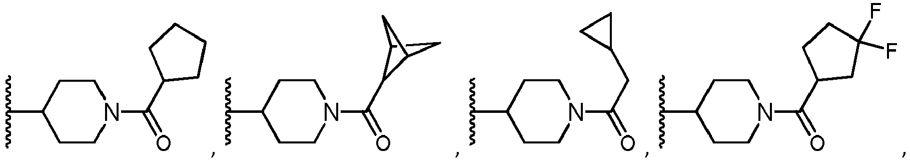 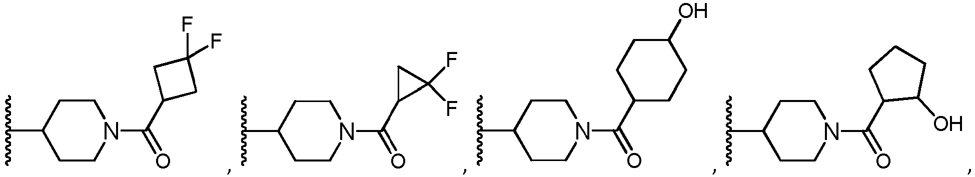 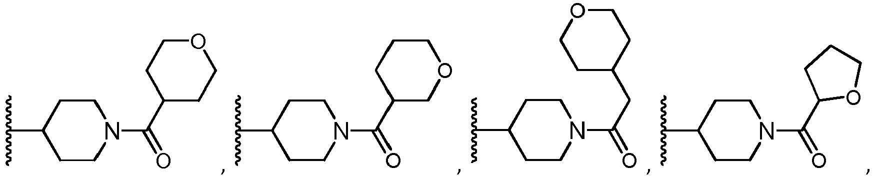 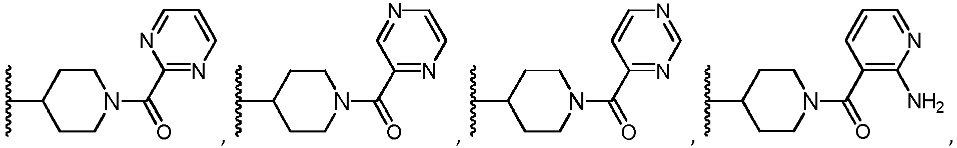 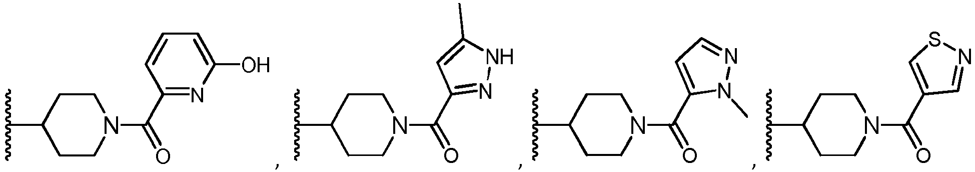 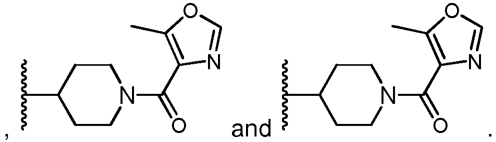 In certain embodiments, the present invention relates to any of the aforementioned compounds, wherein W is 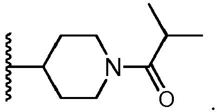 In certain embodiments, the present invention relates to any of the aforementioned compounds, wherein W is

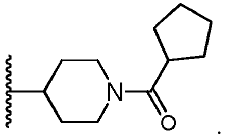

In certain embodiments, the present invention relates to any of the aforementioned compounds, wherein W is 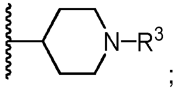 and R³ is -S(=O)₂R⁴.

In certain embodiments, the present invention relates to any of the aforementioned compounds, wherein R³ is aryl, optionally substituted with one, two, three, four or five substitutents independently selected for each occurrence from the group consisting of halo, cyano, (C₁-C₄)alkyl, (C₁-C₄)haloalkyl, hydroxyl, hydroxy(C₁-C₄)alkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio, (C₁-C₄)haloalkylthio, -C(=O)R, -C(=O)OR, -OC(=O)R, -C(=O)NR₂, -N(R)C(=O)R, -CH₂C(=O )R, -CH₂C(=O)OR, -CH₂OC(=O)R, -CH₂C(=O)NR₂, -CH₂N(R)C(=O)R, -S(=O)₂R, -S(=O)₂N R₂, -N(R)S(=O)₂R, -A and -CH₂A.

In certain embodiments, the present invention relates to any of the aforementioned compounds, wherein R³ is heteroaryl, optionally substituted with one, two, three, four or five substitutents independently selected for each occurrence from the group consisting of halo, cyano, (C₁-C₄)alkyl, (C₁-C₄)haloalkyl, hydroxyl, hydroxy(C₁-C₄)alkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio, (C₁-C₄)haloalkylthio, -C(=O)R, -C(=O)OR, -OC(=O)R, -C(=O)NR₂, -N(R)C(=O)R, -CH₂C(=O )R, -CH₂C(=O)OR, -CH₂OC(=O)R, -CH₂C(=O)NR₂, -CH₂N(R)C(=O)R, -S(=O)₂R, -S(=O)₂N R₂ and -N(R)S(=O)₂R, -A and -CH₂A.

In certain embodiments, the present invention relates to any of the aforementioned compounds, wherein R³ is pyrazoyl optionally substituted with one, two, three, four or five substitutents independently selected for each occurrence from the group consisting of halo, cyano, (C₁-C₄)alkyl, (C₁-C₄)haloalkyl, hydroxyl, hydroxy(C₁-C₄)alkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio, (C₁-C₄)haloalkylthio, -C(=O)R, -C(=O)OR, -OC(=O)R, -C(=O)NR₂, -N(R)C(=O)R, -CH₂C(=O )R, -CH₂C(=O)OR, -CH₂OC(=O)R, -CH₂C(=O)NR₂, -CH₂N(R)C(=O)R, -S(=O)₂R, -S(=O)₂N R₂, -N(R)S(=O)₂R, -A and -CH₂A.

In certain embodiments, the present invention relates to any of the aforementioned compounds, wherein R³ is pyrimidinyl optionally substituted with one, two, three, four or five substitutents independently selected for each occurrence from the group consisting of halo, cyano, (C₁-C₄)alkyl, (C₁-C₄)haloalkyl, hydroxyl, hydroxy(C₁-C₄)alkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio, (C₁-C₄)haloalkylthio, -C(=O)R, -C(=O)OR, -OC(=O)R, -C(=O)NR₂, -N(R)C(=O)R, -CH₂C(=O )R, -CH₂C(=O)OR, -CH₂OC(=O)R, -CH₂C(=O)NR₂, -CH₂N(R)C(=O)R, -S(=O)₂R, -S(=O)₂N R₂, -N(R)S(=O)₂R, -A and -CH₂A.

In certain embodiments, the present invention relates to any of the aforementioned compounds, wherein R³ is -C(=O)R⁴. In certain embodiments, the present invention relates to any of the aforementioned compounds, wherein R³ is -C(=O)OR⁴. In certain embodiments, the present invention relates to any of the aforementioned compounds, wherein R³ is -S(=O)₂R⁴

In certain embodiments, the present invention relates to any of the aforementioned compounds, wherein R³ is -C(=O)N(R⁵)₂; and R⁵ is independently selected for each occurrence from the group consisting of hydrogen, (C₁-C₆)alkyl, (C₃-C₈)cycloalkyl, (C₃-C₈)heterocycloalkyl, (C₃-C₈)cycloalkyl)(C₁-C₆)alkyl and (C₃-C₈)heterocycloalkyl)(C₁-C₆)alkyl, optionally substituted with one, two, three, four or five substitutents independently selected for each occurrence from the group consisting of halo, cyano, (C₁-C₄)alkyl, (C₁-C₄)haloalkyl, hydroxyl, (C₁-C₄)hydroxyalkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio, (C₁-C₄)haloalkylthio, -C(=O)R, -C(=O)OR, -OC(=O)R, -C(=O)NR₂, -N(R)C(=O)R, -CH₂C(=O)R, -CH₂C(=O)OR, -CH₂OC(=O)R, -CH₂C(=O)NR₂, -CH₂N(R)C(=O)R, -S(=O)₂R, -S(=O)₂NR₂-, -N(R)S(=O)₂R, -A and -CH₂A.

In certain embodiments, the present invention relates to any of the aforementioned compounds, wherein wherein R³ is -C(=O)N(R⁵)₂; and R⁵ is independently selected for each occurrence from the group consisting of hydrogen, aryl and heteroaryl, optionally substituted with one, two, three, four or five substitutents independently selected for each occurrence from the group consisting of halo, cyano, (C₁-C₄)alkyl, (C₁-C₄)haloalkyl, hydroxyl, (C₁-C₄)hydroxyalkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio,(C₁-C₄)haloalkylthio, -C(=O)R, -C(=O)OR, -OC(=O)R, -C(=O)NR₂, -N(R)C(=O)R, -CH₂C(=O)R, -CH₂C(=O)OR, -CH₂OC(=O)R, -CH₂C(=O)NR₂, -CH₂N(R)C(=O)R, -S(=O)₂R, -S(=O), -N(R) S(=O)₂R, -A and -CH₂A.

In certain embodiments, the present invention relates to any of the aforementioned compounds, wherein R³ is -C(=O)N(R⁵)₂; and the two R⁵ groups are taken together with the nitrogen atom to which they are attached to form a 4-, 5-, 6- or 7-membered saturated C₃₋₈heterocycloalkyl which is substituted with one, two or three substituents independently selected from the group consisting of (C₁-C₄)alkyl, halo, hydroxyl, (C₁-C₄)hydroxyalkyl, (C₁-C₄)haloalkyl and =O.

In certain embodiments, the present invention relates to any of the aforementioned compounds, wherein R⁴ is (C₁-C₆)alkyl, (C₃-C₈)cycloalkyl, (C₃-C₈)heterocycloalkyl, (C₃-C₈)cycloalkyl(C₁-C₆)alkyl or (C₃-C₈)heterocycloalkyl(C₁-C₆)alkyl, optionally substituted with one, two, three, four or five substitutents independently selected for each occurrence from the group consisting of halo, cyano, (C₁-C₄)alkyl, (C₁-C₄)haloalkyl, hydroxyl, (C₁-C₄)hydroxyalkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio, (C₁-C₄)haloalkylthio, -C(=O)R, -C(=O)OR, -OC(=O)R, -C(=O)NR₂, -N(R)C(=O)R, -CH₂C(=O)R, -CH₂C(=O)OR, -CH₂OC(=O)R, -CH₂C(=O)NR₂, -CH₂N(R)C(=O)R, -S(=O)₂R, -S(=O)₂NR₂-, -N(R)S(=O)₂R, -A and -CH₂A. In certain embodiments, the present invention relates to any of the aforementioned compounds, wherein R⁴ is (C₁-C₆)alkyl, (C₃-C₈)cycloalkyl, (C₃-C₈)heterocycloalkyl, (C₃-C₈)cycloalkyl(C₁-C₆)alkyl or (C₃-C₈)heterocycloalkyl(C₁-C₆)alkyl, substituted with cyano and further optionally substituted with an additional one, two, three or four substitutents independently selected for each occurrence from the group consisting of halo, cyano, (C₁-C₄)alkyl, (C₁-C₄)haloalkyl, hydroxyl, (C₁-C₄)hydroxyalkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio, (C₁-C₄)haloalkylthio, -C(=O)R, -C(=O)OR, -OC(=O)R, -C(=O)NR₂, -N(R)C(=O)R, -CH₂C(=O)R, -CH₂C(=O)OR, -CH₂OC(=O)R, -CH₂C(=O)NR₂, -CH₂N(R)C(=O)R, -S(=O)₂R, -S(=O)₂NR₂-, -N(R)S(=O)₂R, -A and -CH₂A.

In certain embodiments, the present invention relates to any of the aforementioned compounds, wherein R⁴ is aryl or heteroaryl, optionally substituted with one, two, three, four or five substitutents independently selected for each occurrence from the group consisting of halo, cyano, (C₁-C₄)alkyl, (C₁-C₄)haloalkyl, hydroxyl, (C₁-C₄)hydroxyalkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio, (C₁-C₄)haloalkylthio, -C(=O)R, -C(=O)OR, -OC(=O)R, -C(=O)NR₂, -N(R)C(=O)R, -CH₂C(=O)R, -CH₂C(=O)OR, -CH₂OC(=O)R, -CH₂C(=O)NR₂, -CH₂N(R)C(=O)R, -S(=O)₂R, -S(=O)₂NR₂-, -N(R)S(=O)₂R, -A and -CH₂A. In certain embodiments, the present invention relates to any of the aforementioned compounds, wherein R⁴ is aryl or heteroaryl, substituted with cyano and optionally further substituted with one, two, three or four substitutents independently selected for each occurrence from the group consisting of halo, cyano, (C₁-C₄)alkyl, (C₁-C₄)haloalkyl, hydroxyl, (C₁-C₄)hydroxyalkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio, (C₁-C₄)haloalkylthio, -C(=O)R, -C(=O)OR, -OC(=O)R, -C(=O)NR₂, -N(R)C(=O)R, -CH₂C(=O)R, -CH₂C(=O)OR, -CH₂OC(=O)R, -CH₂C(=O)NR₂, -CH₂N(R)C(=O)R, -S(=O)₂R, -S(=O)₂NR₂-, -N(R)S(=O)₂R, -A and -CH₂A.

In certain embodiments, the present invention relates to any of the aforementioned compounds, wherein R⁴ is phenyl, optionally substituted with one, two, three, four or five substitutents independently selected for each occurrence from the group consisting of halo, cyano, (C₁-C₄)alkyl, (C₁-C₄)haloalkyl, hydroxyl, (C₁-C₄)hydroxyalkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio, (C₁-C₄)haloalkylthio, -C(=O)R, -C(=O)OR, -OC(=O)R, -C(=O)NR₂, -N(R)C(=O)R, -CH₂C(=O)R, -CH₂C(=O)OR, -CH₂OC(=O)R, -CH₂C(=O)NR₂, -CH₂N(R)C(=O)R, -S(=O)₂R, -S(=O)₂NR₂ and -N(R)S(=O)₂R. In certain embodiments, the present invention relates to any of the aforementioned compounds, wherein R⁴ is phenyl, substituted with cyano and optionally further substituted with an additional one, two, three, four or five substitutents independently selected for each occurrence from the group consisting of halo, cyano, (C₁-C₄)alkyl, (C₁-C₄)haloalkyl, hydroxyl, (C₁-C₄)hydroxyalkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio, (C₁-C₄)haloalkylthio, -C(=O)R, -C(=O)OR, -OC(=O)R, -C(=O)NR₂, -N(R)C(=O)R, -CH₂C(=O)R, -CH₂C(=O)OR, -CH₂OC(=O)R, -CH₂C(=O)NR₂, -CH₂N(R)C(=O)R, -S(=O)₂R, -S(=O)₂NR₂ and -N(R)S(=O)₂R.

In certain embodiments, the present invention relates to any of the aforementioned compounds, wherein R⁴ is pyridyl, optionally substituted with one, two, three, four or five substitutents independently selected for each occurrence from the group consisting of halo, cyano, (C₁-C₄)alkyl, (C₁-C₄)haloalkyl, hydroxyl, (C₁-C₄)hydroxyalkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio, (C₁-C₄)haloalkylthio, -C(=O)R, -C(=O)OR, -OC(=O)R, -C(=O)NR₂, -N(R)C(=O)R, -CH₂C(=O)R, -CH₂C(=O)OR, -CH₂OC(=O)R, -CH₂C(=O)NR₂, -CH₂N(R)C(=O)R, -S(=O)₂R, -S(=O)₂NR₂ and -N(R)S(=O)₂R. In certain embodiments, the present invention relates to any of the aforementioned compounds, wherein R⁴ is pyridyl, substituted with phenyl and further optionally substituted with an additional one, two, three or four substitutents independently selected for each occurrence from the group consisting of halo, cyano, (C₁-C₄)alkyl, (C₁-C₄)haloalkyl, hydroxyl, (C₁-C₄)hydroxyalkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio, (C₁-C₄)haloalkylthio, -C(=O)R, -C(=O)OR, -OC(=O)R, -C(=O)NR₂, -N(R)C(=O)R, -CH₂C(=O)R, -CH₂C(=O)OR, -CH₂OC(=O)R, -CH₂C(=O)NR₂, -CH₂N(R)C(=O)R, -S(=O)₂R, -S(=O)₂NR₂ and -N(R)S(=O)₂R.

In certain embodiments, the present invention relates to any of the aforementioned compounds, wherein R is independently selected for each occurrence from the group consisting of hydrogen, (C₁-C₄)alkyl, (C₁-C₄)haloalkyl, (C₁-C₄)cyanoalkyl, (C₁-C₄)hydroxyalkyl, (C₃-C₈)cycloalkyl and (C₃-C₈)heterocycloalkyl. In certain embodiments, the present invention relates to any of the aforementioned compounds, wherein R is independently selected for each occurrence from the group consisting of hydrogen and (C₁-C₄)alkyl.

Another embodiment of the invention is a compound selected from the group consisting of the compounds of Examples 54, 94 and 95 and pharmaceutically acceptable salts thereof.

### Therapeutic Applications

It is contemplated that the compounds of Formula II provide therapeutic benefits to subjects suffering from an immune disorder or inflammatory disorder. Accordingly, there is disclosed a method of treating a disorder selected from the group consisting of an immune disorder or inflammatory disorder. The method comprises administering a therapeutically effective amount of a compound of Formula II to a subject in need thereof to ameliorate a symptom of the disorder, wherein Formula II is as described above. In certain embodiments, the particular compound of Formula II is a compound defined by one of the embodiments described above.

In certain embodiments, the disorder an immune disorder. In certain other embodiments, the disorder is an inflammatory disorder. In certain other embodiments, the disorder is an autoimmune disorder. In certain other embodiments, the disorder is rheumatoid arthritis, psoriasis, chronic graft-versus-host disease, acute graft-versus-host disease, Crohn's disease, inflammatory bowel disease, multiple sclerosis, systemic lupus erythematosus, Celiac Sprue, idiopathic thrombocytopenic thrombotic purpura, myasthenia gravis, Sjogren's syndrome, scleroderma, ulcerative colitis, asthma, or epidermal hyperplasia.

In certain other embodiments, the disorder is cartilage inflammation, bone degradation, arthritis, juvenile arthritis, juvenile rheumatoid arthritis, pauciarticular juvenile rheumatoid arthritis, polyarticular juvenile rheumatoid arthritis, systemic onset juvenile rheumatoid arthritis, juvenile ankylosing spondylitis, juvenile enteropathic arthritis, juvenile reactive arthritis, juvenile Reter's Syndrome, SEA Syndrome, juvenile dermatomyositis, juvenile psoriatic arthritis, juvenile scleroderma, juvenile systemic lupus erythematosus, juvenile vasculitis, pauciarticular rheumatoid arthritis, polyarticular rheumatoid arthritis, systemic onset rheumatoid arthritis, ankylosing spondylitis, enteropathic arthritis, reactive arthritis, Reter's Syndrome, dermatomyositis, psoriatic arthritis, vasculitis, myolitis, polymyolitis, dermatomyolitis, osteoarthritis, polyarteritis nodossa, Wegener's granulomatosis, arteritis, polymyalgia rheumatica, sarcoidosis, sclerosis, primary biliary sclerosis, sclerosing cholangitis, dermatitis, atopic dermatitis, atherosclerosis, Still's disease, chronic obstructive pulmonary disease, Guillain-Barre disease, Type I diabetes mellitus, Graves' disease, Addison's disease, Raynaud's phenomenon, autoimmune hepatitis, psoriatic epidermal hyperplasia, plaque psoriasis, guttate psoriasis, inverse psoriasis, pustular psoriasis, erythrodermic psoriasis, or an immune disorder associated with or arising from activity of pathogenic lymphocytes. In certain embodiments, the psoriasis is plaque psoriasis, guttate psoriasis, inverse psoriasis, pustular psoriasis, or erythrodermic psoriasis.

In certain other embodiments, the disorder is noninfectious uveitis, Behcet's disease or Vogt-Koyanagi-Harada syndrome.

There is also disclosed the use of a compound of Formula II in the manufacture of a medicament. In certain embodiments, the medicament is for treating a disorder described herein.

There is also disclosed the use of a compound of Formula II for treating a medical disorder, such a medical disorder described herein.

Further, it is contemplated that compounds of Formula II can inhibit the activity of RORγ. Accordingly, there is also disclsoed a method of inhibiting the activity of RORγ. The method comprises exposing a RORγ to an effective amount of a compound of Formula II, to inhibit said RORγ, wherein Formula II is as described above. In certain embodiments, the particular compound of Formula II is the compound defined by one of the embodiments described herein.

Further, it is contemplated that compounds of Formula II can reduce the amount of interleukin-17 (IL-17) in a subject. IL-17 is a cytokine that affects numerous biological functions, including inducing and mediating pro-inflammatory responses. Accordingly, there is also disclosed a method of reducing the amount of IL-17 in a subject. The method comprises administering to a subject an effective amount of a compound of Formula II, to reduce the amount of IL-17 in the subject, wherein Formula II is as described above. In certain embodiments, the particular compound of Formula II is the compound defined by one of the embodiments described herein.

In certain embodiments, the subject is a human. In certain embodiments, administering the compound reduces the amount of IL-17 produced by Th-17 cells in the subject. A change in the amount of IL-17 produced by, for example, Th-17 cells can be measured using procedures described in the literature, such as an ELISA assay or intracellular staining assay.

Further, it is contemplated that compounds of Formula II may inhibit the synthesis of IL-17 in a subject. Accordingly, there is also disclosed a method of inhibiting the synthesis IL-17 in a subject. The method comprises administering to a subject an effective amount of a compound of Formula II, to inhibit the synthesis IL-17 in the subject, wherein Formula II is as described above. In certain embodiments, the particular compound of Formula II is the compound defined by one of the embodiments described herein.

The description above describes multiple embodiments providing definitions for variables used herein. The application specifically contemplates all combinations of such variables.

### Combination Therapy

Another aspect of the invention provides for combination therapy. For example, the compounds of Formula II or their pharmaceutically acceptable salts may be used in combination with additional therapeutic agents to treat medical disorders, such as medical disorders associated with inappropriate IL-17 pathway activity. Exemplary additional therapeutic agents include, for example, (1) a TNF-a inhibitor; (2) a non-selective COX-I/COX-2 inhibitor; (3) a selective COX-2 inhibitor, such as celecoxib and rofecoxib; (4) other agents for treating inflammatory disease and autoimmune disease including, for example, methotrexate, leflunomide, sulfasalazine, azathioprine, penicillamine, bucillamine, actarit, mizoribine, lobenzarit, hydroxychloroquine, d-penicillamine, aurothiomalate, auranofin, parenteral gold, oral gold, cyclophosphamide, Lymphostat-B, a BAFF/ APRIL inhibitor, CTLA-4-Ig, or a mimetic of CTLA-4-Ig; (5) a leukotriene biosynthesis inhibitor, such as a 5-lipoxygenase (5-LO) inhibitor, or a 5-lipoxygenase activating protein (FLAP) antagonist; (6) a LTD4 receptor antagonist; (7) a phosphodiesterase type IV (PDE-IV) inhibitor, such as cilomilast (ariflo) or roflumilast; (8) an antihistamine HI receptor antagonist; (9) an od- and oc2-adrenoceptor agonist; (10) an anticholinergic agent; (11) a β-adrenoceptor agonist; (12) an insulin-like growth factor type I (IGF-1) mimetic; (13) a glucocorticosoid; (14) a kinase inhibitor such as an inhibitor of a Janus Kinase (e.g., JAK 1 and/or JAK2 and/or JAK 3 and/or TYK2), p38 MAPK, Syk or IKK2; (15) a B-cell target biologic such as rituximab; (16) a selective co-stimulation modulator such as abatacept; (17) an interleukin inhibitor or interleukin receptor inhibitor, such as the IL-1 inhibitor anakinra, IL-6 inhibitor tocilizumab, and IL12/IL-23 inhibitor ustekimumab; (18) an anti-IL17 antibody, anti-IL21 antibody, or anti-IL22 antibody (19) a S1P1 agonist, such as fingolimod; (20) an interferon, such as interferon beta 1; (21) an integrin inhibitor such as natalizumab; (22) a mTOR inhibitor such as rapamycin, cyclosporin and tacrolimus; (23) a non-steroidal antiinflammatory agent (NSAID), such as propionic acid derivatives (alminoprofen, benoxaprofen, bucloxic acid, carprofen, fenbufen, fenoprofen, fluprofen, flurbiprofen, ibuprofen, indoprofen, ketoprofen, miroprofen, naproxen, oxaprozin, pirprofen, pranoprofen, suprofen, tiaprofenic acid, and tioxaprofen), acetic acid derivatives (indomethacin, acemetacin, alclofenac, clidanac, diclofenac, fenclofenac, fenclozic acid, fentiazac, furofenac, ibufenac, isoxepac, oxpinac, sulindac, tiopinac, tolmetin, zidometacin, and zomepirac), fenamic acid derivatives (flufenamic acid, meclofenamic acid, mefenamic acid, niflumic acid and tolfenamic acid), biphenylcarboxylic acid derivatives (diflunisal and flufenisal), oxicams (isoxicam, piroxicam, sudoxicam and tenoxican), salicylates (acetyl salicylic acid, sulfasalazine) and pyrazolones (apazone, bezpiperylon, feprazone, mofebutazone, oxyphenbutazone, phenylbutazone); (24) a NRF2 pathway activator, such as the fumaric acid derivative, BG-12; and (25) a chemokine or chemokine receptor inhibitor, such as a CCR9 antagonist.

In certain embodiments, the additional therapeutic agent is selected from the group consisting of Corticosteroids, Vitamin D3, Anthralin and Retinoids. In certain embodiments, the additional therapeutic agent is a Corticosteroid. In certain embodiments, the additional therapeutic agent is Vitamin D3. In certain embodiments, the additional therapeutic agent is Anthralin. In certain embodiments, the additional therapeutic agent is a Retinoids.

The amount of the compound of Formula II and additional therapeutic agent and the relative timing of administration may be selected in order to achieve a desired combined therapeutic effect. For example, when administering a combination therapy to a patient in need of such administration, the therapeutic agents in the combination, or a pharmaceutical composition or compositions comprising the therapeutic agents, may be administered in any order such as, for example, sequentially, concurrently, together, simultaneously and the like. Further, for example, a compound of any one of Formula II may be administered during a time when the additional therapeutic agent(s) exerts its prophylactic or therapeutic effect, or vice versa.

The doses and dosage regimen of the active ingredients used in the combination therapy may be determined by an attending clinician. In certain embodiments, the compound of Formula II and the additional therapeutic agent(s) are administered in doses commonly employed when such agents are used as monotherapy for treating the disorder. In other embodiments, the compound of Formula II and the additional therapeutic agent(s) are administered in doses lower than the doses commonly employed when such agents are used as monotherapy for treating the disorder. In certain embodiments, a compound of Formula II and the additional therapeutic agent(s) are present in the same composition, which is suitable for oral administration.

In certain embodiments, the compound of Formula II and the additional therapeutic agent(s) may act additively or synergistically. A synergistic combination may allow the use of lower dosages of one or more agents and/or less frequent administration of one or more agents of a combination therapy. A lower dosage or less frequent administration of one or more agents may lower toxicity of the therapy without reducing the efficacy of the therapy.

Another aspect of this invention is a kit comprising a therapeutically effective amount of a compound of any one of Formula II, a pharmaceutically acceptable carrier, vehicle or diluent, and optionally at least one additional therapeutic agent listed above.

### Pharmaceutical Compositions and Dosing Considerations

Typically, a compound of the invention is administered in an amount effective to treat a condition as described herein. The compounds of the invention are administered by any suitable route in the form of a pharmaceutical composition adapted to such a route, and in a dose effective for the treatment intended. Therapeutically effective doses of the compounds required to treat the progress of the medical condition are readily ascertained by one of ordinary skill in the art using preclinical and clinical approaches familiar to the medicinal arts. The term "therapeutically effective amount" as used herein refers to that amount of the compound being administered which will relieve to some extent one or more of the symptoms of the disorder being treated.

The term "treating", as used herein, unless otherwise indicated, means reversing, alleviating, inhibiting the progress of, or preventing the disorder or condition to which such term applies, or one or more symptoms of such disorder or condition. The term "treatment", as used herein, unless otherwise indicated, refers to the act of treating as "treating" is defined immediately above. The term "treating" also includes adjuvant and neo-adjuvant treatment of a subject.

As indicated above, the invention provides pharmaceutical compositions, which comprise a therapeutically-effective amount of one or more of the compounds described above, formulated together with one or more pharmaceutically acceptable carriers (additives) and/or diluents. The pharmaceutical compositions may be specially formulated for administration in solid or liquid form, including those adapted for the following: (1) oral administration, for example, drenches (aqueous or non-aqueous solutions or suspensions), tablets, e.g., those targeted for buccal, sublingual, and systemic absorption, boluses, powders, granules, pastes for application to the tongue; (2) parenteral administration, for example, by subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; or (8) nasally.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of one hundred per cent, this amount will range from about 0.1 per cent to about ninety-nine percent of active ingredient, preferably from about 5 percent to about 70 percent, most preferably from about 10 percent to about 30 percent.

In certain embodiments, a formulation of the present invention comprises an excipient selected from the group consisting of cyclodextrins, celluloses, liposomes, micelle forming agents, e.g., bile acids, and polymeric carriers, e.g., polyesters and polyanhydrides; and a compound of the present invention. In certain embodiments, an aforementioned formulation renders orally bioavailable a compound of the present invention.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In solid dosage forms of the invention for oral administration (capsules, tablets, pills, dragees, powders, granules, trouches and the like), the active ingredient is mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds and surfactants, such as poloxamer and sodium lauryl sulfate; (7) wetting agents, such as, for example, cetyl alcohol, glycerol monostearate, and non-ionic surfactants; (8) absorbents, such as kaolin and bentonite clay; (9) lubricants, such as talc, calcium stearate, magnesium stearate, solid polyethylene glycols, sodium lauryl sulfate, zinc stearate, sodium stearate, stearic acid, and mixtures thereof; (10) coloring agents; and (11) controlled release agents such as crospovidone or ethyl cellulose. In the case of capsules, tablets and pills, the pharmaceutical compositions may also comprise buffering agents. Solid compositions of a similar type may also be employed as fillers in soft and hard-shelled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be formulated for rapid release, e.g., freeze-dried. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations of the present invention which are suitable for vaginal administration also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically-acceptable carrier, and with any preservatives, buffers, or propellants which may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present invention to the body. Such dosage forms can be made by dissolving or dispersing the compound in the proper medium. Absorption enhancers can also be used to increase the flux of the compound across the skin. The rate of such flux can be controlled by either providing a rate controlling membrane or dispersing the compound in a polymer matrix or gel.

Ophthalmic formulations, eye ointments, powders, solutions and the like, are also contemplated as being within the scope of this invention. Formulations suitable for topical administration to the eye include, for example, eye drops wherein the compound of this invention is dissolved or suspended in a suitable carrier. A typical formulation suitable for ocular or aural administration may be in the form of drops of a micronised suspension or solution in isotonic, pH-adjusted, sterile saline. Other formulations suitable for ocular and aural administration include ointments, biodegradable (i.e., absorbable gel sponges, collagen) and non-biodegradable (i.e., silicone) implants, wafers, lenses and particulate or vesicular systems, such as niosomes or liposomes. A polymer such as crossed-linked polyacrylic acid, polyvinyl alcohol, hyaluronic acid, a cellulosic polymer, for example, hydroxypropylmethylcellulose, hydroxyethylcellulose, or methylcellulose, or a heteropolysaccharide polymer, for example, gelan gum, may be incorporated together with a preservative, such as benzalkonium chloride. Such formulations may also be delivered by iontophoresis.

For intranasal administration or administration by inhalation, the active compounds of the invention are conveniently delivered in the form of a solution or suspension from a pump spray container that is squeezed or pumped by the patient or as an aerosol spray presentation from a pressurized container or a nebulizer, with the use of a suitable propellant. Formulations suitable for intranasal administration are typically administered in the form of a dry powder (either alone; as a mixture, for example, in a dry blend with lactose; or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

Pharmaceutical compositions of this invention suitable for parenteral administration comprise one or more compounds of the invention in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon the subject compounds may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

Injectable depot forms are made by forming microencapsule matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly (anhydrides). Depot injectable formulations are also prepared by entrapping the drug in liposomes or microemulsions which are compatible with body tissue.

When the compounds of the present invention are administered as pharmaceuticals, to humans and animals, they can be given per se or as a pharmaceutical composition containing, for example, 0.1 to 99% (more preferably, 10 to 30%) of active ingredient in combination with a pharmaceutically acceptable carrier.

The preparations of the present invention may be given orally, parenterally, topically, or rectally. They are of course given in forms suitable for each administration route. For example, they are administered in tablets or capsule form, by injection, inhalation, eye lotion, ointment, suppository, etc. administration by injection, infusion or inhalation; topical by lotion or ointment; and rectal by suppositories. Oral administrations are preferred.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

These compounds may be administered to humans and other animals for therapy by any suitable route of administration, including orally, nasally, as by, for example, a spray, rectally, intravaginally, parenterally, intracisternally and topically, as by powders, ointments or drops, including buccally and sublingually.

Regardless of the route of administration selected, the compounds of the present invention, which may be used in a suitable hydrated form, and/or the pharmaceutical compositions of the present invention, are formulated into pharmaceutically-acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion or metabolism of the particular compound being employed, the rate and extent of absorption, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable daily dose of a compound of the invention will be that amount of the compound which is the lowest dose effective to produce a therapeutic effect. Such an effective dose will generally depend upon the factors described above. Preferably, the compounds are administered at about 0.01 mg/kg to about 200 mg/kg, more preferably at about 0.1 mg/kg to about 100 mg/kg, even more preferably at about 0.5 mg/kg to about 50 mg/kg.

When the compounds described herein are co-administered with another agent (e.g. , as sensitizing agents), the effective amount may be less than when the agent is used alone.

If desired, the effective daily dose of the active compound may be administered as two, three, four, five, six or more sub-doses administered separately at appropriate intervals throughout the day, optionally, in unit dosage forms. Preferred dosing is one administration per day.

The invention further provides a unit dosage form (such as a tablet or capsule) comprising a compound of Formula II or a specific compound described herein, or pharmaceutically acceptable salts thereof, in a therapeutically effective amount for the treatment of an immune or inflammatory disorder, such as one of the particular immune disorders or inflammatory disorders described herein.

### General Synthetic Schemes and Procedures

The compounds of Formula II may be prepared by the methods described below, together with synthetic methods known in the art of organic chemistry, or modifications and derivatizations that are familiar to those of ordinary skill in the art. The starting materials used herein are commercially available or may be prepared by routine methods known in the art (such as those methods disclosed in standard reference books such as the COMPENDIUM OF ORGANIC SYNTHETIC METHODS, Vol. I-VI (published by Wiley-Interscience)). Preferred methods include, but are not limited to, those described below.

During any of the following synthetic sequences it may be necessary and/or desirable to protect sensitive or reactive groups on any of the molecules concerned. This can be achieved by means of conventional protecting groups, such as those described in T. W. Greene, Protective Groups in Organic Chemistry, John Wiley & Sons, 1981; T. W. Greene and P. G. M. Wuts, Protective Groups in Organic Chemistry, John Wiley & Sons, 1991, and T. W. Greene and P. G. M. Wuts, Protective Groups in Organic Chemistry, John Wiley & Sons, 1999.

Compounds of Formula II, or their pharmaceutically acceptable salts, can be prepared according to the reaction Schemes discussed herein below. Unless otherwise indicated, the substituents in the Schemes are defined as above. Isolation and purification of the products is accomplished by standard procedures, which are known to a chemist of ordinary skill.

It will be understood by one skilled in the art that the various symbols, superscripts and subscripts used in the schemes, methods and examples are used for convenience of representation and/or to reflect the order in which they are introduced in the schemes, and are not intended to necessarily correspond to the symbols, superscripts or subscripts in the appended claims. The schemes are representative of methods useful in synthesizing the compounds of the present invention. They are not to constrain the scope of the invention in any way.

Compounds of Formula II may be prepared as single enantiomer or as a mixture of individual enantiomers which includes racemic mixtures. Methods to obtain preferentially a single enantiomer from a mixture of individual enantiomers or a racemic mixture are well known to those ordinarily skilled in the art of organic chemistry. Such methods include but are not limited to preferential crystallization of diastereomeric salts (e.g. tartrate or camphor sulfonate), covalent derivatization by a chiral, non-racemic reagent followed by separation of the resulting diastereomers by common methods (e.g. crystallization, chromatographic separation, or distillation) and chemical reversion to scalemic compound, Simulated Moving Bed technology, or high/medium-pressure liquid chromatography or supercritical fluid chromatography employing a chiral stationary phase. These techniques may be performed on the final compounds of the invention or on any intermediates to compounds of the invention which bear a stereogenic center. Also, to facilitate separation by any of the methods described above, the compounds of the invention or any intermediates to the compounds of the invention which bear a stereogenic center may be transiently reacted with an achiral reagent, separated, and then reverted to scalemic compound by standard synthetic techniques.

Compounds of formula (I) may be prepared as described in **Scheme A.** Condensation of 5-nitro-1H-indole (A-1, Y = H) with tert-butyl 4-oxopiperidine-1-carboxylate affords A-2. The indole nitrogen is then alkylated with alkyl halides of the formula R¹I or R¹Br in the presence of base to provide A-3. Hydrogenation of A-3 provides compounds of the formula A-4. The resulting amine A-4 may be transformed to amides by the reaction with acid chlorides in the presence of base or carboxylic acids with appropriate coupling agents or A-4 may be transformed to ureas by the reaction with a phosgene equivalent followed by an amine. Following deprotection of the nitrogen protecting group, intermediate A-6 may be transformed into compounds of the formula A-7 (Formula I) through common amine transformations including amide formation, sulphonamide formation, urea formation and reductive amination. Similarly, beginning with 4-methyl-5-nitro-1H-indole (A-1, Y = Me) and following similar steps provides compounds of the formula A-7 (Y = Me).

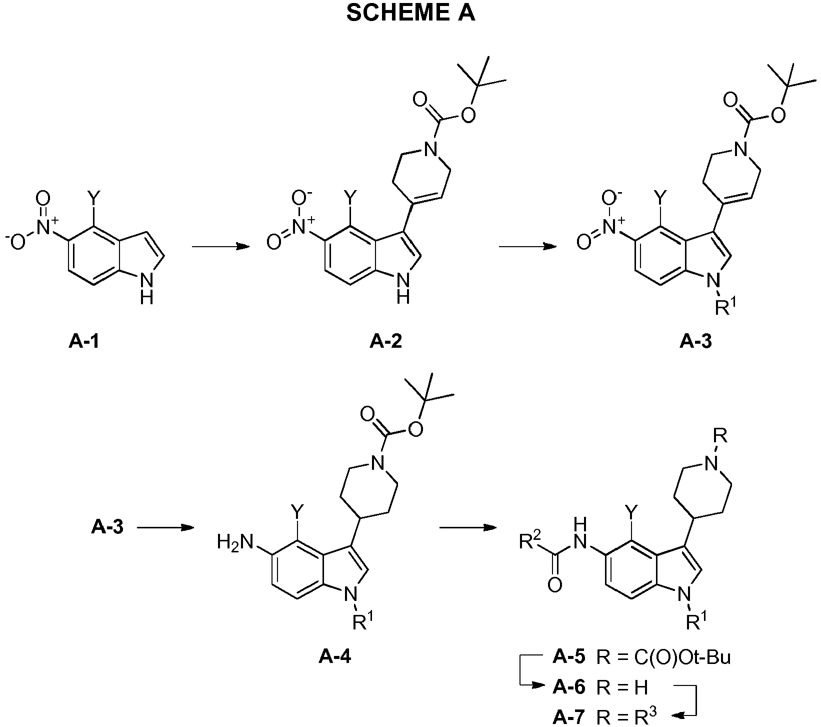

Alternatively, compounds of formula (I) may be prepared as described in **Scheme B.** Intermediate A-4 can be transformed to sulphonamides of the formula B-1 by reacting with sulfonyl chlorides in the presence of a base. Following deprotection of the nitrogen protecting group, intermediate B-2 may be transformed into compounds of the formula B-3 (Formula I) through common amine transformations including amide formation, sulphonamide formation, urea formation and reductive amination.

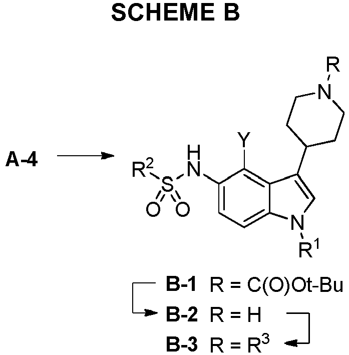

Alternatively, compounds of formula (I) may be prepared as described in **Scheme C.** Deprotection of the nitrogen protecting group in A-3 affords amine C-1 which is then transformed in to amides by the reaction with acid chlorides in the presence of base or carboxylic acids with appropriate coupling agents to provide compounds of the formula C-2. Hydrogenation of intermediate C-2 affords compounds of the formula C-3. The resulting amine C-3 may be transformed to compounds of formula (I) through common procedures for sulphonamide formation, amide formation or urea formation.

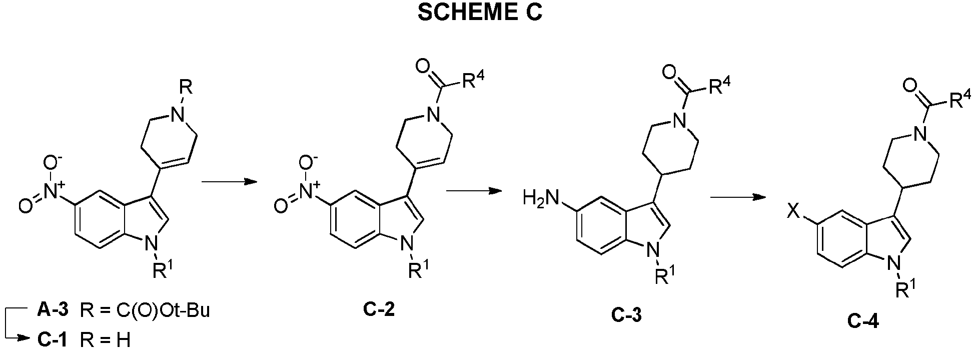

Alternatively, compounds of formula (I) may be prepared as described in **Scheme D.** Condensation of 5-nitro-1H-indole (A-1) with tert-butyl 3-oxopiperidine-1-carboxylate affords D-1. The indole nitrogen is then alkylated with alkyl halides of the formula R¹I or R¹Br in the presence of base to provide D-2. Hydrogenation of D-2 provides compounds of the formula D-3. The resulting amine D-3 may be transformed to amides (Z = CO) by the reaction with acid chlorides in the presence of base or carboxylic acids with appropriate coupling agents or D-3 may be transformed to ureas (Z = CO) by the reaction with a phosgene equivalent followed by an amine or D-3 may be transformed to sulfonamides (Z = SO₂) by the reaction with sulfonyl chlorides in the presence of a base. Following deprotection of the nitrogen protecting group, intermediate D-5 may be transformed into compounds of the formula D-6 (Formula I) through common amine transformations including amide formation, sulphonamide formation, urea formation and reductive amination.

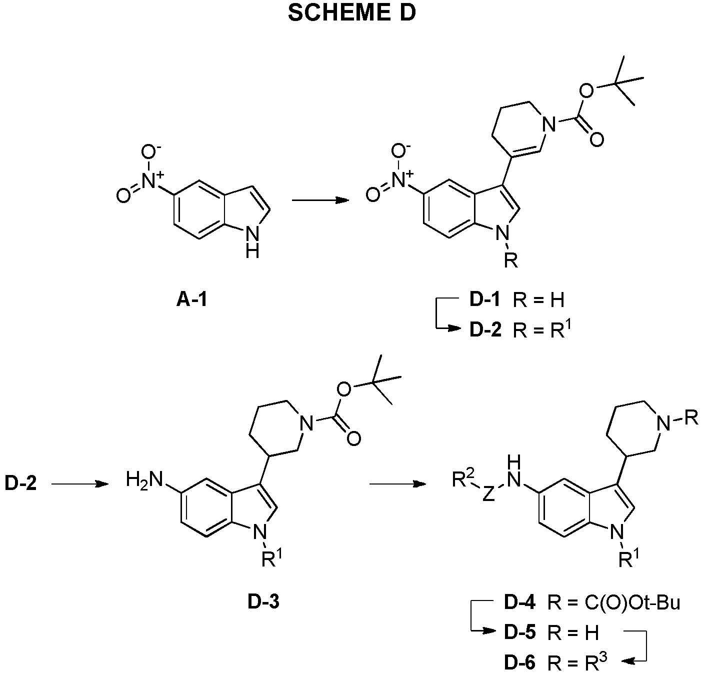

Alternatively, compounds of formula (I) may be prepared as described in **Scheme E.** 5-Nitro-1H-indole (A-1) is halogenated to afford bromide E-1. Palladium mediated coupling of E-1 with tert-butyl 3-(4,4,5,5-tetramethyl-1,3,2-dioxaborolan-2-yl)-1H-pyrrole-1-carboxylate provides E-2. The indole nitrogen is then alkylated with alkyl halides of the formula R¹I or R¹Br in the presence of base to provide compounds of the formula E-3. Hydrogenation of E-3 provides compounds of the formula E-4. The resulting amine E-4 may be transformed to amides (Z = CO) by the reaction with acid chlorides in the presence of base or carboxylic acids with appropriate coupling agents or E-4 may be transformed to ureas (Z = CO) by the reaction with a phosgene equivalent followed by an amine or E-4 may be transformed to sulfonamides (Z = SO₂) by the reaction with sulfonyl chlorides in the presence of a base. Following deprotection of the nitrogen protecting group, intermediate E-6 may be transformed into compounds of the formula E-7 (Formula I) through common amine transformations including amide formation, sulphonamide formation, urea formation and reductive amination. 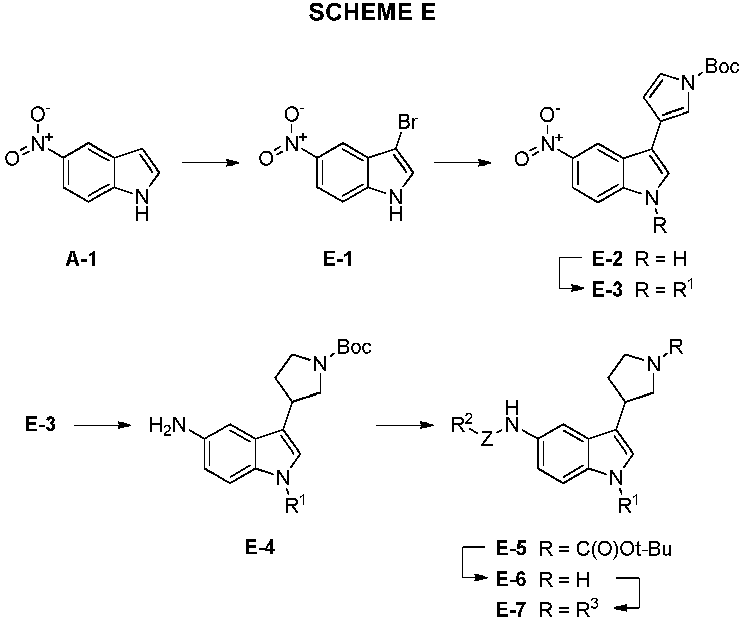 Compounds of formula (II) may be prepared as described in **Scheme F.** Iodination of 5-nitro-1H-pyrrolo[2,3-b]pyridine (F-1) followed by alkylation of the nitrogen by alkyl halides of the formula R¹I or R¹Br in the presence of base provides compounds of the formula F-2. Palladium mediated coupling of F-2 with tert-butyl 4-(4,4,5,5-tetramethyl-1,3,2-dioxaborolan-2-yl)-5,6-dihydropyridine-1(2H)-carboxylate provides compounds of the formula F-3. Hydrogenation of F-3 provides compounds of the formula F-4. The resulting amine F-4 may be transformed to amides (Z = CO) by the reaction with acid chlorides in the presence of base or carboxylic acids with appropriate coupling agents or F-4 may be transformed to ureas (Z = CO) by the reaction with a phosgene equivalent followed by an amine or F-4 may be transformed to sulfonamides (Z = SO₂) by the reaction with sulfonyl chlorides in the presence of a base. Following deprotection of the nitrogen protecting group, intermediate F-6 may be transformed into compounds of the formula F-7 through common amine transformations including amide formation, sulphonamide formation, urea formation and reductive amination. 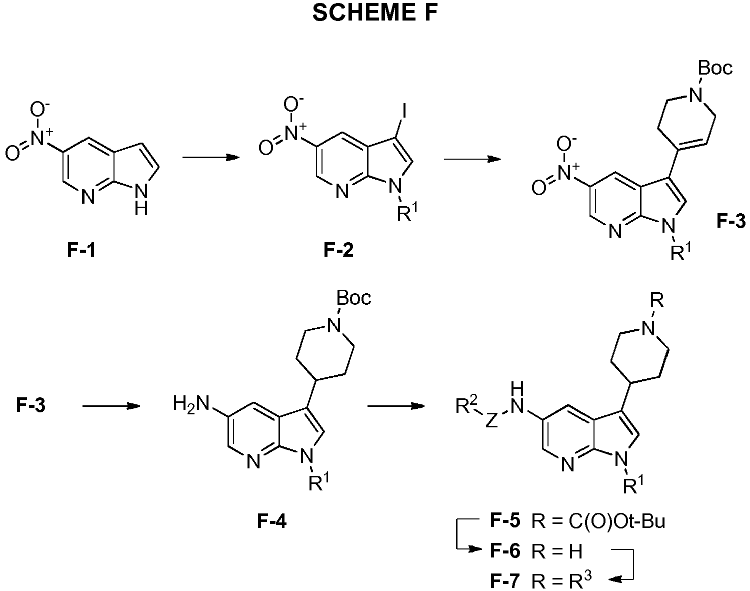 Compounds of formula (III) or formula (IV) may be prepared as described in **Scheme G.** Amine protection of intermediate G-1 (wherein X=N, Y=CH or X=CH, Y=N) followed by hydrogenation and ring closure-elimination provides compounds of the formula G-3. Iodination of G-3 provides compounds of the formula G-4 which are then alkylated by alkyl halides of the formula R¹I or R¹Br in the presence of base provides compounds of the formula G-5. Palladium mediated coupling of G-5 with 4-(4,4,5,5-tetramethyl-1,3,2-dioxaborolan-2-yl)-5,6-dihydropyridine-1(2H)-carboxylate amides provide compounds of the formula G-6. Hydrogenation of G-6 provides compounds of the formula G-7. The resulting amine G-7 may be transformed to amides (Z = CO) by the reaction with acid chlorides in the presence of base or carboxylic acids with appropriate coupling agents or G-7 may be transformed to ureas (Z = CO) by the reaction with a phosgene equivalent followed by an amine or G-7 may be transformed to sulfonamides (Z = SO₂) by the reaction with sulfonyl chlorides in the presence of a base to provide compounds of the formula G-8.

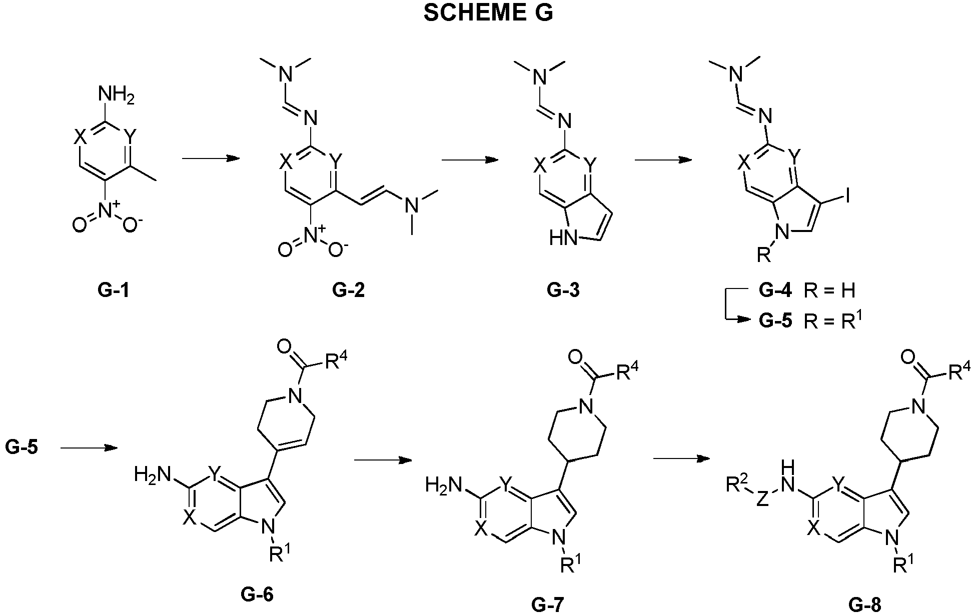

Compounds of formula (V) may be prepared as described in **Scheme H.** Reductive dehalogenation of 2,4-dichloro-5H-pyrrolo[3,2-d]pyrimidine (H-1) provides H-2 which is then iodinated to afford compound H-3. Alkylation of the nitrogen by alkyl halides of the formula R¹I or R¹Br in the presence of base provides compounds of the formula H-4. Palladium mediated coupling of H-4 with 4-(4,4,5,5-tetramethyl-1,3,2-dioxaborolan-2-yl)-5,6-dihydropyridine-1(2H)-carboxylate amides provide compounds of the formula H-5. Hydrogenation of H-5 provides compounds of the formula H-6. The resulting amine H-6 may be transformed to amides (Z = CO) by the reaction with acid chlorides in the presence of base or carboxylic acids with appropriate coupling agents or H-6 may be transformed to ureas (Z = CO) by the reaction with a phosgene equivalent followed by an amine or H-6 may be transformed to sulfonamides (Z = SO₂) by the reaction with sulfonyl chlorides in the presence of a base to provide compounds of the formula H-7. 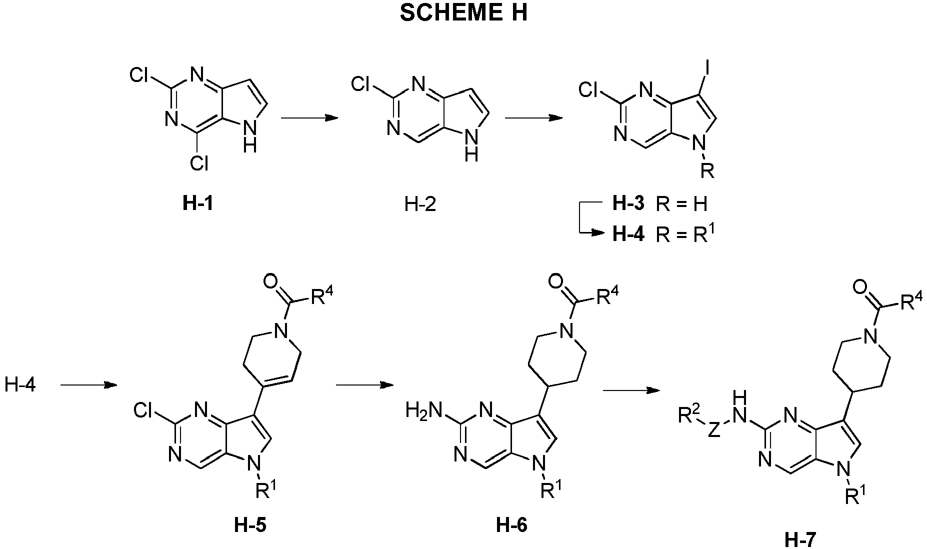 Compounds of formula (VI) may be prepared as described in **Scheme I.** The addition of lithiated 1-bromo-4-fluorobenzene to intermediate I-1 provides the ketone I-2. Condensation of I-2 with hydroxylamine provides hydroxyl imine I-3 which undergoes ring closure in the presence of base to provide isoxazole I-4. Palladium mediated amination of I-4 with an amine surrogate followed by deprotection of the nitrogen protecting group affords intermediate I-5. The resulting amine I-5 may be transformed to amides (Z = CO) by the reaction with acid chlorides in the presence of base or carboxylic acids with appropriate coupling agents or I-5 may be transformed to ureas (Z = CO) by the reaction with a phosgene equivalent followed by an amine or I-5 may be transformed to sulfonamides (Z = SO₂) by the reaction with sulfonyl chlorides in the presence of a base. Following deprotection of the nitrogen protecting group, intermediate I-7 may be transformed into compounds of the formula I-8 through common amine transformations including amide formation, sulphonamide formation, urea formation and reductive amination. 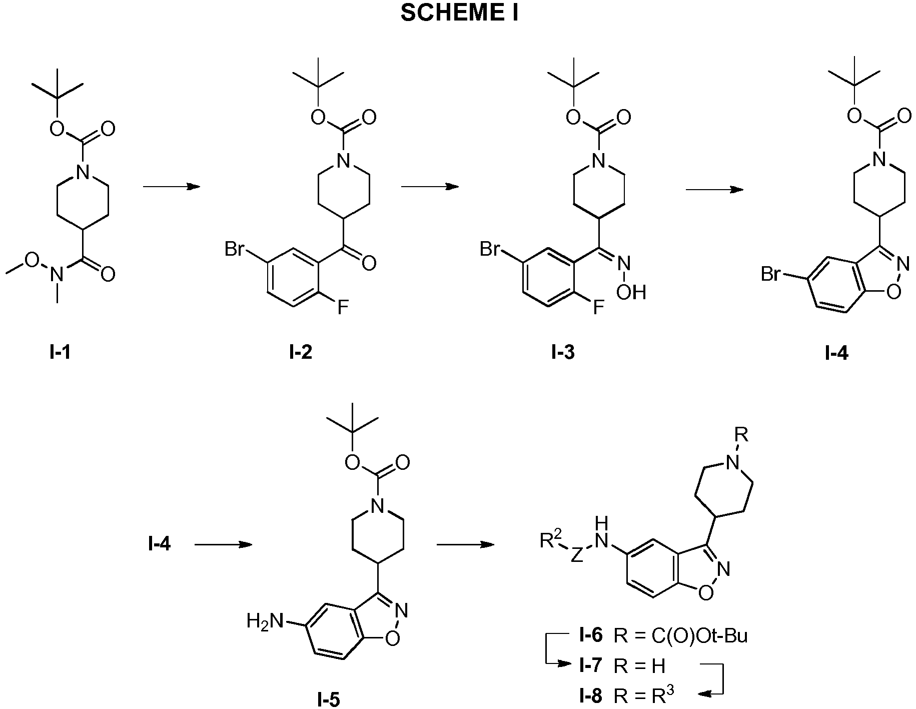 Compounds of formula (VII) may be prepared as described in **Scheme J.** Compounds such as 6-nitro-1H-indazole (J-1 wherein R¹ is H) may be alkylated with tert-butyl 4-((methylsulfonyl)oxy)piperidine-1-carboxylate in the presence of an inorganic base to provide compounds of the formula J-2. Hydrogenation of J-2 provides compounds of the formula J-3. The resulting amine J-3 may be transformed to amides (Z = CO) by the reaction with acid chlorides in the presence of base or carboxylic acids with appropriate coupling agents or J-3 may be transformed to ureas (Z = CO) by the reaction with a phosgene equivalent followed by an amine or J-3 may be transformed to sulfonamides (Z = SO₂) by the reaction with sulfonyl chlorides in the presence of a base. Following deprotection of the nitrogen protecting group, intermediate J-5 may be transformed into compounds of the formula J-6 through common amine transformations including amide formation, sulphonamide formation, urea formation and reductive amination. 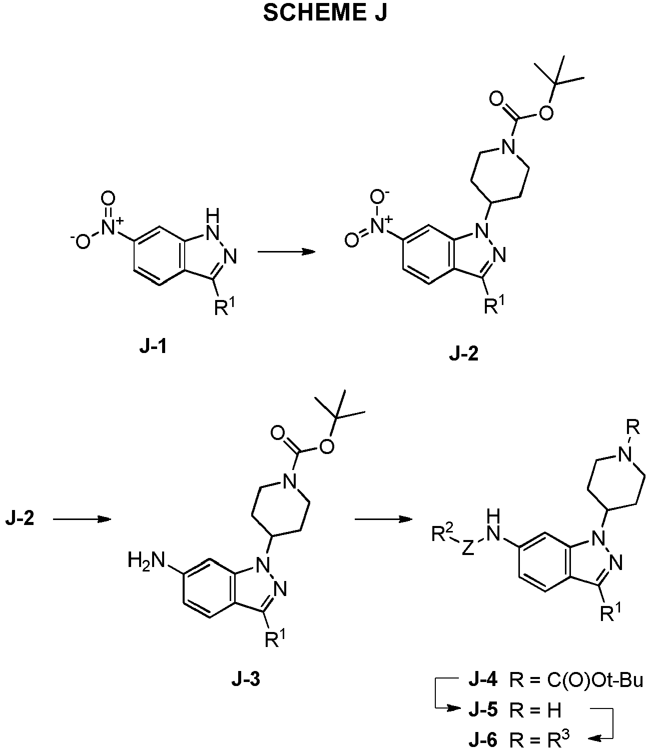 Compounds of formula (I) may be prepared as described in **Scheme K.** Hologenation of indoles such as 4-methyl-5-nitro-1H-indole (K-1, Y = Me) with iodine in the presence of an inorganic base followed by alkylation of the indole nitrogen with an alkylhalide of the formula R¹I or R¹Br in the presence of base provides compounds of the formula K-2. Coupling of K-2 with tert-butyl 4-(4,4,5,5-tetramethyl-1,3,2-dioxaborolan-2-yl)-5,6-dihydropyridine-1(2H)-carboxylate in the presence of a palladium catalyst provides compounds of the formula K-3. Following deprotection of the nitrogen protecting group, intermediate K-4 may be transformed into compounds of the formula K-5 through common amine transformations including amide formation, sulphonamide formation, urea formation and reductive amination. Compounds of the formula K-5 may be treated with reducing conditions to provide K-6. The resulting amine may be transformed to amides by the reaction with acid chlorides in the presence of base or carboxylic acids with appropriate coupling agents or K-6 may be transformed to ureas by the reaction with a phosgene equivalent followed by an amine. 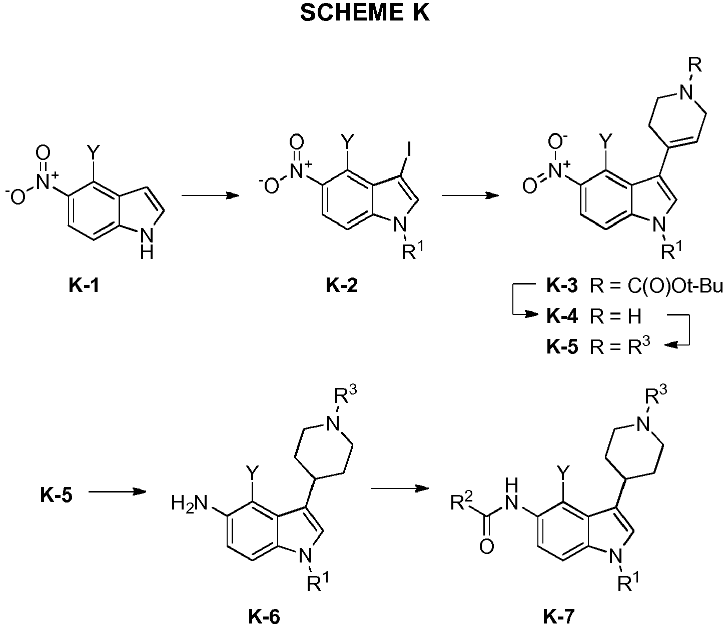 Compounds of formula (II) may be prepared as described in **Scheme L.** Nitration of L-1 provides compound L-2 which is then converted to L-3 by the treatment with trimethylaluminum in the presence of a palladium catalyst. Deprotection of the indole nitrogen followed by iodination provided compound L-5. Alkylation of the indole nitrogen by alkylhalides such as iodomethane provided compound L-6. Coupling of L-6 with tert-butyl 4-(4,4,5,5-tetramethyl-1,3,2-dioxaborolan-2-yl)-5,6-dihydropyridine-1(2H)-carboxylate in the presence of a palladium catalyst provides compound L-7. Compound L-7 may be treated with reducing conditions to provide L-8. The resulting amine may be transformed to amides by the reaction with acid chlorides in the presence of base or carboxylic acids with appropriate coupling agents or L-8 may be transformed to ureas by the reaction with a phosgene equivalent followed by an amine. Following deprotection of the nitrogen protecting group, intermediate L-10 may be transformed into compounds of the formula L-11 through common amine transformations including amide formation, sulphonamide formation, urea formation and reductive amination. 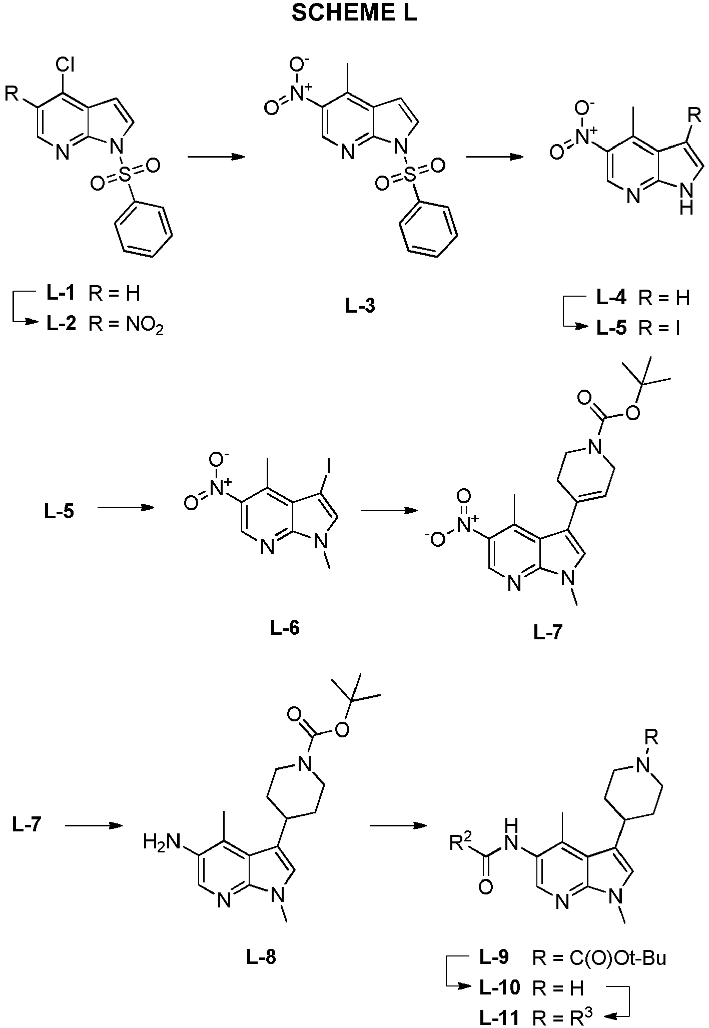 Compounds of formula (I) may be prepared as described in **Scheme M.** Condensation of 2-oxooxazolidine-3-sulfonyl chloride with compounds of the formula M-1 in the presence of pyridine provide compounds of the formula M-2. Reaction of M-2 with primary or secondary amines provides compounds of the formula M-3.

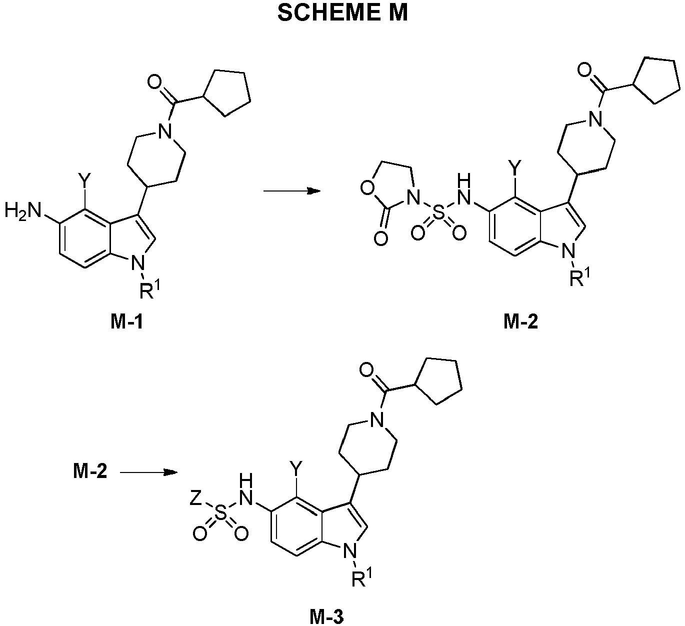

### EXEMPLIFICATION

The invention now being generally described, will be more readily understood by reference to the following examples, which are included merely for purposes of illustration of certain aspects and embodiments of the present invention, and are not intended to limit the invention. The following illustrate the synthesis of various compounds of the present invention. Additional compounds within the scope of this invention may be prepared using the methods illustrated in these Examples, either alone or in combination with techniques generally known in the art.

Experiments were generally carried out under inert atmosphere (nitrogen or argon), particularly in cases where oxygen- or moisture-sensitive reagents or intermediates were employed. Commercial solvents and reagents were generally used without further purification, including anhydrous solvents where appropriate. Mass spectrometry data is reported from either liquid chromatography-mass spectrometry (LCMS), atmospheric pressure chemical ionization (APCI) or gas chromatography-mass spectrometry (GCMS) instrumentation. Chemical shifts for nuclear magnetic resonance (NMR) data are expressed in parts per million (ppm, δ) referenced to residual peaks from the deuterated solvents employed. Coupling constants (J values) are reported in Hertz.

For syntheses referencing procedures in other Examples or Methods, reaction conditions (length of reaction and temperature) may vary. In general, reactions were followed by thin layer chromatography or mass spectrometry, and subjected to work-up when appropriate. Purifications may vary between experiments: in general, solvents and the solvent ratios used for eluants/gradients were chosen to provide appropriate Rf's or retention times (RetT).

The following abbreviations are used herein: DCM: dichloromethane; DMF: dimethylformamide; NMP: N-Methylpyrrolidone; BINAP: 2,2'-bis(diphenylphosphino)-1,1'-binaphthyl; MeOH: methanol; TEA: triethylamine; and THF: tetrahydrofuran.

### Reference Example 1

### Preparation of 3-cyano-N-(3-(1-(cyclpentylarbonyl)piperidin-4-yl)-1-methyl-1H-indol-5-yl)benzamide

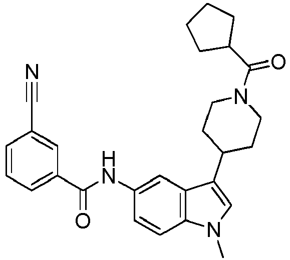

Step 1: tert-Butyl 4-(5-nitro-1H-indol-3-yl)-5,6-dihydropyridine-1 (2H)-carboxylate (Int-1). To freshly prepared sodium methoxide (5.0 g, 92.59 mmol) in MeOH (100 mL) was added 5-nitroindole (5.0 g, 30.87 mmol) and tert-butyl 4-oxopiperidine-1-carboxylate (18.56 g, 19.52 mmol). The reaction mixture was heated to reflux for 24 h. The progress of reaction was monitored by TLC (40% ethyl acetate in hexane). After most of the starting material consumed (By TLC), reaction mixture was cooled to room temperature and concentrated under vacuum. The residue obtained was diluted with water (100 mL), extracted by using ethyl acetate (3x100 mL), dried (Na₂SO₄), filtered and concentrated to obtain crude material. The crude compound was purified by using column chromatography (silica gel, 100-200 mesh) to afford the title compound (9.0 g, 87%) as a yellow solid. ¹H NMR (400 MHz, CDCl₃): δ 11.93 (s, 1H), 8.72 (s, 1H), 8.02 (d, J = 8.8 Hz, 1H), 8.71 (s, 1H), 7.56 (d, J = 8.8 Hz, 1H), 6.20 (s, 1H), 4.18 (m, 2H), 3.64 (m, 2H), 2.58 (m, 2H), 1.45 (s, 9H); LCMS: m/e 243.95 [M-100]+ Boc deprotected

Step 2: tert-Butyl 4-(1-methyl-5-nitro-1H-indol-3-yl)-5,6-dihydropyridine-1(2H)-carboxylate (Int-2). To a solution of tert-butyl 4-(5-nitro-1H-indol-3-yl)-5,6-dihydropyridine-1(2H)-carboxylate (Int-1; 4.5 g, 13.12 mmol) in 80 mL of THF was added NaH (2.1 g, 52.48 mmol, 60% w/w in mineral oil) at 0 °C. The reaction mixture was stirred at room temperature for 1 h and then Mel (3.3 mL, 52.48 mmol) was added dropwise at 0 °C. The reaction mixture was then allowed to stir at room temperature for overnight. The progress of reaction was monitored by TLC (40% ethyl acetate in hexane). After completion, reaction mixture was quenched by addition of ice-water and then extracted by using ethyl acetate (2x100 mL). The combined organic phases were dried (Na₂SO₄), filtered and concentrated to obtain the title compound (4.6 g, 98%) as a yellow solid. ¹H NMR (400 MHz, DMSO-d6): δ 8.72 (s, 1H), 8.07 (d, J = 9.2 Hz, 1H), 7.71 (s, 1H), 7.66 (d, J = 9.2 Hz, 1H), 6.21 (s, 1H), 4.08 (brs, 2H), 3.86-3.84 (m, 3H), 3.58-3.57 (m, 2H), 2.54 (m, 2H, Merged), 1.44 (s, 9H); LCMS: m/e 258.95 [M-100]+ Boc deprotected

Step 3. tert-Butyl 4-(5-amino-1-methyl-1H-indol-3-yl) piperidine-1-carboxylate. To a solution of tert-butyl 4-(1-methyl-5-nitro-1H-indol-3-yl)-5,6-dihydropyridine-1(2H)-carboxylate (4.5 g, 12.61 mmol) in 50 mL of methanol was added Pd/C (0.1g) and the reaction mixture was heated at 40 °C under H₂ atmosphere (Balloon pressure) for 7 h. The progress of reaction was monitored by TLC (50% ethyl acetate in hexane). After completion, the reaction mixture was filtered through Celite, washed with methanol and the combined filtrate was concentrated in vacuum to obtain the title compound (3.4 g, 82%) as a light brown solid. ¹H NMR (400 MHz, DMSO-d6): δ 7.04 (d, J = 8.4 Hz, 1H), 6.88 (s, 1H), 6.69 (s, 1H), 6.51 (dd, J = 1.6, 8.4 Hz, 1H), 4.47 (s, 2H), 4.06-4.03 (m, 2H), 3.60 (s, 3H), 3.09-2.75 (m, 5H), 1.87 (d, J = 12.4 Hz, 2H), 1.41 (s, 9H). LCMS: m/e 352.10 [M+Na]+

Step 4: 3-Cyanobenzoyl chloride. To a solution of 3-cyanobenzoic acid (7 g, 47.61 mmol) in toluene (100 mL) was added SOCl₂ (17.38 mL, 238.09 mmol) and DMF (2-3 drops, catalytic). The reaction mixture was allowed to heat at 95 °C for 4 h. The progress of reaction was monitored by TLC (30% Ethyl acetate in hexane, the reaction mass was quenched with dry methanol for TLC). After completion, reaction mixture was concentrated in vacuo to give a crude 3-cyanobenzoyl chloride (7.84 g, 99%) which is used in the next reaction without further purification.

Step 5: tert-Butyl 4-(5-(3-cyanobenzamido)-1-methyl-1H-indol-3-yl)piperidine-1-carboxylate. To a solution of tert-butyl 4-(5-amino-1-methyl-1H-indol-3-yl)piperidine-1-carboxylate (Int-2; 5 g, 15.19 mmol) in DCM (70 mL) was added TEA (6.4 mL, 45.59 mmol) followed by addition of 3-cyanobenzoyl chloride (3 g, 18.23 mmol) in DCM (10 mL) at 0 °C. The progress of reaction was monitored by TLC (50% ethyl acetate in hexane). After completion, reaction mixture was quenched with water and extracted by using DCM. The combined extracts were dried (Na₂SO₄), filtered and concentrated *in vacuo* to give a crude compound which was subjected to column chromatography (Silica gel, 100-200 mesh) to afford the title compound (4.32 g, 62%) as a light brown solid. ¹H NMR (400 MHz, CDCl₃): δ 8.21 (s, 1H), 8.14 (d, J=7.6 Hz, 1H), 8.00-7.93 (m, 2H), 7.81 (d, J=8.0 Hz, 1H), 7.63 (t, J= 7.6 Hz, 1H), 7.33 (d, J= 8.8 Hz, 1H), 7.28 (d, J= 8.8 Hz, 1H), 6.83 (s, 1H), 4.20-4.13 (m, 2H), 3.75 (s, 3H), 2.98-2.84 (m, 3H), 2.04-1.99 (m, 2H), 1.67-1.56 (m, 2H), 1.48 (s, 9H). LCMS: m/e 358.95 [M-100] + (De-Boc compound observed as a base peak).

Step 6: 3-cyano-N-(1-methyl-3-(piperidin-4-yl)-1H-indol-5-yl)benzamide. To a solution of tert-butyl 4-(5-(4-cyanopyridine-2-carboxamido)-1-methyl-1H-indol-3-yl)piperidine-1-carboxylate (3 g, 9.43 mmol) in 20 mL of methanol was added 4M HCI in dioxane (30 mL) at 0 °C. The reaction mixture was allowed to warm up to room temperature and stirred overnight. The progress of reaction was monitored by TLC (10% methanol in DCM). After completion, reaction mixture was concentrated in vacuo to give a crude compound which was neutralized with sat. NaHCO₃ solution and then extracted with 10% methanol in DCM. The combined extracts were dried (Na₂SO₄), filtered and concentrated in vacuum to give the title compound (1.5 g, 44.64%) as a brown solid. ¹H NMR (400 MHZ, CDCl₃): δ 8.21 (s, 1H), 8.15 (d, J=8.0 Hz, 1H), 8.04-7.99 (m, 2H), 7.81 (d, J=8.4 Hz, 1H), 7.63 (t, J= 8.0 Hz, 1H), 7.36 (d, J= 8.0 Hz, 1H), 7.28 (d, J= 8.0 Hz, 1H), 6.85 (s, 1H), 3.75 (s, 3H), 3.19-2.76 (m, 6H), 2.04-1.65 (m, 4H). LCMS: m/e 358.95 [M+1] +

Step 7: 3-cyano-N-(3-(1-(cyclopentylcarbonyl)piperidin-4-yl)-1-methyl-1H-indol-5-yl)benzamide. Cyclopentanecarboxylic acid (128 mg, 1.114 mmol) and TBTU (430 mg, 1.337 mmol) were dissolved in DMF (15 mL) and DIPEA (720 mg, 5.57mmol) was added. After stirring at 40 °C for 1 hr, 3-cyano-N-(1-methyl-3-(piperidin-4-yl)-1H-indol-5-yl)benzamide (400 mg, 1.114 mmol) was added and the mixture was stirred at 40 °C overnight. The crude product was purified by preparative TLC to give the title compound (21 mg, 4.1%) as a yellow solid. ¹H NMR (400 MHZ,CDCl₃): δ 8.21 (br.s, 1 H), 8.17 (d, J=7.6 Hz, 1 H), 8.01 (br.s, 1H), 7.92 (br.s, 1H), 7.84 (d, J=7.6 Hz, 1H), 7.66 (t, J=7.6 Hz, 1H), 7.29-7.33 (m, 2H), 6.84 (s, 1H), 4.78 (d, J= 13.2 Hz, 1H), 4.10 (d, J= 13.2 Hz,1H), 3.76 (s, 3H), 3.05-3.23 (m, 2H), 2.91-2.98 (m,1H), 2.69-2.76 (m,1H), 2.04-2.16 (m, 2H), 1.69-1.48 (m, 6H), 1.55-1.66 (m, 4H). LCMS: m/e 455.10 [M+1]+.

The following examples were prepared analogous to Reference Example 1 (where "R" indicates that the example is also a reference example).

| **Example** | **Structure** | **Name** | **MS (M+H)** |
|---|---|---|---|
| **R2** | 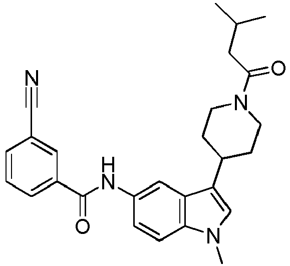 | 3-cyano-N-(1-methyl-3-(1-(3-methylbutanoyl)piperidin-4-yl)-1H-indol-5-yl)benzamide | 442.2 |
| **R3** | 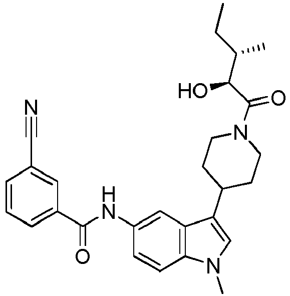 | 3-cyano-N-(3-(1-((2S,3S)-2-hydroxy-3-methylpentanoyl)piperidin-4-yl)-1-methyl-1H-indol-5-yl)benzamide | 472.2 |
| **R4** | 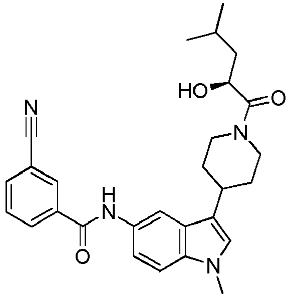 | 3-cyano-N-(3-(1-((2S)-2-hydroxy-4-methylpentanoyl)piperidin-4-yl)-1-methyl-1H-indol-5-yl)benzamide | 472.2 |
| **R5** | 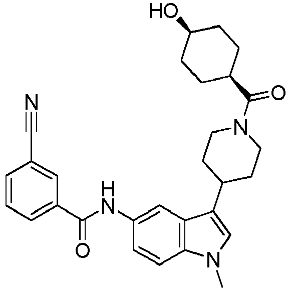 | 3-cyano-N-(3-{1-[(cis-4-hydroxycyclohexyl)carbonyl]piperidin-4-yl}-1-methyl-1H-indol-5-yl)benzamide | 484.2 |

### Example R6

### Preparation of 4-cyano-N-(3-(1-(cyclopentylcarbonyl)piperidin-4-yl)-1-methyl-1H-indol-5-yl)pyridine-2-carboxamide

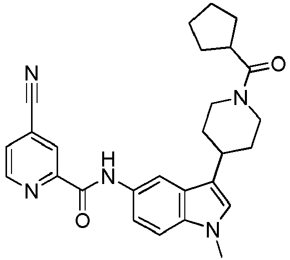

Step 1: tert-Butyl 4-(5-(4-cyanopyridine-2-carboxamido)-1-methyl-1H-indol-3-yl)piperidine-1-carboxylate (Int-2). To a solution of tert-butyl 4-(5-(4-cyanopicolinamido)-1-methyl-1H-indol-3-yl)piperidine-1-carboxylate (prepared in Reference Example **1,** 3.3 g, 10.03 mmol) in DCM (20 mL) was added TEA (4.2 mL, 30.09 mmol) followed by addition of 4-cyanopicolinoyl chloride (2.1 g, 12.53 mmol) in DCM (10 mL) at 0 °C. The progress of reaction was monitored by TLC (50% ethyl acetate in hexane). After completion, reaction mixture was quenched with water and extracted by using DCM. The combined extracts were dried (Na₂SO₄), filtered and concentrated in vacuo to give a crude compound which was subjected to column chromatography (Silica gel, 100-200 mesh) to afford the title compound (3.7 g, 80%) as a light brown solid. ¹H NMR (400 MHz, CDCl3): δ 9.88 (s, 1H), 8.82 (d, J = 4.8 Hz, 1H), 8.55 (s, 1H), 8.13 (s, 1H), 7.71 (dd, J = 1.2, 4.8 Hz, 1H), 7.47 (dd, J = 2.0, 8.8 Hz, 1H), 7.28 (d, J = 8.8 Hz, 1H), 6.84 (s, 1H), 4.23-4.18 (m, 2H), 3.76 (s, 3H), 3.15-2.88 (m, 3H), 2.03 (d, J = 12.8 Hz, 2H), 1.65-1.54 (m, 2H), 1.49 (s, 9H); LCMS: m/e 359.90 [M-100]+ Boc deprotected.

Step 2: 4-Cyano-N-(1-methyl-3-(piperidin-4-yl)-1H-indol-5-yl) picolinamide. To a solution of tert-butyl 4-(5-(4-cyanopyridine-2-carboxamido)-1-methyl-1H-indol-3-yl)piperidine-1-carboxylate (Int-2; 3.7 g, 8.04 mmol) in 15 mL of methanol was added 4M HCI in dioxane (30 mL) at 0 °C. The reaction mixture was allowed to warm up to room temperature and stirred overnight. The progress of the reaction was monitored by TLC (10% methanol in DCM). After completion, reaction mixture was concentrated in vacuo to give a crude compound which was neutralized with sat. NaHCO₃ solution and then extracted with 10% methanol in DCM. The combined extracts were dried (Na₂SO₄), filtered and concentrated in vacuum to give the title compound (2.2 g, 78%) as a brown solid. ¹H NMR (400 MHz, CDCl₃): δ 10.03 (s, 1H), 8.79-8.77 (m, 2H), 8.15 (s, 1H), 8.03 (d, J = 4.8 Hz, 1H), 7.44 (d, J = 8.8 Hz, 1H), 7.28 (d, J = 8.8 Hz, 1H), 6.86 (s, 1H), 5.78 (brs, 1H), 3.75 (s, 3H), 3.22-2.79 (m, 5H), 2.03 (d, J = 10.4 Hz, 2H), 1.78-1.68 (m, 2H). LCMS: m/e 377. 90 [M+H₂O]+

Step 3: 4-cyano-N-(3-(1-(cyclopentylcarbonyl)piperidin-4-yl)-1-methyl-1H-indol-5-yl)pyridine-2-carboxamide. A solution of cyclopentylcarboxylic acid (105 mg, 0.92 mmol), DIPEA (0.75 mL, 4.2 mmol) and EDCI.HCl (531 mg, 2.77 mmol) and HOBT (340 mg, 2.52 mmol) in DMF (1 mL) was stirred at room temperature for 1h. KB 4-Cyano-N-(1-methyl-3-(piperidin-4-yl)-1H-indol-5-yl) picolinamide (300 mg, 0.84 mmol) was added to the above mixture and stirred at room temperature. The progress of reaction was monitored by TLC (5% methanol in DCM) and LCMS. After completion of the reaction (overnight), the reaction was diluted with water and extracted with DCM. The combined organic layers were dried (Na₂SO₄), filtered, concentrated and the crude material was purified by column chromatography (silica gel, 230-400 mesh) to give the title compound (280 mg, 74%) as a yellow solid. ¹H NMR (400 MHZ, CDCl3): δ 9.88 (s, 1H), 8.82 (d, J=4.8 Hz, 1H), 8.55 (s, 1H), 8.18 (s, 1H), 7.71 (dd, J= 1.2, 4.8, Hz, 1H), 7.44 (dd, J= 1.6, 8.8 Hz, 1H), 7.29 (d, J= 8.8, Hz, 1H), 6.84 (s, 1H), 4.78 (d, J= 12.8 Hz, 1H), 4.15-4.08 (m, 1H), 3.76 (s, 3H), 3.23 (t, J= 12.8 Hz, 1H), 3.14-2.94 (m, 2H), 2.75 (t, J= 12.8 Hz, 1H), 2.18-2.10 (m, 2H), 1.87-1.59 (m, 10H). LCMS: m/e 456.00 [M+H]+

The following examples were prepared analogous to Example R6.

| **Example** | **Structure** | **Name** | **MS (M+H)** |
|---|---|---|---|
| **R7** | 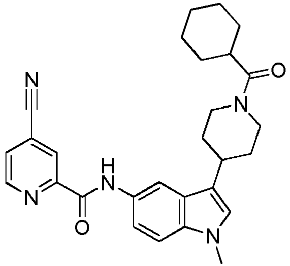 | 4-cyano-N-{3-[1-(cyclohexylcarbonyl)piperidin-4-yl]-1-methyl-1H-indol-5-yl}pyridine-2-carboxamide | 470.2 |
| **R8** | 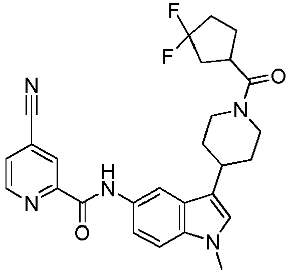 | 4-cyano-N-(3-{1-[(3,3-difluorocyclopentyl)carbonyl]piperidin-4-yl}-1-methyl-1H-indol-5-yl)pyridine-2-carboxamide | 490.1 |
| **R9** | 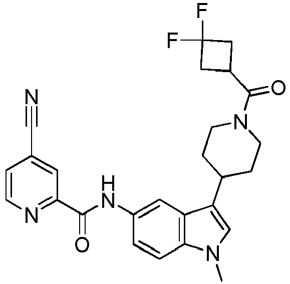 | 4-cyano-N-(3-{1-[(3,3-difluorocyclobutyl)carbonyl]piperidin-4-yl}-1-methyl-1H-indol-5-yl)pyridine-2-carboxamide | 477.2 |
| **R10** | 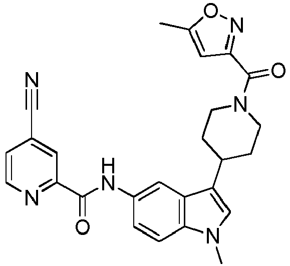 | 4-cyano-N-(1-methyl-3-{1-[(5-methylisoxazol-3-yl)carbonyl]piperidin-4-yl}-1H-indol-5-yl)pyridine-2-carboxamide | 469.15 |
| **R11** | 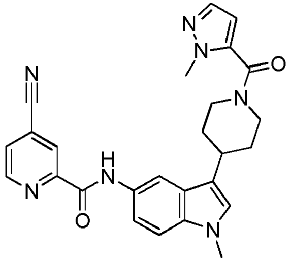 | 4-cyano-N-(1-methyl-3-{1-[(1-methyl-1H-pyrazol-5-yl)carbonyl]piperidin-4-yl}-1H-indol-5-yl)pyridine-2-carboxamide | 468.15 |
| **R12** | 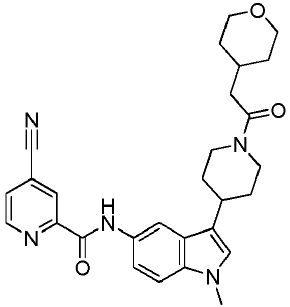 | 4-cyano-N-{1-methyl-3-[1-(tetrahydro-2H-pyran-4-ylacetyl)piperidin-4-yl]-1H-indol-5-yl}pyridine-2-carboxamide | 486.20 |
| **R13** | 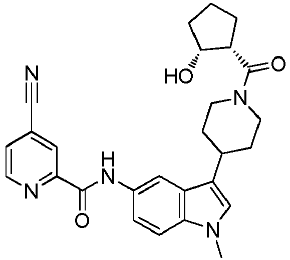 | 4-cyano-N-[3-(1-{[(1S*,2R*)-2-hydroxycyclopentyl]carbonyl}piperidin-4-yl)-1-methyl-1H-indol-5-yl]pyridine-2-carboxamide | 472.20 |
| **R14** | 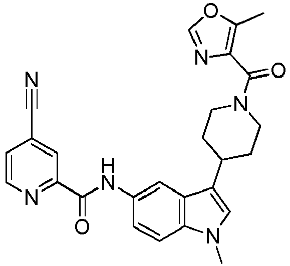 | 4-cyano-N-(1-methyl-3-{1-[(4-methyl-1,3-oxazol-5-yl)carbonyl]piperidin-4-yl}-1H-indol-5-yl)pyridine-2-carboxamide | 469.10 |
| **R15** | 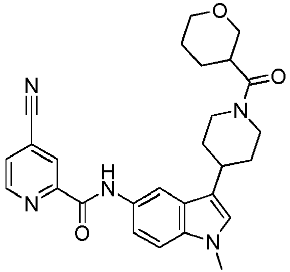 | 4-cyano-N-{1-methyl-3-[1-(tetrahydro-2H-pyran-3-ylcarbonyl)piperidin-4-yl]-1H-indol-5-yl}pyridine-2-carboxamide | 472.20 |
| R16 | 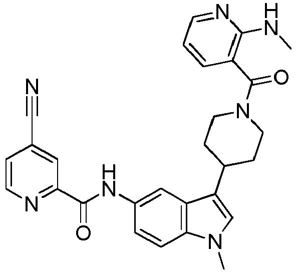 | 4-cyano-N-[1-methyl-3-(1-{[2-(methylamino)pyridin-3-yl]carbonyl}piperidin-4-yl)-1H-indol-5-yl]pyridine-2-carboxamide | 494.15 |
| **R17** | 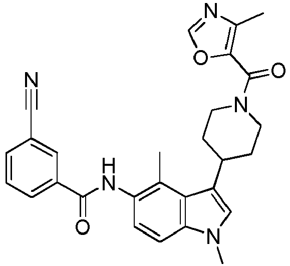 | 4-cyano-N-(1-methyl-3-{1-[(5-methyl-1,3-oxazol-4-yl)carbonyl]piperidin-4-yl}-1H-indol-5-yl)pyridine-2-carboxamide | 469.15 |
| **R18** | 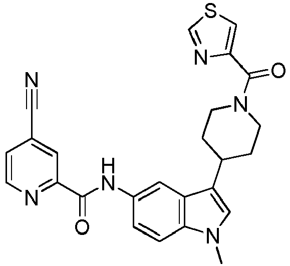 | 4-cyano-N-{1-methyl-3-[1-(1,3-thiazol-4-ylcarbonyl)piperidin-4-yl]-1H-indol-5-yl}pyridine-2-carboxamide | 471.00 |
| **R19** | 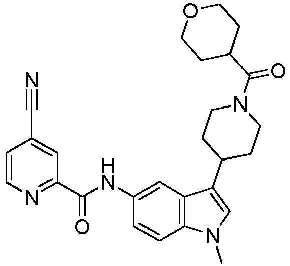 | 4-cyano-N-{1-methyl-3-[1-(tetrahydro-2H-pyran-4-ylcarbonyl)piperidin-4-yl]-1H-indol-5-yl}pyridine-2-carboxamide | 472.20 |
| **R20** | 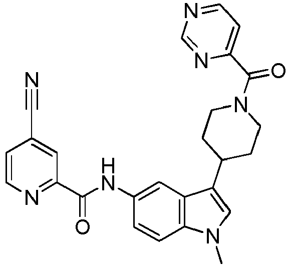 | 4-cyano-N-{1-methyl-3-[1-(pyrimidin-4-ylcarbonyl)piperidin-4-yl]-1H-indol-5-yl}pyridine-2-carboxamide | 466.15 |
| **R21** | 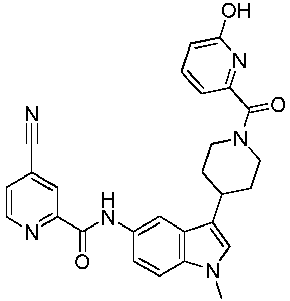 | 4-cyano-N-(3-{1-[(6-hydroxypyridin-2-yl)carbonyl]piperidin-4-yl}-1-methyl-1H-indol-5-yl)pyridine-2-carboxamide | 481.15 |
| **R22** | 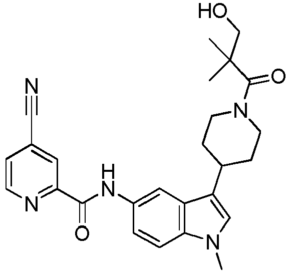 | 4-cyano-N-{3-[1-(3-hydroxy-2,2-dimethylpropanoyl)piperidin-4-yl]-1-methyl-1H-indol-5-yl}pyridine-2-carboxamide | 460.15 |
| **R23** | 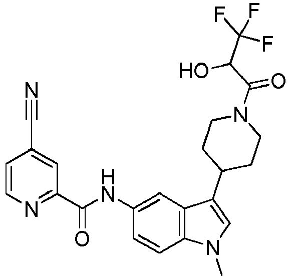 | 4-cyano-N-{1-methyl-3-[1-(3,3,3-trifluoro-2-hydroxypropanoyl)piperidin-4-yl]-1H-indol-5-yl}pyridine-2-carboxamide | 486.20 |
| **R24** | 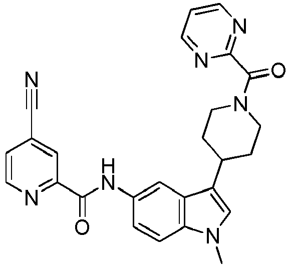 | 4-cyano-N-{1-methyl-3-[1-(pyrimidin-2-ylcarbonyl)piperidin-4-yl]-1H-indol-5-yl}pyridine-2-carboxamide | 466.15 |
| **R25** | 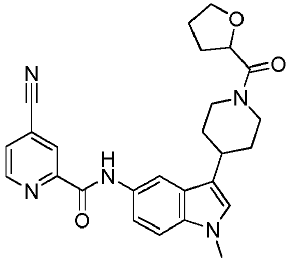 | 4-cyano-N-{1-methyl-3-[1-(tetrahydrofuran-2-ylcarbonyl)piperidin-4-yl]-1H-indol-5-yl}pyridine-2-carboxamide | 458.15 |
| **R26** | 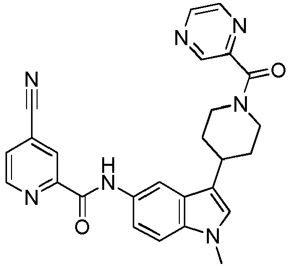 | 4-cyano-N-{1-methyl-3-[1-(pyrazin-2-ylcarbonyl)piperidin-4-yl]-1H-indol-5-yl}pyridine-2-carboxamide | 466.20 |
| **R27** | 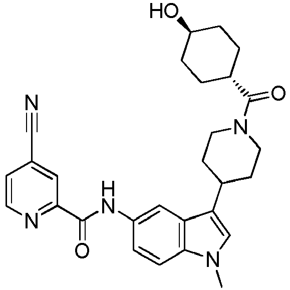 | 4-cyano-N-(3-{1-[(trans-4-hydroxycyclohexyl)carbonyl]piperidin-4-yl}-1-methyl-1H-indol-5-yl)pyridine-2-carboxamide | 486.30 |
| **R28** | 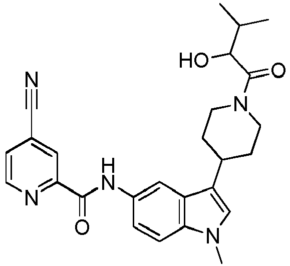 | 4-cyano-N-{3-[1-(2-hydroxy-3-methylbutanoyl)piperidin-4-yl]-1-methyl-1H-indol-5-yl}pyridine-2-carboxamide | 460.25 |
| **R29** | 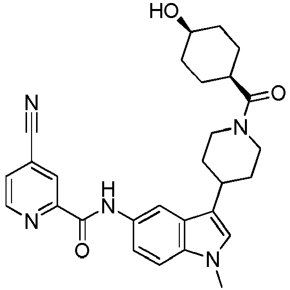 | 4-cyano-N-(3-{1-[(cis-4-hydroxycyclohexyl)carbonyl]piperidin-4-yl}-1-methyl-1H-indol-5-yl)pyridine-2-carboxamide | 486.25 |
| **R30** | 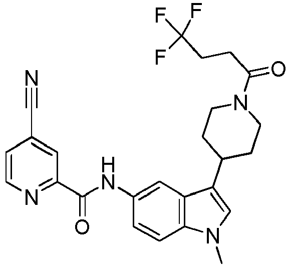 | 4-cyano-N-{1-methyl-3-[1-(4,4,4-trifluorobutanoyl)piperidin-4-yl]-1H-indol-5-yl}pyridine-2-carboxamide | 484.1 |
| **R31** | 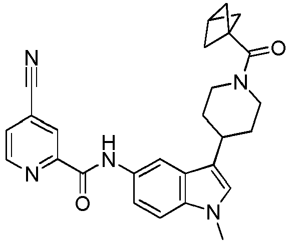 | N-{3-[1-(bicyclo[1.1.1]pent-1-ylcarbonyl)piperidin-4-yl]-1-methyl-1H-indol-5-yl}-4-cyanopyridine-2-carboxamide | 454.2 |
| **R32** | 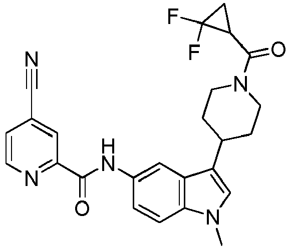 | 4-cyano-N-(3-{1-[(2,2-difluorocyclopropyl)carbonyl]piperidin-4-yl}-1-methyl-1H-indol-5-yl)pyridine-2-carboxamide | 464.2 |
| **R33** | 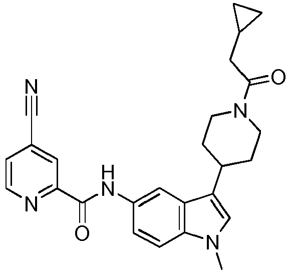 | 4-cyano-N-{3-[1-(cyclopropylacetyl)piperidin-4-yl]-1-methyl-1H-indol-5-yl}pyridine-2-carboxamide | 442.3 |
| **R34** | 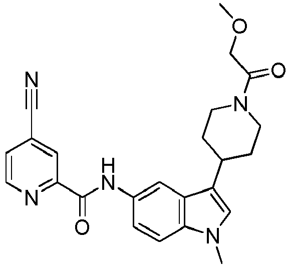 | 4-cyano-N-{3-[1-(methoxyacetyl)piperidin-4-yl]-1-methyl-1H-indol-5-yl}pyridine-2-carboxamide | 432.2 |
| **R35** | 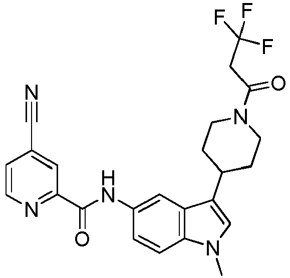 | 4-cyano-N-{1-methyl-3-[1-(3,3,3-trifluoropropanoyl)piperidin-4-yl]-1H-indol-5-yl}pyridine-2-carboxamide | 470.2 |

### Reference Example 36

### Preparation of 4-cyano-N-(3-(1-(cyclopentanecarbonyl)piperidin-4-yl)-1-ethyl-1H-indol-5-yl)picolinamide

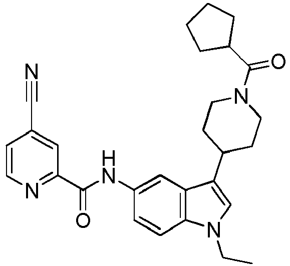

Step 1: preparation of tert-butyl 4-(5-amino-1H-indol-3-yl)piperidine-1-carboxylate. A high pressure flask was charged with ethanol (140 mL), tert-butyl 4-(5-nitro-1H-indol-3-yl)-5,6-dihydropyridine-1(2H)-carboxylate (prepared in Reference Example **1,** 7 g, 0.02 mol), acetic acid (1.2 g, 0.02 mol) and 20% Pd/C (50% wet, 0.7 g). The mixture was agitated under a hydrogen atmosphere for 16 h. The reaction was filtered through a pad of Celite® and the solvent removed under reduced pressure. The resulting solid was partitioned between saturated potassium carbonate and EtOAc. The organic phase was extracted and evaporated to afford the title compound which was carried further in the sequence without any further purification or characterization.

Step 2: preparation of tert-butyl 4-(5-(4-cyanopicolinamido)-1H-indol-3-yl)piperidine-1-carboxylate. To a slurry of tert-butyl 4-(5-amino-1H-indol-3-yl)piperidine-1-carboxylate (8.25 g, 26.2 mmol) in dichloromethane (120 mL) was added Et₃N and the mixture was cooled to 0 °C, whereupon 4-cyanopicolinoyl chloride (4.49 g, 26,9 mmol) in DCM (60 mL) was added slowly. The cooling bath was removed after one hour and the mixture was stirred at room temperature for 20 hours. Saturated aqueous sodium chloride was added, and the phases were separated. The aqueous phase was extracted with DCM and ethyl acetate, and the combined organic extracts were evaporated. The crude was concentrated in vacuo and was then triturated in ethanol (150 ml, 99.7%), filtered and washed with 50 ml ethanol. The product was dissolved in acetone and DCM, filtered, evaporated and dried in vacuo to provide 12.16 g (95%) the title compound as a yellow solid. LC/MS (20-100% CH₃CN:0.05%HCOOH(aq) gradient over 5 min): 3.20 min. 447 M+H.

Step 3: preparation of tert-butyl 4-(5-(4-cyanopicolinamido)-1-ethyl-1H-indol-3-yl)piperidine-1-carboxylate. Tert-butyl 4-(5-(4-cyanopicolinamido)-1H-indol-3-yl)piperidine-1-carboxylate (200 mg, 0.45 mmol) was dissolved in DCM. NaOH (6.3 ml, 2.5M), a few drops of phase transfer catalyst Aliquat 336 and ethyl iodide (1.1 ml, 13.5 mmol) were added and the mixture was stirred at room temperature for 24 h. The phases were partitioned. The aqueous phase was extracted with DCM and the combined organic phase was evaporated. The residue was purified by prep-HPLC to provide 110 mg (52%) of the title compound as a yellow solid. LC/MS (20-100% CH₃CN:0.05%HCOOH(aq) gradient over 5 min): 3.80 min. 474 M+H.

Step 4: preparation of 4-cyano-N-(1-ethyl-3-(piperidin-4-yl)-1H-indol-5-yl)picolinamide. To tert-butyl 4-(5-(4-cyanopicolinamido)-1-ethyl-1H-indol-3-yl)piperidine-1-carboxylate (110 mg, 0.23 mmol) was added HCl (0.9 ml, 4 M in Dioxane) and the mixture was stirred at room temperature for 12 h. Saturated aqueous NaHCO₃ and DCM were added, the phases were separated and the organic phase was evaporated to obtain 86 mg (87%) of the title compound. The crude was used without further purification in the subsequent step. LC/MS (20-100% CH₃CN:0.05% HCOOH(aq) gradient over 5 min): 3.87 min. 374 M+H.

Step 5: preparation of 4-cyano-N-(3-(1-(cyclopentanecarbonyl)piperidin-4-yl)-1-ethyl-1H-indol-5-yl)picolinamide. 4-Cyano-N-(1-ethyl-3-(piperidin-4-yl)-1H-indol-5-yl)picolinamide (HCI salt)(82 mg, 0.20 mmol), triethylamine (32 µl, 0.23 mmol) and cyclopentanecarbonyl chloride (28 µl, 0.23 mmol) were stirred in dry DCM (5 ml) at room temperature for 1M HCl (20 ml) and DCM (50 ml) were added, the phases were separated, and the solvents were evaporated. The residue was purified by prep-HPLC to provide 60 mg (64%) of the title compound as a white powder. LC/MS (20-100% CH₃CN:0.05%HCOOH(aq) gradient over 5 min): 3.12 min. 470 M+H. ¹H NMR (500 MHz, CD₃OD) δ ppm 8.89 (d, J=4.82, 1H), 8.41 (d, J=2.0, 1H), 8.11 (d, J= 2.0, 1H), 7.88 (dd, J=4.94, 1.61, 1H), 7.44 (dd, J=8.64, 2.0, 1H), 7.36 (d, J=8.64, 1H), 7.07 (s, 1H); 4.67 (m, 1H), 4.17 (m, 3H), 3.26 (m, 1H), 3.11 (m, 2H), 2.80 (m, 1H), 2.13 (m, 2H), 1.75 (m, 10H), 1.40 (t, J=7.23, 3H).

### Reference Example 37

### Preparation of 4-cyano-N-(3-(1-(cyclopentanecarbonyl)piperidin-4-yl)-1H-indol-5-yl)picolinamide

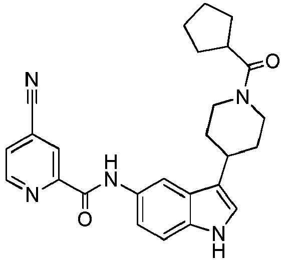

Step 1: (4-(5-amino-1H-indol-3-yl)piperidin-1-yl)(cyclopentyl)methanone. To a solution of tert-butyl 4-(5-amino-1H-indol-3-yl)piperidine-1-carboxylate (prepared in Reference Example **36,** 5.0 g, 16.0 mmol) in 100 mL of dichloromethane was added (9H-fluoren-9-yl)methyl-2,5-dioxopyrrolidin-1-yl carbonate (5.9 g, 17.4 mmol), and *N*-ethyl-*N-*isopropylpropan-2-amine (2.3 g, 17.4 mmol). The mixture was then stirred at room temperature for 18 hours then was washed with water and saturated sodium chloride. The organic layer was then collected and was dried over magnesium sulfate, filtered and evaporated under reduced pressure to give 6.7 g of a crude solid which was immediately diluted with 120 mL of methanol and treated with 30 mL of 4N hydrochloric acid in dioxane. This mixture was then stirred for 3 hours at room temperature and then concentrated under reduced pressure to give 5.5 g of a crude solid which was immediately treated with 30 mL of dimethylformamide. To this stirring solution was then added cyclopentyl carboxylic acid (1.72 g, 15.10 mmol), O-(Benzotriazol-1-yl)-N,N,N',N'-tetramethyluronium tetrafluoroborate (4.99 g, 15.10 mmol), and *N*-ethyl-*N*-isopropylpropan-2-amine (4.59 g, 34.80 mmol). The mixture was then stirred at room temperature for 1 hour then concentrated under reduced pressure and added to 300 mL of ethyl acetate. The mixture was then washed with water, saturated sodium chloride, dried with magnesium sulphate and then concentrated under reduced pressure to give 6.4 g of a crude solid which was immediately dissolved in ethyl acetate (120 mL) and the resulting solution was cooled to 0 °C. To this chilled solution was added piperidine (2.0 g, 23.0 mmol) and the resulting mixture was allowed to warm to room temperature while stirring over the course of 18 hours. The mixture was then concentrated under reduced pressure, then triturated with heptane and dried under vacuum to give 1.4 g (60%) of the titled compound as an off-white solid: ¹H NMR (400 MHz, Methanol-d4): δ: 7.22 (d, 1H, J = 8.4 Hz), 7.14 (s, 1H), 7.00 (s, 1H), 6.78 (d, 1H, J = 7.2 Hz), 4.65 (m, 1H), 4.20 (m, 1H), 3.31 (m, 1H), 3.10 (m, 2H), 2.80 (t, 1H), 2.11 (m, 2H), 1.87 (m, 2H), 1.74 (m, 4H), 1.63 (m, 4H).

Step 2: 4-cyano-N-(3-(1-(cyclopentanecarbonyl)piperidin-4-yl)-1H-indol-5-yl)picolinamide. To a solution of 4-cyanopicolinic acid (21.3 mg, 0.14 mmol) in 1 mL of toluene was added (4-(5-amino-1H-indol-3-yl)piperidin-1-yl)(cyclopentyl)methanone (30.0 mg, 0.09 mmol), O-(Benzotriazol-1-yl)-*N,N,N',N'*-tetramethyluronium tetrafluoroborate (47.7 mg, 0.14 mmol), and diisopropylethylamine (38.0 mg, 0.29 mmol). The mixture was then stirred at room temperature for 30 minutes then was washed with saturated aqueous sodium chloride. The organic layer was then collected and was dried over sodium sulfate, filtered and evaporated to give the title compound. LC/MS (10%-90% CH₃CN:H₂O gradient over 10 min): 5.35 min. 442.1 M+H. ¹H NMR (500 MHz, DMSO-*d*₆) δ ppm 10.82 (br. s., 1 H), 10.56 (s, 1 H), 8.97 (d, J=4.88 Hz, 1 H), 8.47 (s, 1 H), 8.07 - 8.20 (m, 2 H), 7.50 - 7.59 (m, 1 H), 7.31 (d, *J*=8.79 Hz, 1 H), 7.12 (s, 1 H), 4.54 (d, J=12.70 Hz, 1 H), 4.09 (d, *J*=13.18 Hz, 1 H), 3.18 (t, J=12.45 Hz, 1 H), 2.95 - 3.06 (m, 2 H), 2.69 (t, J=11.96 Hz, 1 H), 1.92 - 2.10 (m, 2 H), 1.41 - 1.85 (m, 10 H).

The following examples were prepared analogous to Reference Example 37.

| **Example** | **Structure** | **Name** | **MS (M+H)** |
|---|---|---|---|
| **R38** | 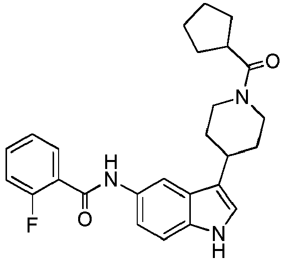 | N-{3-[1-(cyclopentylcarbonyl)piperidin-4-yl]-1H-indol-5-yl}-2-fluorobenzamide | 433.2 |
| **R39** | 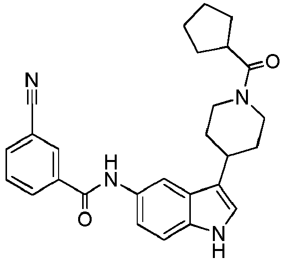 | 3-cyano-N-{3-[1-(cyclopentylcarbonyl)piperidin-4-yl]-1H-indol-5-yl}benzamide | 440.2 |
| **R40** | 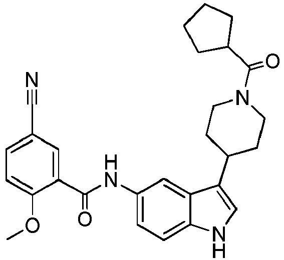 | 5-cyano-N-{3-[1-(cyclopentylcarbonyl)piperidin-4-yl]-1H-indol-5-yl}-2-methoxybenzamide | 470.2 |
| **R41** | 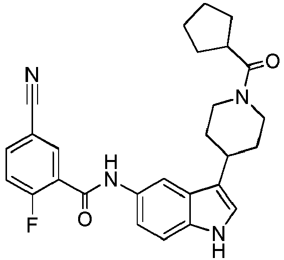 | 5-cyano-N-{3-[1-(cyclopentylcarbonyl)piperidin-4-yl]-1H-indol-5-yl}-2-fluorobenzamide | 458.2 |
| **R42** | 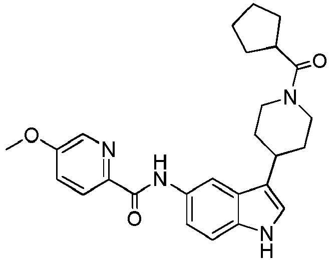 | N-{3-[1-(cyclopentylcarbonyl)piperidin-4-yl]-1H-indol-5-yl}-5-methoxypyridine-2-carboxamide | 446.2 |
| **R43** | 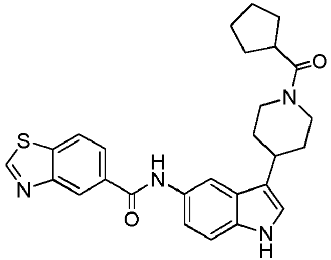 | N-{3-[1-(cyclopentylcarbonyl)piperidin-4-yl]-1H-indol-5-yl}-1,3-benzothiazole-5-carboxamide | 472.2 |
| **R44** | 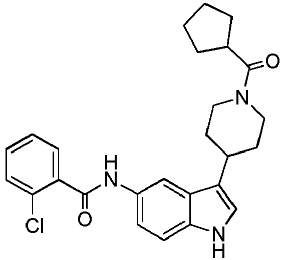 | 2-chloro-N-{3-[1-(cyclopentylcarbonyl)piperidin-4-yl]-1H-indol-5-yl}benzamide | 449.2 |
| **R45** | 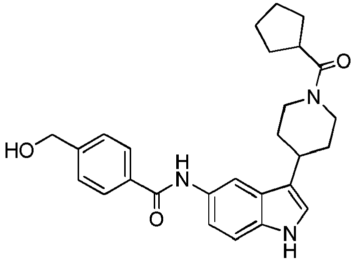 | N-{3-[1-(cyclopentylcarbonyl)piperidin-4-yl]-1H-indol-5-yl}-4-(hydroxymethyl)benzamide | 455.2 |
| **R46** | 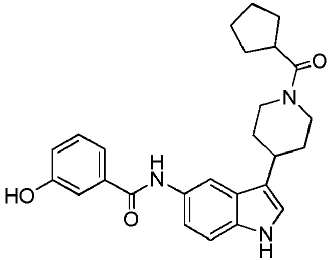 | N-{3-[1-(cyclopentylcarbonyl)piperidin-4-yl]-1H-indol-5-yl}-3-hydroxybenzamide | 431.2 |
| **R47** | 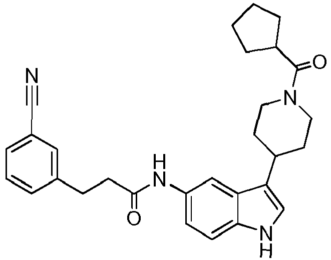 | 3-(3-cyanophenyl)-N-{3-[1-(cyclopentylcarbonyl)piperidin-4-yl]-1H-indol-5-yl}propanamide | 468.3 |
| **R48** | 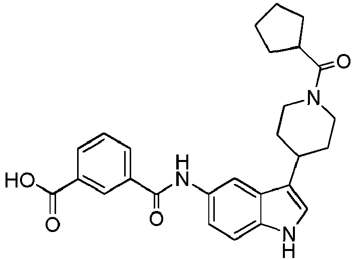 | 3-({3-[1-(cyclopentylcarbonyl)piperidin-4-yl]-1H-indol-5-yl}carbamoyl)benzoic acid | 459.2 |
| **R49** | 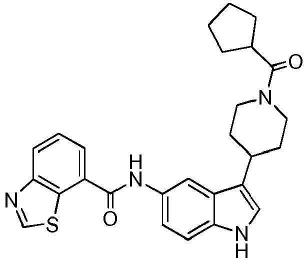 | N-{3-[1-(cyclopentylcarbonyl)piperidin-4-yl]-1H-indol-5-yl}-1,3-benzothiazole-7-carboxamide | 473.0 |
| **R50** | 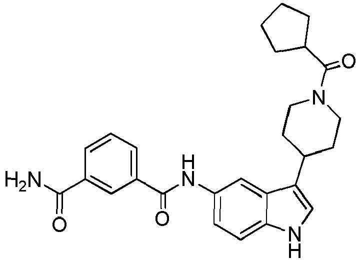 | N-{3-[1-(cyclopentylcarbonyl)piperidin-4-yl]-1H-indol-5-yl}isophthalamide | 459.1 |
| **R51** | 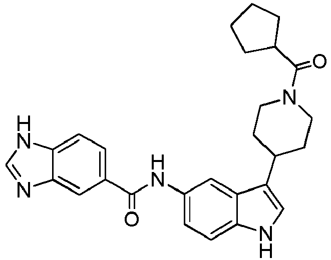 | N-{3-[1-(cyclopentylcarbonyl)piperidin-4-yl]-1H-indol-5-yl}-1H-benzimidazole-5-carboxamide | 456.1 |
| **R52** | 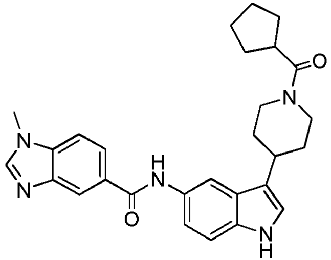 | N-{3-[1-(cyclopentylcarbonyl)piperidin-4-yl]-1H-indol-5-yl}-1-methyl-1H-benzimidazole-5-carboxamide | 470.1 |
| **R53** | 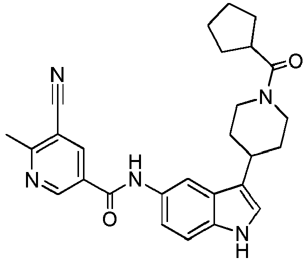 | 5-cyano-N-{3-[1-(cyclopentylcarbonyl)piperidin-4-yl]-1H-indol-5-yl}-6-methylnicotinamide | 456.2 |

### Example 54

### Preparation of 3-cyano-N-(3-(1-(cyclopentanecarbonyl)piperidin-4-yl)-1-methyl-1H-pyrrolo[2,3-b]pyridin-5-yl)benzamide

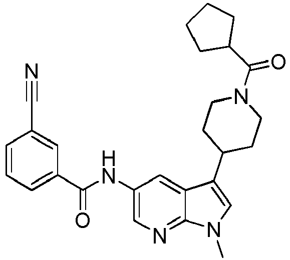

Step 1: preparation of 3-iodo-1-methyl-5-nitro-1H-pyrrolo[2,3-b]pyridine. To a suspension of 5-nitro-1H-pyrrolo[2,3-b]pyridine (500 mg, 3.06 mmol) in DMF (15 ml) was added KOH (241 mg, 4.29 mmol, pellets). The mixture was stirred at room temperature for 10 min. A clear orange solution was obtained. Iodine (856 mg, 3.37 mmol) was then added, and stirring was continued for 90 min. To the mixture was added K₂CO₃ (974 mg, 7.05 mmol) followed by iodomethane (1.14 ml, 18.4 mmol), and stirring was continued at room temperature for 2.5 hours. The mixture was diluted with water (50 ml), treated with NaHSO3 until yellow, and was then stirred for 30 min. The precipitate was collected by filtration, washed with plenty of water, and dried in vacuo to provide 845 mg (91%) of the title compound as a yellow solid. LC/MS (20-100% CH₃CN:0.05% HCOOH(aq) gradient over 5 min): 2.40 min. 288 M+H.

Step 2: preparation of tert-butyl 4-(1-methyl-5-nitro-1H-pyrrolo[2,3-b]pyridin-3-yl)-5,6-dihydropyridine-1(2H)-carboxylate. 3-iodo-1-methyl-5-nitro-1H-pyrrolo[2,3-b]pyridine (500 mg, 1.65 mmol), tert-butyl 4-(4,4,5,5-tetramethyl-1,3,2-dioxaborolan-2-yl)-5,6-dihydropyridine-1(2H)-carboxylate (612 mg, 1.98 mmol), Pd EnCat TPP30 (palladium acetate / PPh3, encapsulated, Aldrich 644706, 110 mg), K₂CO₃ (456 mg, 3.30 mmol), DME (8 ml), EtOH (2 ml) and H₂O (2 ml) were mixed in a reaction vessel, and the mixture was degassed with nitrogen for 10 min. The tube was then sealed, and the mixture was heated at 60 °C for 18 h. CHCl₃ and water were added and the phases were separated. The aqueous layer was extracted three times with CHCl3 and the combined organics were dried over Na₂SO₄. Evaporation of the solvents gave a solid yellow residue. The crude was purified by flash chromatography to provide 410 mg (69%) of the title compound as a bright yellow solid. LC/MS (20-100% CH₃CN:0.05% HCOOH(aq) gradient over 5 min): 3.42 min. 359 M+H.

Step 3: preparation of tert-butyl 4-(5-amino-1-methyl-1H-pyrrolo[2,3-b]pyridin-3-yl)piperidine-1-carboxylate. A mixture of tert-butyl 4-(1-methyl-5-nitro-1H-pyrrolo[2,3-b]pyridin-3-yl)-5,6-dihydropyridine-1(2H)-carboxylate (102 mg, 0.28 mmol), 5% Pd/C (30 mg), ammonium formate (215 mg, 3.42 mmol) in 96% EtOH (5 ml) was flushed with nitrogen, and the mixture was heated at 80 °C for 1 hour in a sealed vessel. The mixture was filtered through a plug of Celite, and the filtrate was concentrated in vacou. The residue was partitioned between CHCl3 and water. The layers were separated, and the aqueous layer was extracted twice with CHCl3. The combined extracts were evaporated. The crude was dissolved in EtOH (10 ml), and treated with 2 M NaOH (10 ml) and the mixture was stirred at 60 °C for 1 hour. The mixture was concentrated in vacuo until most of the EtOH was removed, and was then extracted four times with chloroform. The combined extracts were evaporated in vacuo, leaving 77 mg (82%) of the title compound as a light-tan foam. No further purification was performed. LC/MS (20-100% CH₃CN:0.05% HCOOH(aq) gradient over 5 min): 1.35 min. 331 M+H.

Step 4: preparation of tert-butyl 4-(5-(3-cyanobenzamido)-1-methyl-1H-pyrrolo[2,3-b]pyridin-3-yl)piperidine-1-carboxylate. To an ice-cold solution of tert-butyl 4-(5-amino-1-methyl-1H-pyrrolo[2,3-b]pyridin-3-yl)piperidine-1-carboxylate (100 mg, 0.30 mmol) and 3-cyanobenzoyl chloride (55 mg, 0.33 mmol) in DCM (5 ml) was added triethylamine (169 µl, 1.21 mmol). The temperature was allowed to room temperature and the mixture was stirred for 90 min. The solvents were evaporated and the residue was purified by flash chromatography to provide 105 mg (76%) of the title compound. LC/MS (20-100% CH₃CN:0.05% HCOOH(aq) gradient over 5 min): 2.85 min. 460 M+H.

Step 5: preparation of 3-cyano-N-(1-methyl-3-(piperidin-4-yl)-1H-pyrrolo[2,3-b]pyridin-5-yl)benzamide (HCl salt). To 4-(5-(3-cyanobenzamido)-1-methyl-1H-pyrrolo[2,3-b]pyridin-3-yl)piperidine-1-carboxylate (105 mg, 0.23 mmol) was added a solution of HCl in dioxane (2.3 ml, 4M). The mixture was stirred at room temperature for The solvents were evaporated to provide 78 mg (86%) of the title compound. The crude product was used without further purification in the next step. LC/MS (20-100% CH₃CN:0.05% HCOOH(aq) gradient over 5 min): 1.22 min. 360 M+H.

Step 6: preparation of 3-cyano-N-(3-(1-(cyclopentanecarbonyl)piperidin-4-yl)-1-methyl-1H-pyrrolo[2,3-b]pyridin-5-yl)benzamide. To an ice-cold solution of 3-cyano-N-(1-methyl-3-(piperidin-4-yl)-1H-pyrrolo[2,3-b]pyridin-5-yl)benzamide (HCl salt)(78 mg, 0.2 mmol) and cyclopentanecarbonyl chloride (29 µl, 0.24 mmol) in DCM (4 ml) was added triethylamine (121 µl, 0.87 mmol). The temperature was allowed to room temperature and the mixture was stirred at room temperature for 16 hours. The solvents were evaporated and the residue was purified by flash chromatography to provide 68 mg (75%) of the title compound as a white powder. LC/MS (20-100% CH₃CN:0.05% HCOOH(aq) gradient over 5 min): 2.47 min. 456 M+H. ¹H NMR (500 MHz, CD3OD) δ ppm 8.56 (m, 2H), 8.39 (m, 2H), 8.00 (m, 1H), 7.79 (t, 1H), 7.26 (s, 1H), 4.70 (m, 1H), 4.20 (m, 1H), 3.83 (s, 3H), 3.26 (m, 1H), 3.16-3.03 (m, 3H), 2.85-2.70 (m, 3H), 2.12 (m, 1H), 1.81 (m, 4H), 1.67 (m, 3H), 1.57 (m, 3H).

### Reference Example 55

### Preparation of 4-cyano-N-(3-(1-(cyclopentanecarbonyl)piperidin-4-yl)-1-methyl-1H-pyrrolo[3,2-b]pyridin-5-yl)picolinamide

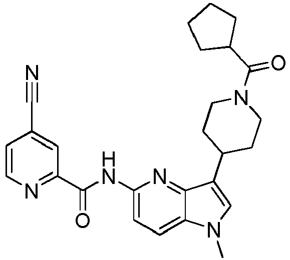

Step 1: preparation of (E)-N'-(6-((E)-2-(dimethylamino)vinyl)-5-nitropyridin-2-yl)-N,N-dimethylformimidamide. To a stirred solution of 6-methyl-5-nitropyridin-2-amine (1.0 g, 6.5 mmol) in DMF (5 ml) was added 1,1-dimethoxy-N,N-dimethylmethanamine (4.3 ml, 33 mmol) and the mixture was heated to 110 °C for 24 h. Evaporation of the solvents in vacuo provided 1.7 g (100%) of the title compound. The crude product was used in the next step without further purification.

Step 2: preparation of (E)-N,N-dimethyl-N'-(1H-pyrrolo[3,2-b]pyridin-5-yl)formimidamide. (E)-N'-(6-((E)-2-(dimethylamino)vinyl)-5-nitropyridin-2-yl)-N,N-dimethylformimidamide (1.7 g, 6.6 mmol) was dissolved in EtOH (12 ml) and Pd/C (27 mg, 10%) was added. The mixture was hydrogenated in a hydrogenation apparatus for four hours at 30 psi. The mixture was passed through a plug of celite and the fitrate was evaporated. The residue was purified by chromatography on neutral alumina to provide 1.24 g (100%) of the title compound. LC/MS (5-50% CH₃CN:0.05% HCOOH(aq) gradient over 5 min): 189 M+H.

Step 3: preparation of (E)-N'-(3-iodo-1H-pyrrolo[3,2-b]pyridin-5-yl)-N,N-dimethylformimidamide. To a stirred solution of (E)-N,N-dimethyl-N'-(1H-pyrrolo[3,2-b]pyridin-5-yl)formimidamide (470 mg, 2.50 mmol) in DMF at 0 °C was added N-iodosuccinimide (590 mg, 2.62 mmol), and the mixture was stirred for 18 hours at room temperature. The solvent was evaporated in vacuo and the residue was purified by neutral alumina chromatography to provide 785 mg (100%) of the title compound. LC/MS (5-50% CH₃CN:0.05%NH4Ac(aq) gradient over 5 min): 2.32 min. 315 M+H.

Step 4: preparation of (E)-N'-(3-iodo-1-methyl-1H-pyrrolo[3,2-b]pyridin-5-yl)-N,N-dimethylformimidamide. To a stirred solution of (E)-N'-(3-iodo-1H-pyrrolo[3,2-b]pyridin-5-yl)-N,N-dimethylformimidamide (785 mg, 2.5 mmol) in DCM (22 ml), tetrabutylammonium bromide (80 mg, 0.25 mmol), NaOH (4 ml, 2M) and Mel (200 µl, 3.25 mmol) were added and the mixture was stirred for 16 hours at room temperature. Water, DCM and EtOAc were added and the phases were separated. The solvents were evaporated and the residue was purified by neutral alumina chromatography to provide 686mg (84%) of the title compound. LC/MS (5-50% CH₃CN:0.05% NH4Ac(aq) gradient over 5 min): 3.05 min. 329 M+H.

Step 5: preparation of (4-(5-amino-1-methyl-1H-pyrrolo[3,2-b]pyridin-3-yl)-5,6-dihydropyridin-1(2H)-yl)(cyclopentyl)methanone. A mixture of (E)-N'-(3-iodo-1-methyl-1H-pyrrolo[3,2-b]pyridin-5-yl)-N,N-dimethylformimidamide (40 mg, 0.12 mmol), cyclopentyl(4-(4,4,5,5-tetramethyl-1,3,2-dioxaborolan-2-yl)-5,6-dihydropyridin-1(2H)-yl)methanone (89 mg, 0.29 mmol), tetrakis(triphenylphosphine)Pd(0)(14 mg, 0.01 mmol) and K₂CO₃ (50 mg, 0.37 mmol) in a mixture of DMF/water 8:1 (1.5 ml) was degassed and flushed with nitrogen gas. The reaction mixture was stirred at 100 °C for 16 hours. Water and DCM were added and the phases were separated. The solvents were evaporated and the residue was purified by preparative HPLC to provide 15mg (35%) of the title compound. LC/MS (20-100% CH₃CN:0.05%NH4Ac(aq) gradient over 5 min): 1.20 min. 325 M+H.

Step 6: preparation of (4-(5-amino-1-methyl-1H-pyrrolo[3,2-b]pyridin-3-yl)piperidin-1-yl)(cyclopentyl)methanone. (4-(5-amino-1-methyl-1H-pyrrolo[3,2-b]pyridin-3-yl)-5,6-dihydropyridin-1(2H)-yl)(cyclopentyl)methanone (12 mg, 0.04 mmol), ammonium formate (47 mg, 0.74 mmol) and palladium black (2.0 mg, 0.02 mmol) were mixed in DMF/NMP 5:1 (1 ml). The reaction mixture was heated under microwave irradiation at 150 °C for 60 minutes. The mixture was filtered through a plug of celite, the solvents were evaporated (except NMP) to provide the crude title compound. Yield not calculated. LC/MS (20-100% CH₃CN:0.05%NH₄Ac(aq) gradient over 5 min): 2.69 min. 327 M+H.

Step 7: preparation of 4-cyano-N-(3-(1-(cyclopentanecarbonyl)piperidin-4-yl)-1-methyl-1H-pyrrolo[3,2-b]pyridin-5-yl)picolinamide. To the crude (4-(5-amino-1-methyl-1H-pyrrolo[3,2-b]pyridin-3-yl)piperidin-1-yl)(cyclopentyl)methanone from step 6 was added DCM (0.5mL), Et₃N (30µL, 0.22 mmol) and 4-cyanopicolinoyl chloride (11mg, 0.07 mmol). The mixture was stirred at room temperature for 30 minutes, whereupon the solvents were evaporated. The residue was purified by preparative HPLC to provide10 mg (59%) of the title compound. LC/MS (20-100% CH₃CN:0.05% HCOOH(aq) gradient over 5 min): 2.63 min. 357 M+H. ¹H NMR (500 MHz, CD3OD) δ ppm 8.96 (dd, J=5.0, 1.0, 1H) 8.54 (s, 1H), 8.25 (d, J=9.0, 1H), 7.98 (dd, J=5.0, 1.56, 1H), 7.89 (d, J= 9.0, 1H), 7.31 (s, 1H), 4.69 (m, 1H), 4.23 (m, 1H), 3.82 (s, 3H), 3.13 (m, 1H), 2.84 (m, 1H), 2.25-2.11 (m, 2H), 1.94-1.60 (m, 12H).

### Reference Example 56

### Preparation of 4-cyano-N-(7-(1-(cyclopentanecarbonyl)piperidin-4-yl)-5-methyl-5H-pyrrolo[3,2-d]pyrimidin-2-yl)picolinamide

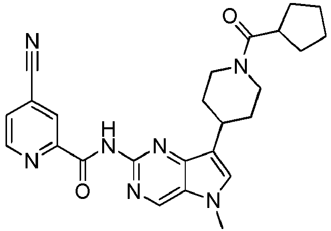

Step 1: preparation of 2-chloro-5H-pyrrolo[3,2-d]pyrimidine. 2,4-dichloro-5H-pyrrolo[3,2-d]pyrimidine (134 mg, 0.71 mmol), NaHCO₃ (66 mg, 0.78 mmol) and Pd/C (1.52 mg, 10%) were mixed in EtOH (4 ml). Hydrogen (3psi) was applied and the mixture was stirred for 2.5 hours at room temperature. The mixture was passed through a plug of celite and the filtrate was evaporated. The residue was purified by flash chromatography to afford 90mg (88%) of the title compound. LC/MS (20-100% CH₃CN:0.05% HCOOH(aq) gradient over 5 min): 1.58 min. 154 M+H.

Step 2: preparation of 2-chloro-7-iodo-5H-pyrrolo[3,2-d]pyrimidine. To a stirred solution of 2-chloro-5H-pyrrolo[3,2-d]pyrimidine (90 mg, 0.59 mmol) in DMF (1 ml) at 0 °C was added N-iodosuccinimide (138 mg, 0.62 mmol). The temperature was allowed to room temperature and the mixture was stirred for 17 hours. The solvent was evaporated and the residue was purified by flash chromatography to provide 110 mg (67%) of the title compound. LC/MS (20-100% CH₃CN:0.05% HCOOH(aq) gradient over 5 min): 2.75 min. 279 M+H.

Step 3: preparation of 2-chloro-7-iodo-5-methyl-5H-pyrrolo[3,2-d]pyrimidine. To a stirred solution of 2-chloro-7-iodo-5H-pyrrolo[3,2-d]pyrimidine (110 mg, 0.39 mmol) in DCM (5 ml) was added tetrabutylammonium bromide (19 mg, 0.06 mmol), NaOH (1 ml, 2M) and Mel (47 µl, 0.47 mmol) were added and the mixture was stirred for 16 hours at room temperature. Water, DCM and EtOAc were added and the phases were separated. Concentration by evaporation followed by trituration using Et2O gave 110mg (94%) of the title compound. LC/MS (20-100% CH₃CN:0.05% HCOOH(aq) gradient over 5 min): 3.07 min. 294 M+H.

Step 4: preparation of (4-(2-chloro-5-methyl-5H-pyrrolo[3,2-d]pyrimidin-7-yl)-5,6-dihydropyridin-1(2H)-yl)(cyclopentyl)methanone. 2-chloro-7-iodo-5-methyl-5H-pyrrolo[3,2-d]pyrimidine (25 mg, 0.09 mmol), cyclopentyl(4-(4,4,5,5-tetramethyl-1,3,2-dioxaborolan-2-yl)-5,6-dihydropyridin-1(2H)-yl)methanone (34 mg, 0.11 mmol), Dichlorobis(triphenylphosphine)-palladium(II)(6 mg, 0.01 mmol) and K₂CO₃ (26 mg, 0.19 mmol) were mixed in DME/EtOH/H₂O 4:1:1 (1 ml) under an atmosphere of nitrogen. The reaction was run at 120 °C for 20 minutes in a microwave reactor. DCM/EtOAc and water were added, the phases were separated and the solvents were evaporated. The residue was purified by prep HPLC to afford 10mg (34%) of the title compound. LC/MS (20-100% CH₃CN:0.05% HCOOH(aq) gradient over 5 min): 2.48 min. 345 M+H.

Step 5: preparation of cyclopentyl(4-(2-(diphenylmethyleneamino)-5-methyl-5H-pyrrolo[3,2-d]pyrimidin-7-yl)-5,6-dihydropyridin-1(2H)-yl)methanone. Pd(OAc)2 (1 mg, 0.15 mmol) and BINAP (4 mg, 0.22 mmol) were dissolved in degassed dioxane (0.5 ml) and stirred for 5 min. This solution was added to a mixture of (4-(2-chloro-5-methyl-5H-pyrrolo[3,2-d]pyrimidin-7-yl)-5,6-dihydropyridin-1(2H)-yl)(cyclopentyl)methanone (10 mg, 0.03 mmol), diphenylmethanimine (16 mg, 0.09 mmol) and sodium tert-butoxide (6 mg, 0.06 mmol) in degassed dioxane (0.5 ml). The mixture was heated under microwave irradiation for 30 min at 140 °C. DCM/EtOAc and a small amount of water was added and the phases were separated. The solvents were evaporated and the crude product was purified by preparative HPLC to afford 10 mg (71%) of the title compound. LC/MS (20-100% CH₃CN:0.05% HCOOH(aq) gradient over 2 min): 0.97 min. 490 M+H.

Step 6: preparation of (4-(2-amino-5-methyl-5H-pyrrolo[3,2-d]pyrimidin-7-yl)piperidin-1-yl)(cyclopentyl)methanone. Cyclopentyl(4-(2-(diphenylmethyleneamino)-5-methyl-5H-pyrrolo[3,2-d]pyrimidin-7-yl)-5,6-dihydropyridin-1(2H)-yl)methanone (10 mg, 0.02 mmol), ammonium formate (52 mg, 0.82 mmol) and Pd black (1 mg, 0.01 mmol) were mixed in THF/NMP 5:1 (0.5 ml). The mixture was heated under microwave irradiation at 150 °C for 60 minutes. The mixture was filtered through a plug of celite, the solvents were evaporated (except NMP). The crude mixture was directly used in the subsequent step without further purification. Yield not calculated. LC/MS (20-100% CH₃CN:0.05% HCOOH(aq) gradient over 5 min): 1.48 min. 328 M+H.

Step 7: preparation of 4-cyano-N-(7-(1-(cyclopentanecarbonyl)piperidin-4-yl)-5-methyl-5H-pyrrolo[3,2-d]pyrimidin-2-yl)picolinamide. To the crude (4-(2-amino-5-methyl-5H-pyrrolo[3,2-d]pyrimidin-7-yl)piperidin-1-yl)(cyclopentyl)methanone from the previous step was added DCM (0.5 ml), Et₃N (30 µl) and 4-cyanopicolinoyl chloride (12mg, 0.07). The reaction mixture was stirred at room temperature for 30 min, whereupon the solvents were evaporated. The residue was purified by preparative HPLC to provide 0.4 mg (4% over two steps) of the title compound. LC/MS (20-100% CH₃CN:0.05% HCOOH(aq) gradient over 5 min): 1.98 min. 458 M+H. ¹H NMR (500 MHz, (CD3)2CO) δ ppm 9.02 (d, J=5.0,1H), 8.86 (s, 1H), 8.54 (s, 1H), 8.11 (dd, J=5.0, 1.8, 1H), 7.58 (s, 1H), 4.68 (m, 1H), 4.18 (m, 1H), 3.96 (s, 3H), 3.23 (m, 2H), 3.07 (m, 1H), 2.24, m, 1H), 1.90-1.50 (m, 12H).

### Reference Example 57

### Preparation of 4-cyano-N-(3-(1-(cyclopentanecarbonyl)piperidin-4-yl)-1-methyl-1H-pyrrolo[2,3-c]pyridin-5-yl)picolinamide

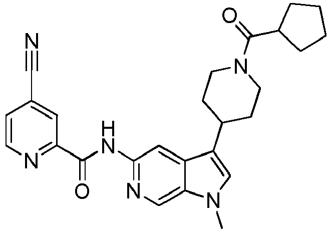

Step 1: preparation of (E)-N'-(4-((E)-2-(dimethylamino)vinyl)-5-nitropyridin-2-yl)-N,N-dimethylformimidamide. To a stirred solution of 4-methyl-5-nitropyridin-2-amine (500 mg, 3.26 mmol) in DMF (5 ml) was added 1,1-dimethoxy-N,N-dimethylmethanamine (4.3 ml, 33 mmol) and the mixture was heated to 110 °C for 24 h. Evaporation of the solvents in vacuo provided 850 mg (99%) of the title compound. The crude product was used in the next step without further purification.

Step 2: preparation of (E)-N,N-dimethyl-N'-(1H-pyrrolo[2,3-c]pyridin-5-yl)formimidamide. The crude (E)-N'-(4-((E)-2-(dimethylamino)vinyl)-5-nitropyridin-2-yl)-N,N-dimethylformimidamide (850 mg g, 3.23 mmol) was dissolved in EtOH (9 ml) and Pd/C (22 mg, 10%) was added. The mixture was hydrogenated in a hydrogenation apparatus for four hours at 40 psi. The mixture was passed through a plug of celite and the fitrate was evaporated. The residue was purified by chromatography on neutral alumina to provide 582 mg (96% over two steps) of the title compound. LC/MS (20-100% CH₃CN:0.05% HCOOH(aq) gradient over 5 min): 1.37 min. 189 M+H.

Step 3: preparation of (E)-N'-(3-iodo-1H-pyrrolo[2,3-c]pyridin-5-yl)-N,N-dimethylformimidamide. To a stirred solution of (E)-N,N-dimethyl-N'-(1H-pyrrolo[2,3-c]pyridin-5-yl)formimidamide (470 mg, 2.50 mmol) in DMF at 0 °C was added N-iodosuccinimide (590 mg, 2.62 mmol), and the mixture was stirred for 18 hours at room temperature. The solvent was evaporated in vacuo and the residue was purified by neutral alumina chromatography to provide 407 mg (52%) of the title compound. LC/MS (20-100% CH₃CN:0.05% HCOOH(aq) gradient over 5 min): 2.62 min. 315 M+H.

Step 4: (E)-N'-(3-iodo-1-methyl-1H-pyrrolo[2,3-c]pyridin-5-yl)-N,N-dimethylformimidamide. To a stirred solution of (E)-N'-(3-iodo-1H-pyrrolo[2,3-c]pyridin-5-yl)-N,N-dimethylformimidamide (407 mg, 1.30 mmol) in DCM (22 ml), tetrabutylammonium bromide (80 mg, 0.25 mmol), NaOH (4 ml, 2M) and Mel (200 µl, 3.25 mmol) were added and the mixture was stirred for 16 hours at room temperature. Water, DCM and EtOAc were added and the phases were separated. The solvents were evaporated and the residue was purified by neutral alumina chromatography to provide 131 mg (31%) of the title compound. LC/MS (20-100% CH₃CN:0.05% HCOOH(aq) gradient over 5 min): 1.48 min. 329 M+H.

Step 5: preparation of N-(3-(1-(cyclopentanecarbonyl)-1,2,3,6-tetrahydropyridin-4-yl)-1-methyl-1H-pyrrolo[2,3-c]pyridin-5-yl)formamide. A mixture of (E)-N'-(3-iodo-1-methyl-1H-pyrrolo[2,3-c]pyridin-5-yl)-N,N-dimethylformimidamide (31 mg, 0.09 mmol), cyclopentyl(4-(4,4,5,5-tetramethyl-1,3,2-dioxaborolan-2-yl)-5,6-dihydropyridin-1 (2H)-yl)methanone (38 mg, 0.12 mmol), tetrakis(triphenylphosphine)Pd(0)(7 mg, 0.01 mmol) and K₂CO₃ (29 mg, 0.21 mmol) in a mixture of DMF/water 8:1 (1 ml) was degassed and flushed with nitrogen gas. The reaction mixture was heated under microwave irradiation at 120 °C for 20 minutes. Water and DCM were added and the phases were separated. The solvents were evaporated and the residue was purified by preparative HPLC to provide 12 mg (36%) of the title compound. LC/MS (20-100% CH₃CN:0.05% HCOOH(aq) gradient over 5 min): 2.90 min. 353 M+H.

Step 6: preparation of (4-(5-amino-1-methyl-1H-pyrrolo[2,3-c]pyridin-3-yl)piperidin-1-yl)(cyclopentyl)methanone. N-(3-(1-(cyclopentanecarbonyl)-1,2,3,6-tetrahydropyridin-4-yl)-1-methyl-1H-pyrrolo[2,3-c]pyridin-5-yl)formamide (6.0 mg, 0.02 mmol), ammonium formate (43 mg, 0.68 mmol) and palladium black (1.0 mg, 0.01 mmol) were mixed in DMF/NMP 5:1 (1 ml). The reaction mixture was heated under microwave irradiation at 150 °C for 60 minutes. The mixture was filtered through a plug of celite, the solvents were evaporated (except NMP) to provide the crude title compound. To the residue was added HCl (1 ml, 2M) and MeOH (1 ml) and the mixture was stirred for 1 hour, whereupon EtOAc was added. The aqueous phase was basified with 2M NaOH and again extracted. The phases were separated and the solvents were evaporated to provide the title compound which was used directly in the subsequent step without further purification. Yield not calculated. LC/MS (5-50% CH₃CN:0.05% HCOOH(aq) gradient over 5 min): 2.20 min. 327 M+H.

Step 7: preparation of 4-cyano-N-(3-(1-(cyclopentanecarbonyl)piperidin-4-yl)-1-methyl-1H-pyrrolo[3,2-b]pyridin-5-yl)picolinamide. To the crude (4-(5-amino-1-methyl-1H-pyrrolo[3,2-b]pyridin-3-yl)piperidin-1-yl)(cyclopentyl)methanone from step 6 was added DCM (0.5mL), Et₃N (30µL, 0.22 mmol) and 4-cyanopicolinoyl chloride (11mg, 0.07 mmol). The mixture was stirred at room temperature for 30 minutes, whereupon the solvents were evaporated. The residue was purified by preparative HPLC to provide 0.7 mg (9% over two steps) of the title compound. LC/MS (5-50% CH₃CN:0.05% HCOOH(aq) gradient over 5 min): 3.75 min. 457 M+H. ¹H NMR (500 MHz, (CD₃)₂CO) δ ppm 9.02 (d, J=5.0, 1H), 8.62 (s, 1H), 8.57 (d, J=10, 2H), 8.10 (dd, J=5.0, 1.70, 1H), 7.34 (s, 1H), 4.73 (m, 1H), 4.22 (m, 1H), 3.93 (s, 3H), 3.31 (m, 1H), 3.19 (m, 1H), 3.08 (m, 1H), 2.16 (m, 1H), 1.94-1.52 (m, 12H).

### Reference Example 58

### Preparation of 3-cyano-N-(3-(1-(cyclopentanecarbonyl)piperidin-4-yl)-1-methyl-1H-indol-5-yl)piperidine-1-carboxamide

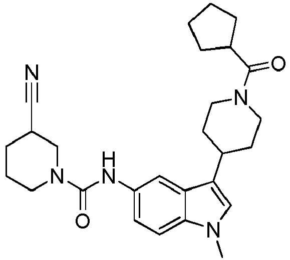

Step 1: preparation of cyclopentyl(4-(5-isocyanato-1-methyl-1H-indol-3-yl)piperidin-1-yl)methanone. Triphosgene (7.3 mg, 0.02 mmol) was added to a stirred solution of (4-(5-amino-1-methyl-1H-indol-3-yl)piperidin-1-yl)(cyclopentyl)methanone (20 mg, 0.06 mmol) and triethylamine (28 µl, 0.22 mmol) in DCM (1 ml) at room temperature. The mixture was heated at reflux for 16 hours. The solvents were evaporated, whereupon the residue was resuspended in diethylether (5 ml) and stirred at room temperature for 5 min. The ensuing crystals were removed by filtration and the filtrate was concentrated in vacuo to afford the crude title compound which was employed for the next step without further purification.

Step 2: preparation of 3-cyano-N-(3-(1-(cyclopentanecarbonyl)piperidin-4-yl)-1-methyl-1H-indol-5-yl)piperidine-1-carboxamide. To the crude cyclopentyl(4-(5-isocyanato-1-methyl-1H-indol-3-yl)piperidin-1-yl)methanone in THF (1 ml) was added triethylamine (28 µl, 0.22 mmol) and piperidine-3-carbonitrile (14 mg, 0.12 mmol). The mixture was stirred at room temperature for one hour, whereupon the reaction mixture was filtered through a syringe filter and then purified by preparative HPLC to afford 5.27mg (19% over two steps) of the title compound. LC/MS (20-100% CH₃CN:0.05% HCOOH(aq) gradient over 5 min): 2.40 min. 462 M+H. ¹H NMR (500 MHz, (CD3)2CO δ ppm 7.93 (s, 1H), 7.76 (d, J=2.0, 1H), 7.25 (dd, J=8.71, 2.0, 1H), 7.21 (d, J=8.71, 1H), 6.97 (s, 1H), 4.68 (m, 1H), 4.17 (m, 1H), 3.90 (dd, J=13.5, 3.80, 1H), 4.17 (m, 1H), 3.90 (m, 1H), 3.73 (s, 3H), 3.70-3.60 (m, 2H), 3.47 (m, 1H), 3.23 (m, 1H), 3.05 (m, 2H), 2.70 (m, 1H), 1.90 (m, 1H), 1.94-1.49 (m, 14 H).

The following examples were prepared analogous to Reference Example 58.

| **Example** | **Structure** | **Name** | **MS (M+H)** |
|---|---|---|---|
| **R59** | 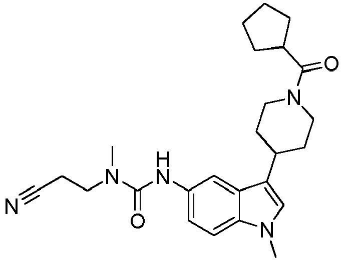 | 1-(2-cyanoethyl)-3-{3-[1-(cyclopentylcarbonyl)piperidin-4-yl]-1-methyl-1H-indol-5-yl}-1-methylurea | 436.3 |
| **R60** | 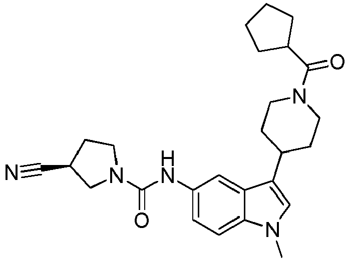 | (3S)-3-cyano-N-{3-[1-(cyclopentylcarbonyl)pi peridi n-4-yl]-1-methyl-1H-indol-5-yl}pyrrolidine-1-carboxamide | 448.3 |
| **R61** | 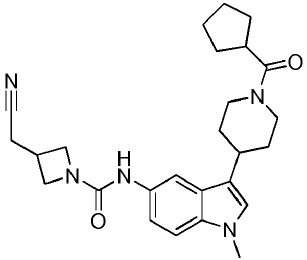 | 3-(cyanomethyl)-N-{3-[1-(cyclopentylcarbonyl)piperidin-4-yl]-1-methyl-1H-indol-5-yl}azetidine-1-carboxamide | 448.3 |
| **R62** | 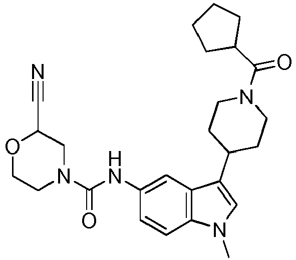 | 2-cyano-N-{3-[1-(cyclopentylcarbonyl)piperidin-4-yl]-1-methyl-1H-indol-5-yl}morpholine-4-carboxamide | 464.4 |
| **R63** | 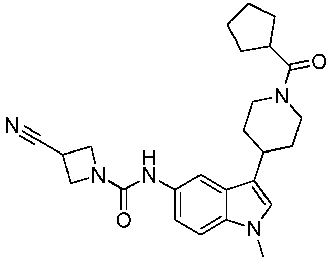 | 3-cyano-N-{3-[1-(cyclopentylcarbonyl)piperidin-4-yl]-1-methyl-1H-indol-5-yl}azetidine-1-carboxamide | 434.4 |

### Reference Example 64

### Preparation of N-(3-(1-(cyclopentylcarbonyl)piperidin-4-yl)-1-methyl-1H-indol-5-yl)-4-fluorobenzenesulfonamide

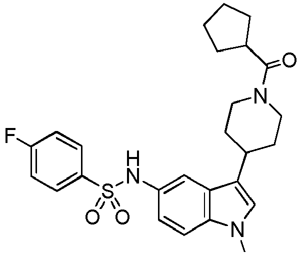

Step 1: Methyl-5-nitro-3-(1,2,3,6-tetrahydropyridin-4-yl)-1H-indole (Int-1). To a solution of tert-butyl 4-(5-nitro-1H-indol-3-yl)-5,6-dihydropyridine-1(2H)-carboxylate (prepared in Reference Example **1,** 2.0g, 5.60 mmol) in 10 mL of methanol was added 4M HCl in dioxane (10 mL) at 0 °C. The reaction mixture was allowed to warm up to room temperature and stirred for overnight. The progress of reaction was monitored TLC (10% methanol in DCM). After completion, reaction mixture was concentrated in vacuo to give a crude compound which was neutralized with sat. NaHCO₃ solution and then extracted with 10% methanol in DCM. The combined extracts were dried over (Na₂SO₄), filtered and concentrated in vacuum to give the title compound (1.2 g, 85.7%) as a brown solid. ¹H NMR (400 MHz, CDCl₃): δ 8.84 (s, 1H), 8.13 (d, J = 9.2 Hz, 1H), 7.29 (d, J = 9.2 Hz, 1H), 7.14 (s, 1H), 6.27 (s, 1H), 3.83 (s, 3H), 3.62 (br.s, 2H), 3.16 (t, J = 5.2 Hz, 2H), 2.47 (br.s, 2H). LCMS: m/e 257.95 [M+H]+

Step 2: Cyclopentyl (4-(1-methyl-5-nitro-1H-indol-3-yl)-5,6-dihydropyridin-1(2H)-yl)methanone (Int-2). To a solution of methyl-5-nitro-3-(1,2,3,6-tetrahydropyridin-4-yl)-1H-indole (Int-1; 1.2 g, 4.66 mmol) in DCM (20 mL) was added TEA (2 mL, 14 mmol) followed by addition of cyclopentanecarbonyl chloride (0.810 g) in DCM (5 mL) at 0 °C. The progress of reaction was monitored TLC (5% methanol in DCM). After completion, reaction mixture was quenched with water and extracted by using DCM. The combined extracts were dried (Na₂SO₄), filtered and concentrated in vacuum to give a crude compound which was washed with diethyl ether and hexane to afford the title compound (1.7 g, 97.7%) as a light yellow colored solid. ¹H NMR (400 MHz, CDCl₃): δ 8.79 (d, J = 14.0 Hz, 1H), 8.15 (d, J = 8.8 Hz, 1H), 7.35-7.32 (m, 1H), 7.15 (d, J = 14.4 Hz, 1H), 6.21 (s, 1H), 4.31 (d, J = 9.2 Hz, 2H), 3.90-3.78 (m, 5H), 3.12-2.96 (m, 2H), 2.61-2.55 (m, 2H), 1.89-1.60 (m, 6H), 1.40 (t, J = 7.6 Hz, 1H); LCMS: m/e 353.90 [M+H]+

Step 3: 4-(5-Amino-1-methyl-1H-indol-3-yl)piperidin-1-yl)(cyclopentyl)methanone (Int-3). To a solution of cyclopentyl (4-(1-methyl-5-nitro-1H-indol-3-yl)-5,6-dihydropyridin-1(2H)-yl)methanone (Int-2; 4.5 g, 12.74 mmol) in 50 mL of methanol was added Pd/C (400mg) and reaction mixture was stirred under 50 psi of H2 at 40 °C overnight. The progress of reaction was monitored by TLC (50% ethyl acetate in hexane). The mixture was filtered through Celite and the filtrate was concentrated in vacuum to give the title compound. ¹H NMR (400 MHz, CDCl₃): δ 7.14-7.12 (m, 2H), 6.87 (d, J = 8.0 Hz, 1H), 6.75 (s, 1H), 4.74 (d, J = 12.0 Hz, 1H), 4.04 (d, J = 12.8 Hz, 1H), 3.68 (s, 3H), 3.49 (q, J = 7.2 Hz, 1H), 3.19-2.67 (m, 4H), 2.09-1.56 (m, 10H), 1.21 (t, J = 7.2 Hz, 2H); LCMS: m/e 325.10 [M+H]+

Step 4: N-(3-(1-(cyclopentylcarbonyl)piperidin-4-yl)-1-methyl-1H-indol-5-yl)-4-fluorobenzenesulfonamide. To a stirred solution of of (4-(5-amino-1-methyl-1H-indol-3-yl)piperidin-1-yl)(cyclopentyl)methanone (Int-3; 100 mg, 1eq.) in DCM (3 mL) were added pyridine (5 eq.), DMAP (0.1 eq.) and sulphonyl chloride (1.2-2 eq.) and the reaction mixture was stirred at room temperature for 1-5 h. LC/MS after 1 h showed almost full conversion to the desired product. The conversions were in the range of 70-80% by LCMS (Starting material was 85% pure by LCMS). After completion, reaction mixture was diluted with water and extracted with DCM. The combined organic layer was dried (Na₂SO₄), filtered and concentrated to give crude material which was purified by column chromatography followed by triturating with ether to provide 19 mg of the title compound. ¹H NMR (400 MHZ, CDCl₃): δ 7.69-7.65 (m, 2H), 7.28-7.04 (m, 4H), 6.82-6.81 (m, 2H), 6.31 (s, 1H), 4.76 (d, J=12.0 Hz, 1H), 4.05 (d, J=12.0 Hz, 1H), 3.71 (s, 3H), 3.17 (t, J=12.4 Hz, 1H), 2.96-2.92 (m, 2H), 2.70 (t, J=12.4 Hz, 1H), 2.04-1.54 (m, 12H). LCMS: m/e 484.20 [M+H]+

The following examples were prepared analogous to Reference Example 64.

| **Example** | **Structure** | **Name** | **MS (M+H)** |
|---|---|---|---|
| **R65** | 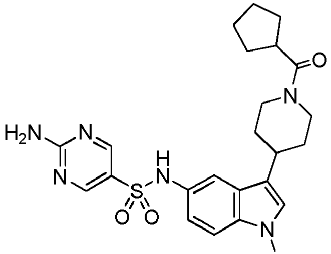 | 2-amino-N-{3-[1-(cyclopentylcarbonyl)piperidin-4-yl]-1-methyl-1H-indol-5-yl}pyrimidine-5-sulfonamide | 483.25 |
| **R66** | 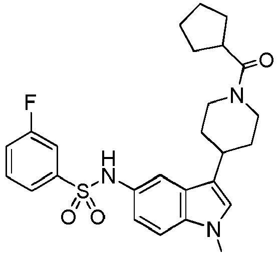 | N-{3-[1-(cyclopentylcarbonyl)piperidin-4-yl]-1-methyl-1H-indol-5-yl}-3-fluorobenzenesulfonamide | 484.20 |
| **R67** | 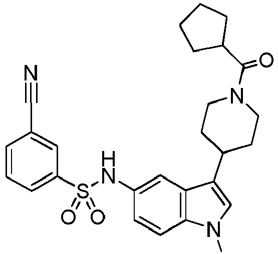 | 3-cyano-N-{3-[1-(cyclopentylcarbonyl)piperidin-4-yl]-1-methyl-1H-indol-5-yl}benzenesulfonamide | 491.15 |
| **R68** | 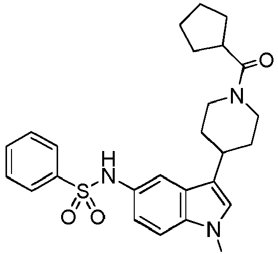 | N-{3-[1-(cyclopentylcarbonyl)piperidin-4-yl]-1-methyl-1H-indol-5-yl}benzenesulfonamide | 466.20 |
| **R69** | 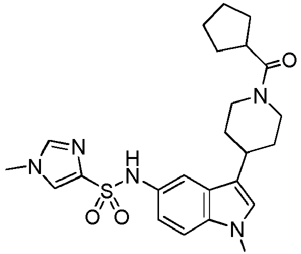 | N-{3-[1-(cyclopentylcarbonyl)piperidin-4-yl]-1-methyl-1H-indol-5-yl}-1-methyl-1H-imidazole-4-sulfonamide | 470.20 |
| **R70** | 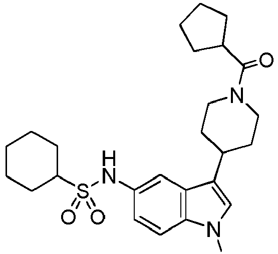 | N-{3-[1-(cyclopentylcarbonyl)piperidin-4-yl]-1-methyl-1H-indol-5-yl}cyclohexanesulfonamide | 472.20 |
| **R71** | 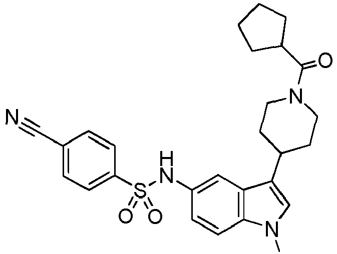 | 4-cyano-N-{3-[1-(cyclopentylcarbonyl)piperidin-4-yl]-1-methyl-1H-indol-5-yl}benzenesulfonamide | 491.15 |
| **R72** | 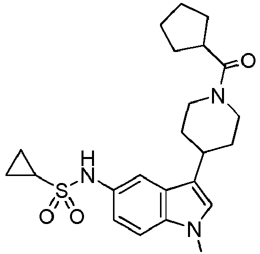 | N-{3-[1-(cyclopentylcarbonyl)piperidin-4-yl]-1-methyl-1H-indol-5-yl}cyclopropanesulfonamide | 430.15 |
| **R73** | 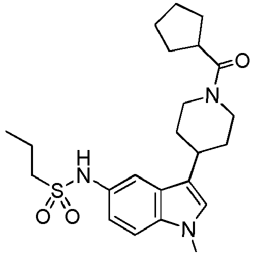 | N-{3-[1-(cyclopentylcarbonyl)piperidin-4-yl]-1-methyl-1H-indol-5-yl}propane-1-sulfonamide | 432.2 |
| **R74** | 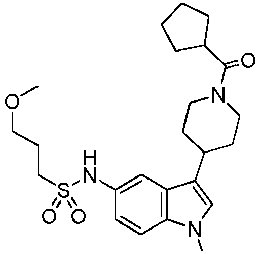 | N-{3-[1-(cyclopentylcarbonyl)piperidin-4-yl]-1-methyl-1H-indol-5-yl}-3-methoxypropane-1-sulfonamide | 462.25 |
| **R75** | 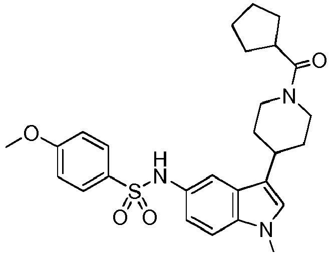 | N-{3-[1-(cyclopentylcarbonyl)piperidin-4-yl]-1-methyl-1H-indol-5-yl}-4-methoxybenzenesulfonamide) | 496.20 |
| **R76** | 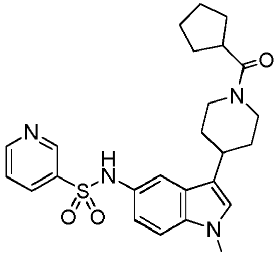 | N-{3-[1-(cyclopentylcarbonyl)piperidin-4-yl]-1-methyl-1H-indol-5-yl}pyridine-3-sulfonamide | 467.20 |
| **R77** | 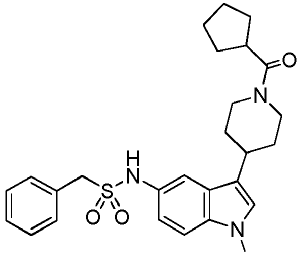 | N-{3-[1-(cyclopentylcarbonyl)piperidin-4-yl]-1-methyl-1H-indol-5-yl}-1-phenyl methanesulfonamide | 480.20 |

### Reference Example 78

### Preparation of N-(3-(1-(1H-Pyrazole-4-carbonyl)piperidin-3-yl)-1-methyl-1H-indol-5-yl)-4-cyanopicolinamide

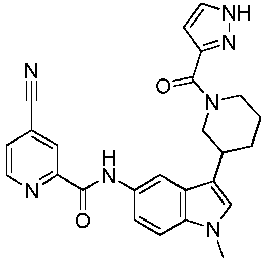

Step 1: tert-Butyl 5-(5-nitro-1H-indol-3-yl)-3,4-dihydropyridine-1(2H)-carboxylate. To freshly prepared sodium methoxide (5 g, 30.9 mmol) in MeOH (100 mL) were added 5-nitroindole (5 g, 15.4 mmol) and tert-butyl 3-oxopiperidine-1-carboxylate (11 g, 55.55 mmol). The reaction mixture was heated to reflux for 24 h. The progress of reaction was monitored by TLC (40% ethyl acetate in hexane). After most of the starting material was consumed (By TLC), reaction mixture was cooled to room temperature and concentrated under vacuum. The residue obtained was diluted with water (50 mL), extracted by using ethyl acetate (3x100 mL), dried (Na₂SO₄), filtered and concentrated to obtain crude material. The crude compound was purified by using column chromatography (silica gel, 100-200 mesh) to afford the title compound (3.39 g, 32%; 1.7 g 5-nitroindole was recovered) as a brown solid. ¹H NMR (400 MHz, DMSO-d6): δ 11.85 (s, 1H), 8.69-8.65 (m, 1H), 8.02 (dd, J = 2.4, 9.2 Hz, 1H), 7.63-7.41 (m, 3H), 3.60-3.50 (m, 2H), 2.45-2.40 (m, 2H), 1.96-1.90 (m, 2H), 1.55 (s, 5H), 1.50 (s, 4H); LCMS: m/e 244 [M-Boc]+

Step 2: tert-Butyl 5-(1-methyl-5-nitro-1H-indol-3-yl)-3,4-dihydropyridine-1(2H)-carboxylate. To a solution of tert-butyl 5-(5-nitro-1H-indol-3-yl)-3,4-dihydropyridine-1(2H)-carboxylate (1 g, 2.92 mmol) in 15 mL of THF was added NaH (466 mg, 11.66 mmol, 60% w/w in mineral oil) at 0 °C. The reaction mixture was stirred at room temperature for 1 h and Mel (0.73 mL, 11.66 mmol) was added drop wise at 0 °C. The reaction mixture was then allowed to stir at room temperature for overnight. The progress of reaction was monitored by TLC (40% ethyl acetate in hexane). After completion, reaction mixture was quenched by addition of ice-water and then extracted by using ethyl acetate (2x50 mL). The combined organic phases were dried (Na₂SO₄), filtered and concentrated to obtain the title compound (725 mg, 70%) as a brown solid. ¹H NMR (400 MHz, DMSO-d6): δ 8.70 (s, 1H), 8.08-8.12 (m, 1H), 7.70-7.65 (m, 2H), 7.45 (s, 1H), 3.84 (s, 3H), 3.58-3.51 (m, 2H), 2.42-2.33 (m, 2H), 1.94-1.91 (m, 2H), 1.52-1.38 (s, 9H); LCMS: m/e 258 [M-Boc]+

Step 3: tert-Butyl 3-(5-amino-1-methyl-1H-indol-3-yl)piperidine-1-carboxylate. To a solution of tert-butyl 5-(1-methyl-5-nitro-1H-indol-3-yl)-3,4-dihydropyridine-1(2H)-carboxylate (700 mg, 1.96 mmol) in 20 mL of methanol was added Pd/C (100mg) and the reaction mixture was heated at 40 °C under H2 atmosphere (Balloon pressure) for 7 h. The progress of reaction was monitored by TLC (50% ethyl acetate in hexane). After completion, the reaction mixture was filtered through Celite, washed with methanol and the combined filtrate was concentrated in vacuum to obtain the title compound (480 mg, 74%) as a light brown solid. ¹H NMR (400 MHz, DMSO-d6): δ 7.05 (d, J = 8.8 Hz, 1H), 6.92 (s, 1H), 6.70 (s, 1H), 6.52 (d, J = 8.8 Hz, 1H), 4.51-3.94 (m, 3H), 3.61 (s, 3H), 2.76-2.67 (m, 3H), 1.99 (d, J = 11.6 Hz, 1H), 1.73-1.46 (m, 4H), 1.42 (s, 9H). LCMS: m/e 352.10 [M+Na]+

Step 4: tert-Butyl 3-(5-(4-cyanopicolinamido)-1-methyl-1H-indol-3-yl)piperidine-1-carboxylate. To a solution of tert-butyl 3-(5-amino-1-methyl-1H-indol-3-yl)piperidine-1-carboxylate (475 mg, 1.44 mmol) in DCM (10 mL) was added TEA (0.9 mL, 6.49 mmol) followed by addition of 4-cyanopicolinoyl chloride (217 mg, 1.29 mmol) in DCM (5 mL) at 0 °C. The progress of reaction was monitored TLC (50% ethylacetate in hexane). After completion, reaction mixture was quenched with water and extracted by using DCM. The combined extracts were dried (Na₂SO₄), filtered and concentrated in vacuo to give a crude compound which was subjected to column chromatography (Silica gel, 100-200 mesh) to afford the title compound (444 mg, 67%) as a light brown colored solid. ¹H NMR (400 MHz, CDCl₃): δ 9.89 (s, 1H), 8.82 (d, J = 4.8 Hz, 1H), 8.55 (s, 1H), 8.05 (s, 1H), 7.72 (d, J = 4.8 Hz, 1H), 7.61 (d, J = 8.8 Hz, 1H), 7.29 (d, J = 8.8 Hz, 1H), 6.89 (s, 1H), 4.44-4.06 (m, 2H), 3.76 (s, 3H), 3.04-2.86 (m, 3H), 2.19-1.59 (m, 4H), 1.49 (s, 9H); LCMS: m/e 365.05 [M-Boc]+

Step 5: 4-Cyano-N-(1-methyl-3-(piperidin-3-yl)-1H-indol-5-yl) picolinamide. To a solution of tert-Butyl 3-(5-(4-cyanopicolinamido)-1-methyl-1H-indol-3-yl)piperidine-1-carboxylate (430 mg, 0.93 mmol) in 10 mL of methanol was added 4M HCl in dioxane (8 mL) at 0 °C. The reaction mixture was allowed to warm up to room temperature and stirred overnight. The progress of reaction was monitored TLC (10% methanol in DCM). After completion, reaction mixture was concentrated in vacuo to give a crude compound which was neutralized with sat. NaHCO₃ solution and then extracted with 10% methanol in DCM. The combined extracts were dried (Na₂SO₄), filtered and concentrated in vacuo to give the title compound (275 mg, 88%) as a brown solid. ¹H NMR (400 MHz, DMSO-d6): δ 10.60 (s, 1H), 8.89 (d, J = 8.4 Hz, 1H), 8.47 (s, 1H), 8.09-8.08 (m, 1H), 8.04 (d, J = 4.8 Hz, 1H), 7.50 (d, J = 8.8 Hz, 1H), 7.33 (d, J = 8.8 Hz, 1H), 7.03 (s, 1H), 3.89 (s, 3H), 3.11-2.43 (m, 6H, merged), 2.10-1.97 (m, 1H), 1.63-1.51 (m, 3H); LCMS: m/e 360 [M+H]+

Step 6: N-(3-(1-(1H-Pyrazole-4-carbonyl)piperidin-3-yl)-1-methyl-1H-indol-5-yl)-4-cyanopicolinamide. A solution of 1H-pyrazole-3-carboxylic acid (1.1eq.), DIPEA (3 eq.) and HATU (3.3 eq.) in DMF (1 mL) was stirred at room temperature for 1h. 4-Cyano-N-(1-methyl-3-(piperidin-3-yl)-1H-indol-5-yl) picolinamide (1eq.) was then added to the reaction mixture and stirred at room temperature for 24 hr. The crude product was purified by reverse phase HPLC to afford the title compound. ¹H NMR (400 MHZ, CDCl₃): δ 10.02 (s, 1H), 8.93 (s, 1H), 8.85 (d, J=4.4 Hz, 1H), 8.59 (s, 1H), 7.75 (d, J=4.4 Hz, 1H), 7.62 (d, J= 2.4 Hz, 1H), 7.27 (d, J=8.8 Hz, 1H), 7.07 (d, J=8.8 Hz, 1H), 6.94 (s, 1H), 677 (s, 1H), 5.19 (d, J=12.4 Hz, 1H), 4.87 (d, J=12.4 Hz, 1H), 3.76 (s, 3H), 3.50-3.48 (m, 1H), 2.78-2.68 (m, 2H), 2.22 (d, J=9.2 Hz, 1H), 1.94-1.79 (m, 4H). LCMS: m/e 454.20 [M+H]+.

### Reference Example 79

### Preparation of 3-cyano-N-(3-(1-(cyclopentylcarbonyl)pyrrolidin-3-yl)-1-methyl-1H-indol-5-yl)benzamide

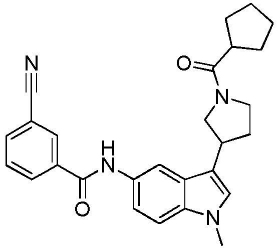

Step 1: preparation of tert-butyl 3-(4,4,5,5-tetramethyl-1,3,2-dioxaborolan-2-yl)-1H-pyrrole-1-carboxylate. A mixture of the compound N-Boc-pyrrole (6 g, 35.9 mmol) and Bis (pinacolato)diboron (53 mg, 5.3 mmol) in THF (50 mL) was degassed for 3 times and then [Ir(OMe)cod]₂ (714 mg, 1.1 mmol) and 4,4-di-tert-butyl-2,2-bipyridine (48 mg, 0.018 mmol) was added to the above mixture. The mixture was degassed for another 3 times and heated to reflux 5 h. The progress of reaction was monitored by TLC (10% ethyl acetate in hexane). After completion, reaction mixture was concentrated in vacuum to give a crude compound which was purified by column chromatography on silica gel (100-200 mesh) to afford the title compound (6.5 g, 61.90 %). ¹H NMR (400 MHz, CDCl₃): δ 7.65 (s, 2H), 6.47 (s, 1H), 1.58 (s, 9H), 1.32 (12H). LCMS: m/e 294.0 [M+H]+

Step 2: 3-Bromo-5-nitro-1H-indole. To a solution of 5-nitro-indole (200 mg, 1.2 mmol) in pyridine (5 mL) was added Py.HBr₃ (474 mg, 1.4 mmol) at -10 °C and mixture was stirred for 10 min. Then reaction mixture was quenched by addition of water at 0 °C and then the reaction mixture was extracted with diethyl ether. The organic layer was washed successively with 6N HCl (20 mL), 5% NaHCO₃ (20 mL) followed by brine. The combined organic layers were dried (Na₂SO₄), filtered and concentrated to obtain the title compound (200 mg, 67%) as yellow colored solid. ¹H NMR (400 MHz, CDCl₃): δ 8.58-8.57 (m, 2H), 8.16 (dd, J = 2.0, 8.8 Hz, 1H), 7.44 (d, J = 8.8 Hz, 1H), 7.41 (d, J = 2.8 Hz, 1H). LCMS: m/e 240.80 [M+H]+

Step 3: tert-Butyl 3-(5-nitro-1H-indol-3-yl)-1H-pyrrole-1-carboxylate. To a solution of the 3-bromo-5-nitro-1H-indole (200 mg, 0.83 mmol) and tert-butyl 3-(4,4,5,5-tetramethyl-1,3,2-dioxaborolan-2-yl)-1H-pyrrole-1-carboxylate (267 mg, 0.91 mmol) in THF (10 mL) was added Na₂CO₃ (175 mg, 1.7 mmol) in H₂O (3 mL). The mixture was degassed and refilled with nitrogen for 3 times. Then Pd (PPh₃)₂Cl₂ (47 mg, 0.065 mmol) was added to the above mixture and the mixture was degassed for another 3 times. The mixture was heated to reflux for overnight. Then the reaction mixture was concentrated in vacuo and the residue was extracted with ethyl acetate. The combined organic layer was dried (Na₂SO₄), and concentrated to get crude product, which was purified by column chromatography on 100-200 mesh silica gel to afford the title compound (100 mg, 37%) as yellow solid. ¹H NMR (400 MHz, CDCl₃): δ 8.81 (d, J = 1.2 Hz, 1H), 8.50 (br.s, 1H), 8.14-8.17 (dd, J = 2.0, 8.8 Hz, 1H), 7.56 (s, 1H), 7.36-7.46 (m, 3H), 6.53-6.52 (m, 1H), 1.65 (s, 9H). LCMS: m/e 327.85 [M+H]+

Step 4: tert-Butyl 3-(1-methyl-5-nitro-1H-indol-3-yl)-1H-pyrrole-1-carboxylate. To a solution of tert-butyl 3-(5-nitro-1H-indol-3-yl)-1H-pyrrole-1-carboxylate (650 mg, 1.98 mmol) in THF (10 mL) was added NaH (190 mg, 7.9 mmol, 60% w/w in mineral oil) at 0°C and reaction mixture was stirred at same the same temperature for 1h. To this was added methyl iodide (1.12 g, 7.9 mmol) and then reaction mixture was stirred at room temperature for 3h. The progress of reaction was monitored by TLC. After completion, reaction mixture was concentrated in vacuum, diluted with water and extracted with ethyl acetate. The combined organic layer was dried (Na₂SO₄), filtered and concentrated to give crude product which was purified by column chromatography on 100-200 mesh silica gel to afford the title compound (400 mg, 59%) as a yellow solid. ¹H NMR (400 MHz, DMSO-d6): δ 8.78 (d, J = 1.6 Hz, 1H), 8.15-8.18 (dd, J = 1.6, 9.2 Hz, 1H), 7.53 (br.s, 1H), 7.26-7.53 (m, 3H), 6.51 (s, 1H), 3.88 (s, 3H), 1.65 (s, 9H); LCMS: m/e 341.85 [M+H]

Step 5: tert-Butyl 3-(5-amino-1-methyl-1H-indol-3-yl)pyrrolidine-1-carboxylate. To a solution of tert-Butyl 3-(1-methyl-5-nitro-1H-indol-3-yl)-1H-pyrrole-1-carboxylate (500 mg, 1.15 mmol) in 20 mL of methanol was added Pd/C (100 mg) and the reaction mixture was heated under 50 psi of H₂ atmosphere at 40°C for 12 h. The progress of reaction was monitored by TLC (5% methanol in DCM). After 12 h, LCMS showed only reduction of nitro group and pyrrole ring intact. Then reaction was further continued with excess Pd/C (400 mg) at 45 °C under 100 psi for 12 hrs, when LCMS shows 49% formation of product. Reaction was cooled and filtered through Celite and concentrated to give 220 mg of the title compound which was directly used for next reaction. ¹H NMR (400 MHz, MeOD): δ 7.15 (d, J = 8.4 Hz, 1H), 6.99 (s, 1H), 6.91 (s, 1H), 6.76 (d, J = 8.4 Hz, 1H), 3.75-3.83 (m, 1H), 3.68 (s, 3H), 3.51-3.55 (m, 4H), 3.31-3.43 (m, 2H), 2.08-2.29 (m, 2H), 1.48 (s, 9H). LCMS: m/e 315.41 [M+H]

Step 6: tert-Butyl 3-(5-(3-cyanobenzamido)-1-methyl-1H-indol-3-yl)pyrrolidine-1-carboxylate. To a solution of tert-butyl 3-(5-amino-1-methyl-1H-indol-3-yl)pyrrolidine-1-carboxylate (47.5 mg, 0.15 mmol) in DCM (5 mL) was added TEA (61 mg,0.60 mmol) followed by addition of 3-cyanobenzoyl chloride (50 mg, 0.3 mmol) in DCM (5 mL) at 0 °C. The progress of reaction was monitored TLC (5% methanol in Chloroform). After completion, reaction mixture was diluted with water and extracted by using DCM. The combined extracts were dried (Na₂SO₄), filtered and concentrated in vacuum to give a crude compound which was subjected to column chromatography (Silica gel, 100-200 mesh) to afford the title compound (30 mg, 36%) as a brown colored solid. ¹H NMR (400 MHz,CDCl₃): δ 8.31 (s, 1H), 8.26 (d, J = 7.6 Hz, 1H) 7.92-7.95 (m, 3H), 7.71 (d, J = 7.6 Hz, 1H), 7.44 (d, J = 8.4 Hz, 1H), 7.36 (d, J = 8.4 Hz, 1H), 7.06 (s, 1H), 3.81-3.87 (m, 1H), 3.78 (s, 3H), 3.55-3.63 (m, 2H), 3.35-3.45 (m, 2H), 2.11-2.35 (m, 2H), 1.48 (s, 9H). LCMS: m/e 345.05 [M-Boc]

Step 7: 3-Cyano-N-(1-methyl-3-(pyrrolidin-3-yl)-1H-indol-5-yl)benzamide. To a solution of Int-5 (1eq) in methanol was added 4M HCl solution in dioxane at 0°C and then reaction was stirred at room temperature for 4-5 hrs. The progress of reaction was monitored TLC (20% methanol in DCM), after completion, reaction mixture was concentrated and diluted with water and extracted by using chloroform. Aqueous layer was separated and basified with sat. solution of NaHCO₃ and extracted with 10% solution of methanol in chloroform. The combined extracts were dried (Na₂SO₄), filtered and concentrated in vacuum to give the title compound as a brown colored solid. ¹H NMR (400 MHz, MeOD): δ 8.32 (s, 1H), 8.26 (d, J = 8 Hz, 1H), 7.92-7.96 (m, 2H), 7.69-7.73 (m, 1H), 7.42 (d, J = 1.6 Hz 1H), 7.40 (d, J=1.6 Hz, 1H), 7.35 (d, J = 8 Hz 1H), 7.05 (s, 1H), 3.76 (s, 3H), 3.35-3.38 (m, 2H), 2.98-3.16 (m, 2H), 2.87-2.91 (m, 1H), 2.26-2.34 (m, 1H), 1.88-2.03 (m, 2H). LCMS: m/e 345.2 [M+H]

Step 8: 3-cyano-N-(3-(1-(cyclopentylcarbonyl)pyrrolidin-3-yl)-1-methyl-1H-indol-5-yl)benzamide. A solution of cyclopentanecarboxylic acid (1.1eq.), DIPEA (3 eq. mmol) and EDCI.HCl (2.2eq) and HOBT (1.2 eq.) in DMF (2mL) was stirred at room temperature for 1hr. 3-Cyano-N-(1-methyl-3-(pyrrolidin-3-yl)-1H-indol-5-yl)benzamide (1 eq.) was added to the above mixture and stirred at room temperature for 24 hr. The crude product was purified by reverse phase HPLC to afford the title compound. ¹H NMR (400 MHZ, CDCl₃): δ 8.23-8.21 (m, 2H), 8.17 (d, J=7.6 Hz, 1H), 7.99 (s, 0.5H), 7.89 (s, 0.5H), 7.81 (d, J=7.6 Hz, 1H), 7.64-7.60 (m, 1H), 7.44-7.29 (m, 2H), 6.90 (s, 0.5H), 6.87 (s, 0.5H), 4.02-3.49 (m, 8H), 2.86-2.75 (m, 1H), 2.45-1.54 (m, 10H). LCMS: m/e 441.20 [M+H]

### Reference Example 80

### Preparation of N-(3-cyanophenyl)-3-(1-(5-isopropylpyrimidin-2-yl) piperidin-4-yl)-1-methyl-1H-indole-5-carboxamide

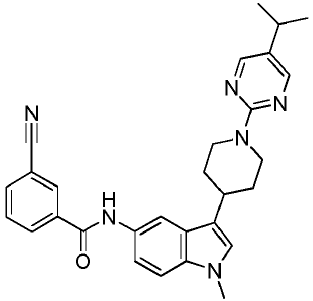

A solution of 3-cyano)-N-(1-methyl-3(piperidin-4-yl)-1H-indol-5-yl)benzamide (prepared in Reference Example **1,** 1eq.), TEA (6eq.) in IPA (3 mL) was stirred at room temperature for 15min. 2-Chloro-5-isopropylpyrimidine (1eq.) was added to the above reaction mixture and stirred at 85 °C for 6-10h. The progress of reaction was monitored by TLC (5% methanol in DCM) and LCMS. After completion of the reaction, the mixture was concentrated, water was added to it, the mixture was extracted with DCM, dried (Na₂SO₄) and concentrated. The crude material was purified by column chromatography (Silica gel-100-200mesh, 1-2% MeOH in DCM) and then triturated with ether to provide the title compound. ¹H NMR (400 MHZ, CDCl₃): δ 10.29 (s, 1H), 8.44 (s, 1H), 8.23-8.03 (m, 5H), 7.75 (t, J=8 Hz, 1H), 7.50 (m, J=8.8 Hz, 1H), 7.36 (d, J=9.2 Hz, 1H), 7.11 (s, 1H), 4.79-4.76 (m, 2H), 3.72 (s, 3H), 3.05-2.73 (m, 4H), 2.04-1.55 (m, 4H), 1.19 (s, 3H) 1.17 (s, 3H). LCMS: m/e 479.30 [M+1]+.

The following examples were prepared analogous to Reference Example **80.**

| **Example** | **Structure** | **Name** | **MS (M+H)** |
|---|---|---|---|
| **R81** | 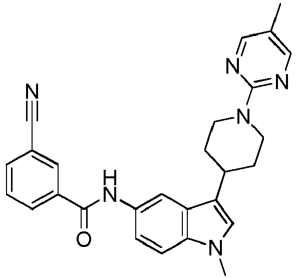 | 3-cyano-N-(1-methyl-3-(1-(5-methylpyrimidin-2-yl)piperidin-4-yl)-1H-indol-5-yl)benzamide | 451.25 |
| **R82** | 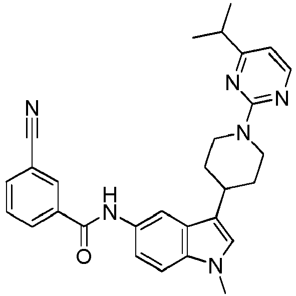 | 3-cyano-N-(3-[1-(5-isopropylpyrimidin-2-yl)piperidin-4-yl]-1-methyl-1H-indol-5-yl)benzamide | 479.30 |

### Reference Example 83

### Preparation of 3-cyano-N-(3-(1-(cyclopentanecarbonyl)piperidin-4-yl)benzo[d]isoxazol-5-yl)benzamide

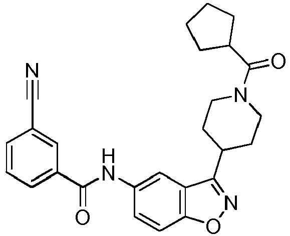

Step 1: tert-butyl 4-(5-bromo-2-fluorobenzoyl)piperidine-1-carboxylate. To a mixture of tert-butyl 4-(methoxy(methyl)carbamoyl)piperidine-1-carboxylate (24 g, 240 mmol) in anhydrous tetrahydrofuran (500 mL) at -70 °C was added butyllithium (100 mL, 2.5 N) dropwise and then the mixture was stirred for 1 h. Then 1-bromo-4-fluorobenzene (35 g, 200 mmol) in anhydrous THF (50 mL) was added to the solution dropwise at -70 °C and stirred for 1 h. After that, 1-bromo-4-fluorobenzene (54.4 g, 200 mmol) in anhydrous tetrahydrofuran (20 mL) was added to the solution dropwise at -70 °C and stirred for another 30 minutes. Saturated ammonium chloride (200 mL) was added to the solution and the mixture was stirred for 10 minutes. Then the mixture was poured into water (500 mL) and extracted with ethyl acetate (100 mL x 3). The combined organic layers were dried over sodium sulfate, filtered, and concentrated in vacuum under reduced pressure to afford the titled compound as an off-white solid (48 g, 62%): ¹H NMR (400 MHz, DMSO-d6): δ: 7.86-7.84 (s, 1H), 7.60-7.57 (s, 1H), 7.02 (t, 1H), 4.09 (s, 2H), 3.20 (t, 1H), 2.83 (d, 2H), 1.86 (d, 2H), 1.62-1.54 (m, 2H), 1.42 (s, 9H).

Step 2: (Z)-tert-butyl 4-((5-bromo-2-fluorophenyl)(hydroxyimino)methyl)piperidine-1-carboxylate. To a solution of tert-butyl 4-(5-bromo-2-fluorobenzoyl)piperidine-1-carboxylate (19.3 g, 50 mmol), hydroxylamine hydrochloride (8.75 g, 125 mmol) in 1:1 isopropanol/water (300 mL) was added potassium hydroxide (28 g, 500 mmol). The mixture was refluxed for 16 hours. And then was poured into water (500 mL) and extracted with ethyl acetate (100 mL x 3). The combined organic layers were concentrated under reduced pressure to afford the titled compound (12 g, 60 %) as a white solid which was carried on in the synthetic sequence without further purification: ¹H NMR (400 MHz, CDCl₃): δ 9.12 (s, 1H), 7.48-7.44 (m, 1H), 7.32-7.30 (m, 1H), 6.98 (t, 1H), 4.13 (t, 2H), 3.35 (t, 1H), 2.72 (s, 2H), 1.76 (d, 2H), 1.428 (s, 9H).

Step 3: tert-butyl 4-(5-bromobenzo[d]isoxazol-3-yl)piperidine-1-carboxylate. A mixture of (Z)-tert-butyl 4-((5-bromo-2-fluorophenyl)(hydroxyimino)methyl)piperidine-1-carboxylate (4.5 g, 11.0 mmol) and potassium tert-butoxide (2.4 g, 22.0 mmol) in dimethylformamide (30 mL) was stirred at room temperature for 1 hour. Then the mixture was heated to 80 °C and stirred for 16 hours. The organic solvent was removed in vacuum under reduced pressure to give the crude residue, which was purified by column chromatography on silica gel (Petroleum ether/ethyl acetate (10:1)) to afford the titled compound (1.4 g, 33 %) as a white solid which was carried further in the sequence immediately without any further purification.

Step 4: tert-butyl 4-(5-(diphenylmethyleneamino)benzo[d]isoxazol-3-yl)piperidine-1-carboxylate. To a mixture of tert-butyl 4-(5-bromobenzo[d]isoxazol-3-yl)piperidine-1-carboxylate (1.15 g, 3 mmol), benzophenone imine (0.65 g, 3.6 mmol), sodium tert-butoxide (0.42 g, 4.2 mmol), 2,2'-bis(diphenylphosphino)-1,1'-binaphthyl (0.37 g, 0.6 mmol) and palladium dibenzylidene acetone (0.275 g, 0.3 mmol) was added toluene (30 mL) was heated to 90 °C for 16 h under nitrogen atmosphere. The mixture was concentrated in vacuum under reduced pressure to give the residue, which was purified by column chromatography on silica gel (Petreum ether/EtOAc (10:1)) to afford the titled compound (0.89 g, 67 %) as a light yellow solid which required no further purification and was immediately carried forward in the synthetic sequence.

Step 5: tert-butyl 4-(5-aminobenzo[d]isoxazol-3-yl)piperidine-1-carboxylate. To a stirred solution of tert-butyl 4-(5-(diphenylmethyleneamino)benzo[d]isoxazol-3-yl)piperidine-1-carboxylate (7.6 g, 17.2 mmol) in tetrahydrofuran (40 mL) was added saturated citric acid (200 mL) and the resulting mixture was stirred at room temperature for 16 hours. Saturated sodium carbonate (500 mL) was added to the solution and then the mixture was extracted with ethyl acetate (50 mL x 5). The combined organic layers were concentrated in vacuum under reduced pressure to give the crude residue, which was purified by column chromatography on silica gel (Petroleum ether/EtOAc (2:1)) to afford the titled compound (2.39 g, 44 %) as a light yellow solid: ¹H NMR (400 MHz, DMSO-d6): δ: 7.36 (d, 1H), 6.92 (dd, 1H), 6.85 (s, 1H), 5.13 (s, 2H), 4.03 (d, 2H), 3.22-3.17 (m, 1H), 2.94 (s, 2H), 1.97 (d, 2H), 1.72-1.62 (m, 2H), 1.42 (s, 9H).

Step 6: tert-butyl 4-(5-(3-cyanobenzamido)benzo[d]isoxazol-3-yl)piperidine-1-carboxylate. To a mixture of tert-butyl 4-(5-aminobenzo[d]isoxazol-3-yl)piperidine-1-carboxylate (0.18 g, 1.2 mmol), O-(Benzotriazol-1-yl)-N,N,N',N'-tetramethyluronium tetrafluoroborate (0.38 g, 1.2 mmol), and diisopropylethylamine (0.38 mg, 1.2 mmol) was added dimethylformamide (10 mL) and the mixture was stirred for 1 hour at room temperature. To this mixture was added 3-cyanobenzoic acid (0.32 g, 1 mmol) in dimethylformamide (5 mL) and the solution was stirred overnight. Saturated sodium bicarbonate (200 mL) was added to the solution and stirred for 10 minutes. Then the mixture was poured into water (20 mL) and extracted with ethyl acetate (10 mL x 3). The combined organic layers were concentrated in vacuum under reduced pressure to give the crude product, which was purified by column chromatography on silica gel (Petroleum ether/ethyl acetate (1:1)) to afford the titled compound (0.3 g, 67 %) as a white solid which was carried forward in the sequence immediately without further purification.

Step 7: 3-cyano-N-(3-(piperidin-4-yl)benzo[d]isoxazol-5-yl)benzamide. To a stirred suspension of tert-butyl 4-(5-(3-cyanobenzamido)benzo[d]isoxazol-3-yl)piperidine-1-carboxylate (0.30 g, 0.62 mmol) in dichloromethane (5 mL) at 0 °C was added trifluoroacetic acid (20 mL) dropwise. The mixture was allowed to warm up to room temperature and stirred for 6 hours. The mixture was then concentrated under reduced pressure and then washed with ethyl acetate (10 mL) to give the titled compound as a white solid which was used directly in the next step without further purification.

Step 8: 3-cyano-N-(3-(1-(cyclopentanecarbonyl)piperidin-4-yl)benzo[d]isoxazol-5-yl)benzamide. A mixture of 3-cyano-N-(3-(piperidin-4-yl)benzo[d]isoxazol-5-yl)benzamide (0.092 g, 0.804 mmol), O-(Benzotriazol-1-yl)-N,N,N',N'-tetramethyluronium tetrafluoroborate (0.26 g, 0.804 mmol) and diisopropylethylamine (2 mL) in dimethylformamide (10 mL) was stirred at room temperature for 2 hours. Then, cyclopentanecarboxylic acid (0.23 g, 0.67 mmol) was added to the solution and stirred overnight. Saturated sodium bicarbonate (5 mL) was added to the solution and stirred for 10 minutes. Then the mixture was poured into water (20 mL) and extracted with ethyl acetate (5 mL x 3). The combined organic layers were concentrated in vacuum under reduced pressure to give the crude product, which was purified to give the titled compound (0.112 g, 38 %) as a white solid: ¹H NMR (400 MHz, DMSO-d6): δ 10.65 (s, 1H), 8.43 (s, 1H), 8.39 (s, 1H), 8.28 (d, 1H), 8.14 (d, 1H), 7.92 (dd, 1H), 7.78-7.74 (m, 2H), 4.49 (d, 1H), 4.12 (d, 1H), 3.49-3.44 (m, 1H), 3.26 (t, 1H), 3.03 (t, 1H), 2.83 (t, 1H), 2.12-2.05 (m, 2H), 1.78-1.52 (m, 10H). LC/MS (10%-90% CH₃CN:H₂O gradient over 8 min): 4.40 min. 443.2 M+H.

### Reference Example 84

### Preparation of 3-cyano-N-(1-(1-(cyclopentanecarbonyl)piperidin-4-yl)-1H-indazol-6-yl)benzamide

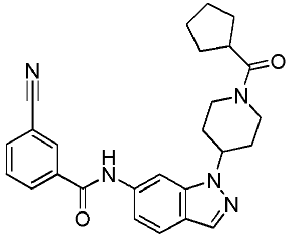

Step 1: tert-butyl 4-(6-nitro-1H-indazol-1-yl)piperidine-1-carboxylate. To a solution of 6-nitro-1H-indazole (8.1 g, 50.0 mmol) and tert-butyl 4-((methylsulfonyl)oxy)piperidine-1-carboxylate (14.0 g, 50.0 mmol) in dimethylformamide (150 mL) was added cesium carbonate (19.4 g, 100.0 mmol). The mixture was heated to 60 °C for 24 hours. The mixture was combined with water (200 mL) and extracted with ethyl acetate (100 mL x 3). The combined organic layers were dried over sodium sulfate, filtered, and concentrated in vacuum under reduced pressure to afford the crude product, which was purified by column chromatography on silica gel (petroleum ether/ethyl acetate 5:1) to afford the titled compound (6.4 g, 37%) as a yellow solid: ¹H NMR (400 MHz, CDCl₃): δ: 8.43 (s, 1H), 8.13 (s, 1H), 8.04-8.01 (m, 1H), 7.84 (d, 1H), 4.66 (s, 1H), 4.35 (s, 2H), 3.08 (s, 2H), 2.27-2.23 (m, 2H), 2.03 (d, 2H), 1.50 (s, 9H).

Step 2: tert-butyl 4-(6-amino-1H-indazol-1-yl)piperidine-1-carboxylate. To a solution of tert-butyl 4-(6-nitro-1H-indazol-1-yl)piperidine-1-carboxylate (5.0 g, 14.5 mmol) in tetrahydrofuran (100 mL) was added a catalytic amount of 5% palladium on carbon (0.5 g) at room temperature. The mixture was degassed and refilled with hydrogen in triplicate. Then the mixture was then shaken at room temperature for 16 hours under an atmosphere of hydrogen (47 psi). The mixture was filtered and the filtrates were then concentrated in vacuum under reduced pressure to give the crude product, which was purified by column chromatography on silica gel (Petroleum ether/EtOAc 2:1) to afford the titled compound (3.8 g, 83 %) as a yellow solid: ¹H NMR (400 MHz, DMSO-d6): δ: 7.72 (s, 1H), 7.35 (d, 1H), 6.54 (s, 1H), 6.49 (d, 1H), 5.30 (s, 2H), 4.47 (t, 1H), 4.06 (d, 2H), 2.95 (s, 2H), 1.86 (s, 4H), 1.42 (s, 9H).

Step 3: tert-butyl 4-(6-(3-cyanobenzamido)-1H-indazol-1-yl)piperidine-1-carboxylate. A mixture of 3-cyanobenzoic acid (0.7 g, 5.0 mmol), O-(Benzotriazol-1-yl)-*N*,*N*,*N'*,*N'-*tetramethyluronium tetrafluoroborate (1.7 g, 5.5 mmol) and diisopropylethylamine (0.9 mL, 5.5 mmol) in dimethylformamide (15 mL) was stirred at room temperature for 2 hours.. Then tert-butyl 4-(6-amino-1H-indazol-1-yl)piperidine-1-carboxylate (1.6 g, 5.0 mmol) in dimethylformamide (15 mL) was added to the solution dropwise and the mixture was stirred at room temperature for 18 hours. Then saturated sodium bicarbonate (200 mL) was added to the solution and stirred for 10 minutes. Then the mixture was poured into water (50 mL) and extracted with ethyl acetate (20 mL x 3). The combined organic layers were concentrated in vacuum under reduced pressure to give the crude product, which was purified by column chromatography on silica gel (Petroleum ether/ethyl acetate 1:1) to afford the titled compound (1.2 g, 54 %) as a pale solid: ¹H NMR (400 MHz, DMSO-d6): δ: 10.59 (s, 1H), 8.44 (s, 1H), 8.32 (s, 1H), 8.28 (d, 1H), 8.08 (d, 1H), 8.03 (s, 1H), 7.79-7.72 (m, 2H), 7.38 (d, 1H), 4.72 (s, 1H), 4.09 (d, 2H), 3.03 (s, 2H), 1.96 (d, 4H), 1.42 (s, 9H).

Step 4: 3-cyano-N-(1-(1-(cyclopentanecarbonyl)piperidin-4-yl)-1H-indazol-6-yl)benzamide. To a solution of tert-butyl 4-(6-(3-cyanobenzamido)-1H-indazol-1-yl)piperidine-1-carboxylate (0.9 g, 2.0 mmol) in methanol (10 mL) at room temperature was added 4N hydrochloric acid in dioxane (10 mL) dropwise. Then, the mixture was stirred for 2.5 hours at room temperature. The solvent was then removed in vacuum under reduced pressure, and the crude product, 3-cyano-N-(1-(piperidin-4-yl)-1H-indazol-6-yl)benzamide, was used in the next step without further purification. A mixture of 3-cyano-N-(1-(piperidin-4-yl)-1H-indazol-6-yl)benzamide (0.23 g, 2 mmol), ), O-(Benzotriazol-1-yl)-*N*,*N*,*N'*,*N'*-tetramethyluronium tetrafluoroborate (0.71 g, 2.2 mmol) and diisopropylethylamine (2 mL) in dimethylformamide (10 mL) was stirred at room temperature for 1 hour. Then, the cyclopentanecarboxylic acid (0.69 g, 2 mmol) in dimethylformamide (5 mL) was added dropwise and stirred for 18 hours. Saturated sodium bicarbonate (10 mL) was added to the solution and stirred for 10 minutes. Then the mixture was poured into water (20 mL) and extracted with ethyl acetate (10 mL x 3). The combined organic layers were concentrated in vacuum under reduced pressure to give the crude product, which gave the titled compound (0.50 g, 57 %) as a pale solid: ¹H NMR (400 MHz, DMSO-d6): δ: 10.60 (s, 1H), 8.45 (s, 1H), 8.34 (s, 1H), 8.29 (d, 1H), 8.08 (d, 1H), 8.02 (s, 1H), 7.79-7.73 (m, 2H), 7.38 (d, 1H), 4.81 (t, 1H), 4.55 (d, 1H), 4.14 (d, 1H), 3.06 (t, 1H), 2.84 (t, 1H), 2.07-1.52 (m, 13H).

### Reference Example 85

### Preparation of 4-(5-(3-cyanobenzamido)-1H-indol-3-yl)-N-cyclopentylpiperidine-1-carboxamide

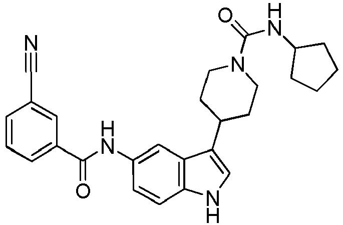

Step 1: 3-cyano-N-(3-(piperidin-4-yl)-1H-indol-5-yl)benzamide. To a suspension of tert-butyl 4-(5-amino-1H-indol-3-yl)piperidine-1-carboxylate (prepared in Reference Example 36, 5.0 g, 16.0 mmol) in 60 mL of dimethylformamide at room temperature was added 3-cyanobenzoic acid (2.3 g, 15.9 mmol), O-(Benzotriazol-1-yl)-N,N,N',N'-tetramethyluronium tetrafluoroborate (5.8 g, 17.4 mmol), and N-ethyl-N-isopropylpropan-2-amine (2.3 g, 17.4 mmol). The mixture was then stirred at rt for 96 hours then concentrated under reduced pressure, diluted with 30 mL of ethyl acetate, washed with water and brine, dried with magnesium sulphate, then filtered and concentrated under reduced pressure to give a crude solid which was immediately dissolved in 100 mL and treated with 20 mL of 4N hydrochloric acid in dioxane. The mixture was then stirred at room temperature for 4 hours then concentrated under reduced pressure to give 5.9 g (99%) of the titled compound as an off-white solid: ¹H NMR (400 MHz, Methanol-d4): δ 8.31 (s, 1H), 8.24 (d, 1H, J = 8.0 Hz), 8.07 (s, 1H), 7.93 (d, 1H, J = 7.6 Hz), 7.71 (m, 1H), 7.36 (d, 1H, J = 7.6 Hz), 7.23 (d, 1H, J = 8.4 Hz), 7.12 (s, 1H), 3.49 (m, 2H), 3.19 (m, 3H), 2.29 (m, 2H), 1.97 (m, 2H).

Step 2: 4-(5-(3-cyanobenzamido)-1H-indol-3-yl)-N-cyclopentylpiperidine-1-carboxamide. To a solution of 3-cyano-N-(3-(piperidin-4-yl)-1H-indol-5-yl)benzamide (100.0 mg, 0.26 mmol) in 1.5 mL of dimethylformamide was added is isocyanatocyclopentane (35.1 mg, 0.32 mmol), and triethylamine (53.2 mg, 0.53 mmol). The mixture was then stirred at room temperature for 30 minutes then was washed with saturated aqueous sodium chloride. The organic layer was then collected and was dried over sodium sulfate, filtered and evaporated to give the title compound. LC/MS (10%-90% CH₃CN:H₂O gradient over 8 min): 3.87 min. 456.1 M+H. ¹H NMR (400 MHz, DMSO-*d*₆) δ ppm 10.81 (br. s., 1 H), 10.28 (s, 1 H), 8.44 (s, 1 H), 8.29 (d, *J*=7.32 Hz, 1 H), 8.05 (d, *J*=8.05 Hz, 1 H), 7.99 (s, 1 H), 7.75 (t, *J*=8.05 Hz, 1 H), 7.44 (d, *J*=8.79 Hz, 1 H), 7.32 (d, *J*=8.79 Hz, 1 H), 7.12 (d, *J*=1.46 Hz, 1 H), 6.21 (d, *J*=6.59 Hz, 1 H), 4.11 (d, *J*=12.45 Hz, 2 H), 3.85 - 3.97 (m, 1 H), 2.88 (t, J*=*12.08 Hz, 1 H), 2.78 (t, *J*=12.08 Hz, 2 H), 2.54 (s, 1 H), 1.91 (d, *J*=11.71 Hz, 2 H), 1.73 - 1.85 (m, 2 H), 1.33 - 1.69 (m, 9 H).

### Reference Example 86

### Preparation of 3-cyano-N-(3-(1-(cyclopentylsulfonyl)piperidin-4-yl)-1H-indol-5-yl)benzamide

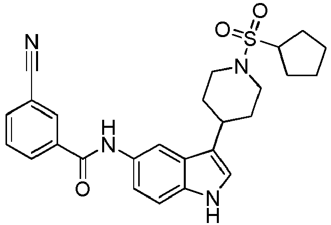

A mixture of 3-cyano-N-(3-(piperidin-4-yl)-1H-indol-5-yl)benzamide (prepared in Reference Example **85,** 40.0 mg, 0.11 mmol), cyclopentanesulfonyl chloride (21.2 mg, 0.13 mmol) and triethylamine (26.6 mg, 0.26 mmol) in dimethylformamide (1 mL) was stirred at room temperature for 1 hour. Saturated sodium bicarbonate (1 mL) was added to the solution and stirred for 10 minutes. Then the mixture was poured into water (2 mL) and extracted with ethyl acetate (1 mL x 3). The combined organic layers were concentrated in vacuum under reduced pressure to give the crude product, which was purified to give the titled compound (0.50 g, 57 %) as a pale solid: ¹H NMR (400 MHz, DMSO-d6) δ ppm 10.82 (1 H, d, J=2.2 Hz), 10.26 (1 H, s), 8.40 (1 H, s), 8.25 (1 H, s), 8.08 - 7.94 (1 H, m), 7.71 (1 H, t, J=8.1 Hz), 7.47 - 7.24 (1 H, m), 7.12 (1 H, d, J=2.2 Hz), 3.81 - 3.50 (1 H, m), 3.06 - 2.80 (1 H, m), 2.46 (1 H, d, J=1.5 Hz), 2.11 - 1.45 (1 H, m).

### Reference Example 87

### Preparation of 3-cyano-N-(3-(1-(cyclopentylmethyl)piperidin-4-yl)-1H-indol-5-yl)benzamide

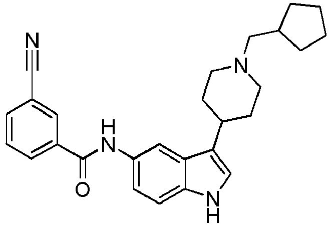

A mixture of 3-cyano-N-(3-(piperidin-4-yl)-1H-indol-5-yl)benzamide (prepared in Reference Example **85,** 50.0 mg, 0.13 mmol), cyclopentanecarbaldehyde (16.7 mg, 0.17 mmol), sodium triacetoxyborohydride (41.8 mg, 0.20 mmol), and triethylamine (19.9 mg, 0.20 mmol) in dichloromethane (2 mL) was stirred at room temperature for 1 hour. Acetic acid (0.1 mL) was then added to the reaction mixture at room temperature and the mixture was then stirred overnight. Saturated sodium bicarbonate (1 mL) was added to the solution and stirred for 10 minutes. Then the mixture was poured into water (2 mL) and extracted with ethyl acetate (1 mL x 3). The combined organic layers were concentrated in vacuum under reduced pressure to give the crude product, which was purified to give the titled compound: MS (ES+) m/z 427 (M+H). ¹H NMR (400 MHz, DMSO-*d*₆) δ ppm 10.85 (br. s., 1 H), 10.31 (s, 1 H), 8.43 (s, 1 H), 8.28 (d, *J*=8.05 Hz, 1 H), 8.04 (d, *J*=8.05 Hz, 1 H), 8.00 (s, 1 H), 7.70 - 7.80 (m, 1 H), 7.47 (d, *J*=8.05 Hz, 1 H), 7.37 - 7.44 (m, 1 H), 7.28 - 7.35 (m, 1 H), 7.17 - 7.27 (m, 1 H), 7.09 (s, 1 H), 3.21 (d, *J*=10.98 Hz, 1 H), 2.76 - 2.88 (m, 1 H), 2.51 - 2.68 (m, 3 H), 2.15 (dt, *J*=15.19, 7.41 Hz, 1 H), 1.99 (d, *J*=12.45 Hz, 2 H), 1.68 - 1.91 (m, 5 H), 1.42 - 1.62 (m, 5 H), 1.13 - 1.27 (m, 2 H).

### Reference Examples 88

### Preparation of 3-cyano-N-(3-(1-(cyclopentanecarbonyl)piperidin-4-yl)-1,4-dimethyl-1H-indol-5-yl)benzamide

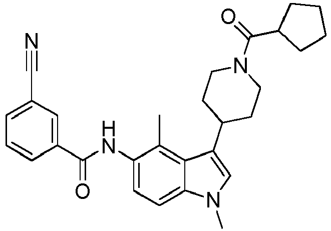

Step 1: 3-iodo-1,4-dimethyl-5-nitro-1H-indole. To a cooled solution (ice-water bath) of 4-methyl-5-nitroindole (1.76 g, 10 mmol) in DMF (30 mL) was added KOH pellets (0.78 g, 14 mmol). The cooling bath was removed, and the mixture was stirred at rt for 10 min. Iodine (2.79 g, 11 mmol) was added, and the stirring was continued for 5 h at room temperature. To this mixture was added potassium carbonate (3.17 g, 23 mmol) and methyl iodide (3.1 mL, 50 mmol), and stirring was continued at room temperature for 16h. The mixture was diluted with water (150 mL), and treated with solid NaHSO₃ with stirring until all excess iodine was quenched. The crude product was collected by filtration, washed with plenty of water, and dried. This crude product was suspended in 96% EtOH. After stirring for 15 min, the precipitate was collected and washed with two small portions of EtOH to provide 2.78 g (88%) of a golden-brownish solid. MS *m*/*z* 316 (M+H).

Step 2: tert-butyl 4-(1,4-dimethyl-5-nitro-1H-indol-3-yl)-5,6-dihydropyridine-1(2H)-carboxylate. 3-iodo-1,4-dimethyl-5-nitro-1H-indole (2.70 g, 8.5 mmol), tert-butyl 4-(4,4,5,5-tetramethyl-1,3,2-dioxaborolan-2-yl)-5,6-dihydropyridine-1(2H)-carboxylate (3.17 g, 10.25 mmol), Pd EnCat® TPP30 (polymer based Pd-PPh₃, 337 mg), and potassium carbonate (2.36 g, 17.1 mmol) were charged in a vessel, followed by a mixture of DME/ethanol/water (4:1:1, 18 mL) and the mixture was degassed with nitrogen for 10 min. The vessel was then sealed, and the mixture was stirred at 70 °C for 18h.The mixture was filtered, diluted with water, and extracted three times with EtOAc. The combined organic layers were washed with brine, and concentrated in vacuo. The residue was subjected to flash chromatography (n-heptane - 30% EtOAc/n-heptane), to provide 2.17 g (68%) of the title compound as a yellow foam. MS *m*/*z* 372 (M+H]).

Step 3: 1,4-dimethyl-5-nitro-3-(1,2,3,6-tetrahydropyridin-4-yl)-1H-indole hydrochloride. A solution of tert-butyl 4-(1,4-dimethyl-5-nitro-1H-indol-3-yl)-5,6-dihydropyridine-1(2H)-carboxylate (1.53 g, 4.12 mmol) in dioxane (30 mL) was treated with HCl (4 M solution in dioxane, 12 mL) over 10 min. The resulting mixture was stirred at room temperature for 48h. The solvents were removed in vacuo. The residue was co-evaporated twice with with MeOH, followed by thorough drying in vacuo, providing 1.26g (99%) of the title compound as an orange solid. MS *m*/*z* 272 (M+H).

Step 4: cyclopentyl(4-(1,4-dimethyl-5-nitro-1H-indol-3-yl)-5,6-dihydropyridin-1(2H)-yl)methanone. 1,4-dimethyl-5-nitro-3-(1,2,3,6-tetrahydropyridin-4-yl)-1H-indole hydrochloride (1.26 g, 4.1 mmol) was suspended in DCM (30 mL), and the mixture was cooled in an ice-water bath. Triethylamine (2.3 mL, 16.5 mmol), followed by cyclopentanecarbonyl chloride (0.65 mL, 5.35 mmol) were added. The mixture was slowly allowed to reach room temperature for 16h. Water was added, and the layers were separated. The aqueous layer was extracted with twice with DCM and the layers were separated. The combined organic extracts were concentrated to a yellow oily residue. The crude was subjected to flash chromatography (n-heptane - 50% EtOAc/n-heptane), to provide 1.34 g (89%) of the title compound as a yellow solid. LCMS *m*/*z* 368 (M+H).

Step 5: preparation of (4-(5-amino-1,4-dimethyl-1H-indol-3-yl)piperidin-1-yl)(cyclopentyl)methanone. A mixture of the cyclopentyl(4-(1,4-dimethyl-5-nitro-1H-indol-3-yl)-5,6-dihydropyridin-1(2H)-yl)methanone (1.0 g, 2.72 mmol), ammonium formate (2.1 g, 33 mmol), 5% Pd/C (150 mg), and 96% EtOH (50 mL) was heated at 85 °C for 90 min under an atmosphere of nitrogen. The mixture was filtered and concentrated in vacuo. The residue was diluted with water, and extracted four times with CHCl₃. The combined organics were dried, and concentrated in vacuo, to provide 0.84 g (91%) of the title compound as an off-white foam. MS *m*/*z* 340 (M+H).

Step 6: 3-cyano-N-(3-(1-(cyclopentanecarbonyl)piperidin-4-yl)-1,4-dimethyl-1H-indol-5-yl)benzamide. (4-(5-amino-1,4-dimethyl-1H-indol-3-yl)piperidin-1-yl)(cyclopentyl)methanone (0.84 g, 2.5 mmol) was dissolved in DCM (50 mL), and the mixture was cooled in an ice-water bath. Triethylamine (1.14 mL, 8.16 mmol) was added over 2 min, followed by 3-cyanobenzoyl chloride (0.68 g, 4.1 mmol) in one portion. The mixture was allowed to reach room temperature, and was then stirred for 20 h. Water was added and the mixture was then extracted four times with EtOAc. The combined organics were washed with brine, dried (Na₂SO₄), and evaporated. The crude product was purified by prep-HPLC to provide 0.73 g (62%) as an off-white foamy solid. ¹H NMR (500 MHz, (CD₃)CO) δ ppm 8.38 (d, 1H, J=8.3Hz), 7.99 (d, 1H, J=8.0Hz), 7.78 (t, 1H, J=7.7Hz), 7.20 (d, 1H, J=8.5Hz), 7.16 (d, 1H, J=8.5Hz), 7.09 (s, 1H), 4.72 (d, 1H, J=12.9Hz), 4.19 (d, 1H, J=13.2Hz), 3.77 (s, 3H), 3.41 (m, 1H), 3.23 (m, 1H), 3.05 (m, 1H), 2.68 (m, 1H), 2.63 (s, 3H), 2.14 (m, 1H), 2.02 (m, 1H) and 1.83-1.45 (m, 10H). LCMS: m/e 469 [M+H].

### Reference Example 89

### Preparation of (R)-3-cyano-N-(1,4-dimethyl-3-(1-(4,4,4-trifluoro-3-hydroxybutanoyl)-piperidin-4-yl)-1H-indol-5-yl)benzamide

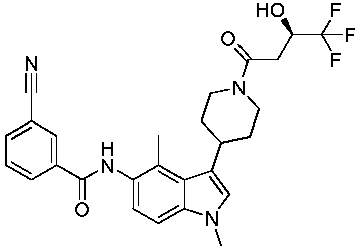

Step 1: tert-butyl 4-(5-amino-1,4-dimethyl-1H-indol-3-yl)-5,6-dihydropyridine-1(2H)-carboxylate. A mixture of tert-butyl 4-(1,4-dimethyl-5-nitro-1H-indol-3-yl)-5,6-dihydropyridine-1(2H)-carboxylate (180 mg, 0.48 mg), ammonium formate (367 mg, 5.8 mmol), 5% Pd/C (30 mg) in 96% EtOH (10 mL) was heated at 85 °C for 90 min under an atmosphere of nitrogen. The mixture was filtered through a Teflon filter and the filtrate was concentrated. The residue was then partitioned between CHCl₃ and water and the aqueous layer was extracted three more times with CHCl₃. The combined organics were concentrated in vacuo to provide the title compound. MS *m*/*z* 344 [M+H].

Step 2: tert-butyl 4-(5-(3-cyanobenzamido)-1,4-dimethyl-1H-indol-3-yl)piperidine-1-carboxylate. The residue from the previous step was dissolved in pyridine (4 mL). 3-Cyanobenzoyl chloride (120 mg, 0.73 mmol) was added, and the mixture was stirred at room temperature overnight. The pyridine was removed in vacuo. The residue was partitioned between CHCl₃ and water. The aqueous layer was extracted twice with CHCl₃, and the combined organics were concentrated in vacuo. The residue was subjected to flash chromatography (n-heptane - EtOAc/n-heptane 1:1). Further purification was done by prep-HPLC to provide 74 mg of the title compound as an off-white solid. MS: *m*/*z* 473 [M+H].

Step 3: 3-cyano-N-(1,4-dimethyl-3-(piperidin-4-yl)-1H-indol-5-yl)benzamide hydrochloride. A solution tert-butyl 4-(5-(3-cyanobenzamido)-1,4-dimethyl-1H-indol-3-yl)piperidine-1-carboxylate (74 mg, 0.16 mmol) in 1,4-dioxane (6 mL) cooled in an ice-water bath was treated with HCl (0.47 mL, 4 M solution in dioxane) over 5 min. The cooling bath was removed and the resulting mixture was stirred at room temperature for 36h. The mixture was evaporated in vacuo, and then co-evaporated twice with MeOH to provide the title compound. The compound was used as such in the subsequent step. MS *m*/*z* 377 [M+H].

Step 4: (R)-3-cyano-N-(1,4-dimethyl-3-(1-(4,4,4-trifluoro-3-hydroxybutanoyl)piperidin-4-yl)-1H-indol-5-yl)benzamide. 3-cyano-N-(1,4-dimethyl-3-(piperidin-4-yl)-1H-indol-5-yl)benzamide hydrochloride was suspended in DMF (3 mL). A solution of DIPEA (109 µl, 0.63 mmol), HATU (77 mg, 0.20 mmol), and (R)-4,4,4-trifluoro-3-hydroxybutanoic acid (32 mg, 0.20 mmol) in 0.5 mL DMF) was added at room temperature. A yellow solution was obtained within minutes. The reaction mixture was then stirred at room temperature overnight. The mixture was purified directly by prep-HPLC to provide 57 mg (71% over two steps) of the title compound an off-white solid. ¹H NMR (500 MHz, (CD₃)₂CO) δ ppm 8.42 (s, 1H), 8.36 (d, 1H, J=7.8Hz), 7.97 (d, 1H, J=7.8Hz), 7.75 (t, 1H, J=7.7Hz), 7.17 (m, 2H), 7.07 (s, 1H), 4.71 (m, 1H), 4.58 (m, 1H), 4.13 (m, 1H), 3.75 (s, 3H), 3.42 (m, 1H), 3.28 (m, 1H), 2.82-2.68 (m, 3H), 2.62 (s, 3H), 2.10 (m, 2H), 1.66 (m, 1H) and 1.52 (m, 1H). MS *m*/*z* 513 [M+H].

### Reference Example 90

### Preparation of (R)-3-cyano-N-(1-methyl-3-(1-(4,4,4-trifluoro-3-hydroxybutanoyl)piperidin-4-yl)-1H-indol-5-yl)benzamide

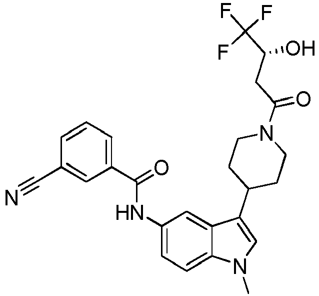

3-cyano-N-(1-methyl-3-(piperidin-4-yl)-1H-indol-5-yl)benzamide (40 mg, 0.11 mmol), (R)-4,4,4-trifluoro-3-hydroxybutanoic acid (26 mg, 0.17 mmol), dimethylaminopropylcarbodiimide (26 mg, 0.17 mmol), 1-hydroxy-benzotriazole (23 mg, 0.17 mmol) and triethylamine (39 µl, 0.28 mmol) were mixed with DMF (0.5 mL). The mixture was stirred at room temperature for 3h. The volatiles were evaporated and the residue was passed through a plug of silica (0.5g) using EtOAc as eluent. After evaporation, the residue was purified by flash chromatography with heptane/EtOAc (1:1 - 4:6) to provide 30 mg (54%) of the title compound as a white solid. ¹H NMR (500 MHz, (CD₃)₂CO) δ ppm 8.41 (s, 1H), 8.35 (d, 1H, J=8.1 Hz), 8.19 (d, 1H, J=11.4Hz), 7.98 (d, 1H, J=7.6Hz), 7.77 (t, 1H, J=8.0Hz), 7.54 (m, 1H), 7.35 (d, 1H, J=8.6Hz), 7.06 (d, 1H, J=2.8Hz), 4.70 (m, 1H), 4.59 (m, 1H), 4.15 (m, 1H), 3.79 (s, 3H), 3.23 (m, 1H), 3.13 (m, 1H), 2.85-2.80 (m, 4H), 2.11 (m, 1H) and 1.84-1.58 (m, 2H). MS *m*/*z* 499 [M+H].

### Reference Example 91

### Preparation of (R)-4-fluoro-N-(1-methyl-3-(1-(4,4,4-trifluoro-3-hydroxybutanoyl)piperidin-4-yl)-1H-indol-5-yl)benzenesulfonamide

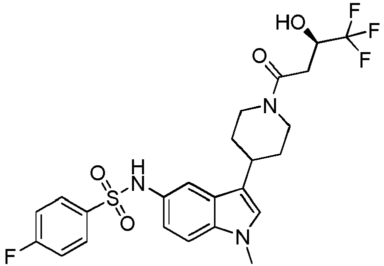

Step 1: tert-butyl 4-(5-(4-fluorophenylsulfonamido)-1-methyl-1H-indol-3-yl)piperidine-1-carboxylate. 4-Fluorobenzene-1-sulfonyl chloride (236 mg, 1.21 mmol) was added to a stirred solution of tert-butyl 4-(5-amino-1-methyl-1H-indol-3-yl)piperidine-1-carboxylate (200 mg, 0.61 mmol) and pyridine (100 µL, 1.21 mmol) in dichloromethane (6 mL). The reaction mixture was stirred at room temperature overnight. Water (10 mL) was added. The phases were separated and the organic layer was concnetrated. The crude product was purified by flash chromatography (EtOAc/heptane; 2:8-3:7) to provide 250 mg (84%) of the title compound as a white/yellow solid. LCMS *m*/*z* 488 [M+H].

Step 2: 4-fluoro-N-(1-methyl-3-(piperidin-4-yl)-1H-indol-5-yl)benzenesulfonamide. A solution of HCl in 1,4-dioxane (1.7 mL, 4M) was added to a slurry of tert-butyl 4-(5-(4-fluorophenylsulfonamido)-1-methyl-1H-indol-3-yl)piperidine-1-carboxylate (210 mg, 0.44 mmol) in methanol cooled on an ice-water bath. The temperature was allowed to warm to room temperature for 30 min, whereupon the pH was adjusted to 9 by addition of NaHCO₃ (sat.), followed by extraction with dichloromethane/MeOH (9:1) four times. The extracts were dried with Na₂SO₄ and concentrated. The crude product was purified by flash chromatography with dichloromethane/EtOH/Et₃N (7.6:1.9:0.5) to provide 160 mg (97%) of the title compound as a yellow solid. LCMS *m*/*z* 388 [M+H].

Step 3: (R)-4-fluoro-N-(1-methyl-3-(1-(4,4,4-trifluoro-3-hydroxybutanoyl)piperidin-4-yl)-1H-indol-5-yl)benzenesulfonamide. 4-fluoro-N-(1-methyl-3-(piperidin-4-yl)-1H-indol-5-yl)benzenesulfonamide (2.2 g, 5.7 mmol), (R)-4,4,4-trifluoro-3-hydroxybutanoic acid (1.1 g, 6.8 mmol), dimethylaminopropylcarbodiimide (1.1 g, 6.8 mmol) and 1-hydroxy-benzotriazole (0.9 g, 6.8 mmol) were mixed. DMF (20 mL) was added, and the mixture was cooled in an ice-water bath. Triethylamine (2.0 mL, 14 mmol) was added over 5 min. The mixture was allowed to reach room temperature overnight. The reaction was quenched by adding water. The aqueous phase was extracted 3 times with EtOAc, and the combined organics were washed sequentially with 5% citric acid, sat. NaHCO₃, brine, and were then dried over MgSO₄. Evaporation of the solvents in vacuo, followed by thorough drying under vacuum provided 2.4 g (81%) of the title compound as a light-tan foamy solid. ¹H NMR (500 MHz, (CD₃)₂CO) δ ppm 7.75 (m, 2H), 7.34 (d, 1H, J=4.1), 7.27-7.22 (m, 3H), 7.03 (s, 1H), 6.99 (d, 1H, J=8.6Hz), 4.67 (d, 1H, J=12.6Hz), 4.58 (m, 1H), 4.11 (m, 1H), 3.73 (s, 3H), 3.28 (m, 1H), 3.03 (m, 1H), 2.81-2.69 (m, 4H), 1.97 (m, 1H), 1.65 (m, 1H) and 1.52 (m, 1H). MS *m*/*z* 528 [M+H].

### Reference Example 92

### Preparation of 4-fluoro-N-(3-(1-isobutyrylpiperidin-4-yl)-1-methyl-1H-indol-5-yl)benzenesulfonamide

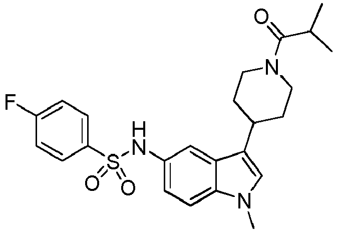

4-Fluoro-N-(1-methyl-3-(piperidin-4-yl)-1H-indol-5-yl)benzenesulfonamide (1.8 g, 4.7 mmol) was suspended in DCM, and the mixture was cooled in an ice-water bath. Pyridine (1.1 mL, 14 mmol) was added, followed by the isobutyryl chloride (660 µl, 6.3 mmol) over 10 min. The resulting mixture was stirred on an ice-water bath for 2h. The temperature was allowed to room temperature for 2 h, whereupon water was added. The layers were separated. The aqueous layer was extracted twice with DCM, and the combined organics were concentrated. The residue was purified by prep-HPLC to provide 1.3 g (61%) as an off-white solid. ¹H NMR (500 MHz, (CD₃)₂CO) δ ppm 7.75 (m, 2H), 7.32 (d, 1H, J=1.9), 7.27-7.22 (m, 3H), 7.03 (s, 1H), 6.95 (dd, 1H, J=8.7, 1.9Hz), 4.67 (d, 1H, J=12.6Hz), 4.12 (d, 1H, J=13.6Hz), 3.73 (s, 3H), 3.23 (m, 1H), 3.02-2.90 (m, 2H), 2.67 (m, 1H), 1.96 (m, 2H), 1.52 (m, 2H) and 1.08 (s, 6H). LCMS: m/e 458 [M+H].

### Reference Example 93

### Preparation of N-(3-(1-(cyclopentanecarbonyl)piperidin-4-yl)-1-methyl-1H-indol-5-yl)-4-methylpiperidine-1-sulfonamide

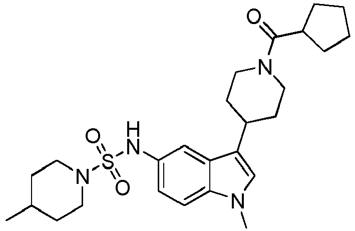

Step 1: 2-oxooxazolidine-3-sulfonyl chloride. To solution of oxazolidin-2-one (4 g, 46 mmol), DMAP (0.56 g, 4.6 mmol) and triethylamine (7.7 mL, 55 mmol) in dichloromethane (100 mL) cooled on an ic-water bath was added sulfuryl chloride (3.7 mL, 46 mmol) drop-wise. The cooling bath was removed and the mixture was stirred at room temperature overnight. The mixture was washed with 0.2 M HCl (2x15 mL). The phases were separated and the solvents were evaporated. The residue was filtered through a plug of silica with dichloromethane as eluent. After evaporation of the solvents, the residue was washed with isohexane and a small amount of EtOAc to provide 5.3 g (62%) of the title compound as white powder. MS *m*/*z* 186 [M+H].

Step 2: N-(3-(1-(cyclopentanecarbonyl)piperidin-4-yl)-1-methyl-1H-indol-5-yl)-2-oxooxazolidine-3-sulfonamide. A slurry of (4-(5-amino-1-methyl-1H-indol-3-yl)piperidin-1-yl)(cyclopentyl)methanone hydrochloride (30 mg, 0.08 mmol) in MeCN (1.5 mL) was added drop-wise to a solution of 2-oxooxazolidine-3-sulfonyl chloride (92 mg, 0.5 mmol) and pyridine (33 µl, 0.41 mmol) in acetonitrile (2 mL) under an atmosphere of nitrogen at room temperature. The mixture was stirred at room temperature overnight. The solvents were evaporated and the residue was purified by prep-HPLC to provide 5 mg (13%) of the title compound as a purple solid. LCMS *m*/*z* 475 [M+H].

Step 3: N-(3-(1-(cyclopentanecarbonyl)piperidin-4-yl)-1-methyl-1H-indol-5-yl)-4-methylpiperidine-1-sulfonamide. 4-methylpiperidine (8 mg, 0.08 mmol) was added to a solution of N-(3-(1 -(cyclopentanecarbonyl)piperidin-4-yl)-1-methyl-1H-indol-5-yl)-2-oxooxazolidine-3-sulfonamide (4 mg, 0.01 mmol) and triethylamine (12 µl, 0.08 mmol) in MeCN (1.5 mL) under an atmosphere of nitrogen at room temperature. The mixture was stirred at 70 °C overnight. After filtration through a syringe filter, purification was done by prep-HPLC to provide 2 mg (49%) of the title compound as a white powder. ¹H NMR (500 MHz, (CD₃OD) δ ppm 7.48 (d, 1H, J=1.9Hz), 7.25 (d, 1H, J=8.7Hz), 7.07 (dd, 1H, J=8.7, 1.9Hz), 6.98 (s, 1H), 4.65 (m, 1H), 4.20 (m, 1H), 3.72 (s, 3H), 3.65 (m, 2H), 3.09 (m, 2H), 2.81 (m, 1H), 2.65 (m, 2H), 2.11 (m, 1H), 2.04 (m, 1H), 1.92-1.56 (m, 12H), 1.34 (m, 2H), 1.03 (m, 2H) and 0.85 (d, 3H, J=6.5Hz). LCMS *m*/*z* 487 [M+H].

### Example 94

### Preparation of 3-cyano-N-(3-(1-(cyclopentanecarbonyl)piperidin-4-yl)-1,4-dimethyl-1H-pyrrolo[2,3-b]pyridin-5-yl)benzamide

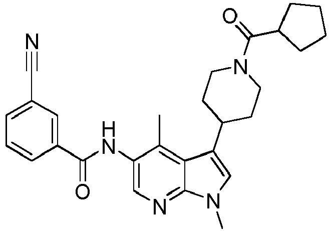

Step 1: 4-chloro-1-(phenylsulfonyl)-1H-pyrrolo[2,3-*b*]pyridine. 4-chloro-1H-pyrrolo[2,3-*b*]pyridine (5.25 g, 34 mmol), benzenesulfonyl chloride (6.6 mL, 52 mmol), triethylamine (5.2 mL, 38 mmol) and N,N-dimethylpyridin-4-amine (420 mg, 3.4 mmol) were mixed and stirred at room temperature for 2 h. The mixture was washed with 1 M HCl followed by saturated NaHCO₃ (aq), filtered through a phase separator. The organic phase was evaporated, and the residue was triturated with 2-propanol to give 7.76 g (77%) of the title product. MS *m*/*z* 293 [M+H].

Step 2: 4-chloro-5-nitro-1-(phenylsulfonyl)-1H-pyrrolo[2,3-*b*]pyridine. To an ice cold solution of 4-chloro-1-(phenylsulfonyl)-1H-pyrrolo[2,3-b]pyridine (5.0 g, 17 mmol) in dichloromethane (60 mL) was added dropwise under stirring a solution of tetrabutylammonium nitrate (7.8 g, 26 mmol) in dichloromethane (30 mL). When addition was complete, a solution of trifluoroacetic anhydride (3.6 mL, 26 mmol) in dichloromethane (30 mL) was added dropwise under stirring. After complete addition, the reaction mixture was stirred at 0 °C for another 30 minutes, then at room temperature overnight. The mixture was washed with water, the phases were separated. The organic phase was dried, filtered, and evaporated, and the residue was recrystallized from dichloromethane - methanol to provide 3.2 g (55%) of the title compound. MS *m*/*z* 338 [M+H].

Step 3: 4-Methyl-5-nitro-1-(phenylsulfonyl)-1H-pyrrolo[2,3-*b*]pyridine. To a solution of 4-chloro-5-nitro-1-(phenylsulfonyl)-1H-pyrrolo[2,3-*b*]pyridine (3.45 g, 10 mmol) and tetrakis triphenylphosphine palladium (0.18 g, 0.15 mmol) in dioxane (12 mL) under nitrogen was added a solution of trimethylaluminum in toluene (2 M, 5.1 mL, 10 mmol) and the mixture was heated to 130 °C for 30 minutes in a microwave reactor. CAUTION: fast buildup of pressure when heating. The mixture should be heated at the slowest possible setting. After cooling to room temperature, the reaction mixture was poured on ice (200 mL). CAUTION: exothermic reaction! The mixture was extracted twice with dichloromethane, the organic phase was dried by filtration through a phase separator, the solvent was evaporated and the residue was recrystallized from dichloromethane - ethanol to give 2.54 g (78%) of the title product. LCMS: m/e 318 [M+H].

Step 4: 4-Methyl-5-nitro-1H-pyrrolo[2,3-*b*]pyridine. A mixture of 4-methyl-5-nitro-1-(phenylsulfonyl)-1H-pyrrolo[2,3-*b*]pyridine (4.89 g, 15.4 mmol), potassium carbonate (4.26 g, 30.8 mmol) and morpholine (13.4 mL, 154 mmol) in methanol (150 mL) was refluxed for 10 minutes, then quickly cooled in an ice bath. The solvent was evaporated and the residue was slurried in chloroform (100 mL), ammonium chloride (saq, 100 mL) and water (25 mL). The mixture was magnetically stirred for 15 minutes, then left standing in a refrigerator at 3 °C over the weekend. The mixture was then filtered through a glass Büchner funnel (P3) and the precipitate was washed with water and chloroform, then dried under vacuum. The precipitate was the title compound (2.61 g, 96%). LCMS: m/e 178 [M+H].

Step 5: 3-Iodo-1,4-dimethyl-5-nitro-1H-pyrrolo[2,3-*b*]pyridine. To a stirred suspension of the 4-methyl-5-nitro-1H-pyrrolo[2,3-*b*]pyridine (200 mg, 1.13 mmol) in a 2:1 mixture of 2-methyltetrahydrofuran and 99.7% EtOH (9 mL) at rt, was added KOH (pellets, 203 mg, 3.61 mmol). The mixture was stirred at rt for 10 min. Iodine (573 mg, 2.26 mmol) was then added, and stirring was continued at room temperature for 25 min. Potassium carbonate (780 mg, 5.64 mmol) and iodomethane (0.70 mL, 11.3 mmol) were added and stirring at room temperature was continued for 2.5 h. 10% aqueous sodium bisulfite (10 mL) was added dropwise which led to a precipitate between the organic and the aqueous phase. After complete addition the mixture was stirred at room temperature for 30 minutes, then filtered through a glass Büchner funnel and the precipitate was washed with water and dried under vacuum to provide 315 mg (88%) of the pure title compound. LCMS: m/e 318 [M+H].

Step 6: tert-Butyl 4-(1,4-dimethyl-5-nitro-1H-pyrrolo[2,3-*b*]pyridin-3-yl)-5,6-dihydropyridine-1(2H)-carboxylate. To a mixture of 3-iodo-1,4-dimethyl-5-nitro-1H-pyrrolo[2,3-*b*]pyridine (145 mg, 0.46 mmol), tert-butyl 4-(4,4,5,5-tetramethyl-1,3,2-dioxaborolan-2-yl)-5,6-dihydropyridine-1(2H)-carboxylate (184 mg, 0.59 mmol), potassium carbonate (126 mg, 0.91 mmol), and Pd EnCat™ TPP30 (palladium acetate and triphenylphosphine, microencapsuled in polyuria matrix, 0,4mmol Pd/g, 1.0/0.8 Pd/TPP; 35 mg) under nitrogen was added dimethoxyethane (2.7 mL), ethanol (0.7 mL), and water (0.7 mL) and the mixture was stirred at 60 °C overnight. The mixture was poured into water and extracted with chloroform, the phases were separated with a phase separator, the organic phase was evaporated and the residue was purified by column chromatography (mobile phase: 1% methanol in dichloromethane) to provide 150 mg (88%) of the title compound. LCMS: m/e 373 [M+H].

Step 7: tert-Butyl 4-(5-amino-1,4-dimethyl-1H-pyrrolo[2,3-*b*]pyridin-3-yl)piperidine-1-carboxylate. A mixture of tert-butyl 4-(1,4-dimethyl-5-nitro-1H-pyrrolo[2,3-*b*]pyridin-3-yl)-5,6-dihydropyridine-1(2H)-carboxylate (200 mg, 0.54 mmol), ammonium formate (406 mg, 6.4 mmol), and 5 % Palladium on active carbon (30 mg) in ethanol (10 mL) were stirred at 85 °C over a weekend. The reaction mixture was filtered through a Teflon filter, the solvent was evaporated, the residue was dissolved in dichloromethane and washed with sodium bicarbonate (saq), filtered through a phase separator, the organic phase was evaporated to give the title compound, used as such in the next step (yield not determined). LCMS: m/e 345 [M+H].

Step 8: *tert*-Butyl 4-(5-(3-cyanobenzamido)-1,4-dimethyl-1H-pyrrolo[2,3-*b*]pyridin-3-yl)piperidine-1-carboxylate. To an ice cold solution of *tert*-butyl 4-(5-amino-1,4-dimethyl-1H-pyrrolo[2,3-*b*]pyridin-3-yl)piperidine-1-carboxylate (185 mg, 0.54 mmol) was added triethylamine (225 µL, 1.61 mmol) followed by 3-cyanobenzoyl chloride (116 mg, 0.70 mmol). The ice bath was removed and the mixture was stirred at room temperature overnight. Water was added and the mixture was extracted with dichloromethane. After filtration through a phase separator the organic phase was evaporated and the residue was purified by preparative HPLC (sunfire column, 25 mL/min, 20 -> 100 % acetonitrile, 0.05% formic acid (aq), 40 minutes gradient time) to provide 65 mg (26%) of the title compound. LCMS: m/e 474 [M+H].

Step 9: 3-Cyano-N-(1,4-dimethyl-3-(piperidin-4-yl)-1H-pyrrolo[2,3-*b*]pyridin-5-yl)benzamide. To a solution of tert-butyl 4-(5-(3-cyanobenzamido)-1,4-dimethyl-1H-pyrrolo[2,3-*b*]pyridin-3-yl)piperidine-1-carboxylate(50 mg, 0.11 mmol) in dichloromethane (1.5 mL) was added trifluoroacetic acid (81 µL, 1.06 mmol) and the mixture was stirred at room temperature for 2 h. The solvents were evaporated to provid 39 mg (100%) of the title compound. LCMS *m*/*z* 374 [M+H].

Step 10: 3-Cyano-N-(3-(1-(cyclopentanecarbonyl)piperidin-4-yl)-1,4-dimethyl-1H-pyrrolo[2,3-*b*]pyridin-5-yl)benzamide. To a solution of 3-cyano-N-(1,4-dimethyl-3-(piperidin-4-yl)-1H-pyrrolo[2,3-*b*]pyridin-5-yl)benzamide (20 mg, 0.06 mmol) in dimethylformamide (0.5 mL) was added cyclopentanecarbonyl chloride (14 mg, 0.11 mmol) and triethylamine (29 µL, 0.21 mmol) and the mixture was stirred at room temperature overnight. The mixture was injected into a preparative HPLC (sunfire column) and eluted according to the following: 25 mL/min, 20 -> 50 % acetonitrile, 0.05% formic acid (aq), 30 minutes gradient time to provide 11.4 mg (46%) of the title compound as a white solid. ¹H NMR (500 MHz, (CD₃)₂CO) δ ppm 8.45 (s, 1H), 8.39 (d, 1H, J=7.7Hz), 8.20 (s, 1H), 8.01 (d, 1H, J=7.8Hz), 7.78 (t, 1H, J=7.8Hz), 7.24 (s, 1H), 4.71 (d, 1H, J=12.6Hz), 4.20 (d, 1H, J=13.1Hz), 3.80 (s, 3H), 3.34 (m, 1H), 3.24 (m, 1H), 3.05 (m, 1H), 2.68 (m, 1H), 2.64 (s, 3H), 2.12 (d, 1H, J=12.7Hz), 2.03 (d, 1H, J=13.8Hz) and 1.84-1.44 (m, 10H). LCMS *m*/*z* 470 [M+H].

### Example 95

### Preparation of (R)-3-Cyano-N-(1,4-dimethyl-3-(1-(4,4,4-trifluoro-3-hydroxybutanoyl)piperidin-4-yl)-1H-pyrrolo[2,3-b]pyridin-5-yl)benzamide

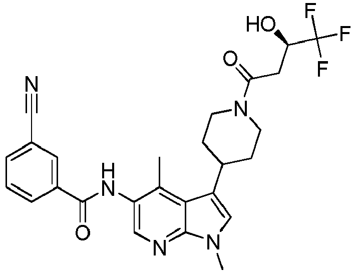

To a solution of 3-cyano-N-(1,4-dimethyl-3-(piperidin-4-yl)-1H-pyrrolo[2,3-*b*]pyridin-5-yl)benzamide (20 mg, 0.06 mmol) in dimethylformamide (0.5 mL) was added (*R*)-4,4,4-trifluoro-3-hydroxybutanoic acid (17 mg, 0.11 mmol), *O*-(7-azabenzotriazol-1-yl)-*N*,*N*,*N'*,*N'-*tetramethyluronium hexafluorophosphate (40 mg, 0.11 mmol) and diisopropylethylamine (35 µL, 0.21 mmol) and the mixture was stirred at room temperature overnight. The mixture was injected into a preparative HPLC (sunfire column) and eluted according to the following: 25 mL/min, 20 -> 50 % acetonitrile, 0.05% formic acid (aq), 30 minutes gradient time to provide 13.7 mg (50%) of the title compound as a white solid. ¹H NMR (500 MHz, (CD₃)₂CO) δ ppm 8.22 (s, 1H), 8.05 (d, 1H, J=7.9Hz), 7.83 (d, 1H, J=7.9Hz), 7.62 (t, 1H, J=7.9Hz), 6.95 (s, 1H), 4.76 (m, 1H), 4.48 (m, 1H), 3.94 (m, 1H), 3.81 (s, 3H), 3.24 (m, 2H), 2.77-2.69 (m, 3H), 2.58 (s, 3H), 2.09 (m, 2H) and 1.58 (m, 2H). MS *m*/*z* 514 [M+H].

### Example 96

### Assay of co-activator recruitment by TR-FRET

The activity of compound of the invention can be determined by a co-activator recruitment by TR-FRET (time-resolved fluorescence resonance energy transfer) assay. In general, the assay is based on the interaction between N-terminally Six-Histidine-tagged-RORC2 ligand binding domain (6-His-RORC2 LBD), expressed in E. coli and purified by affinity chromatography, and biotin-coactivator peptide SRC1-2 (biotin-aminohexanoic acid-CPSSHSSLTERHKILHRLLQEGSPS-NH2; SEQ ID NO: 1) containing the LXXLL consensus domain which is responsible for receptor binding. This interaction is detected by addition of Europium labeled-anti-His antibody (Ex. 337 nm, Em. 620 nm, which binds to 6His) and Streptavidin-APC (Ex. 620 nm, Em. 665 nm, which binds to biotin). When receptor and coactivator are bound to each other, upon shining light at 337 nm on the sample, the Europium emits fluorescence that excites APC due to close proximity (FRET) and this signal is measured at 665 nm. Due to the long lasting fluorescence emission of Europium, the non-specific, short-lived fluorescence is time-resolved (TR) from the fluorescence of interest. Inhibitors of the interaction of receptor and coactivator peptide are detected by a decrease in TR-FRET signal.

Specifically, in one embodiment the aforementioned assay was performed as outlined below. The assay was carried out in black polystyrene, 384-well plates in a total assay volume of 50.5 µL. The assay buffer contained 50 mM TRIS-HCL pH 7.5, 1 mM NaCl, 2 mM MgCl₂, 0.5 mg/mL bovine serum albumin, and 5 mM dithiothreitol. The final concentration of reagents was 6.3 nM RORC2 LBD, 200 nM SRC1-2, 50 nM streptavidin APC, 1 nM Europium-labeled anti-His antibody, and varying concentrations of compounds such that final concentration of DMSO is 1% (v/v). The assay steps were: (1) dispensing 500 µL compound at 100x final concentration in DMSO (test wells) or DMSO only (control wells for no inhibition); and (2) dispensing 50 µL mixture of the other assay components including receptor (test wells) or excluding receptor (control wells for maximal inhibition).

Assay mixtures were incubated are room temperature for 3 hours and read in EnVision 2100 Multilabel Reader (PerkinElmer Life Sciences) at Excitation Filter 320, Emission Europium Filter 615, Emission APC Filter 665, Dichroic Mirror D400/D630.

TR-FRET signal was determined by calculating the ratio of 665 nm by 615 nm and IC₅₀ values of compounds of the invention were determined by the non-linear regression analysis of dose response curves.

References which relate to the above-referenced assay include: Kallen et al. Structure, 2002, 10, 1697-1707; Stehlin et al. EMBO J 2001, 20, 5822-5831; and Zhou et al. Mol Endocrinol 1998, 12, 1594-1604.

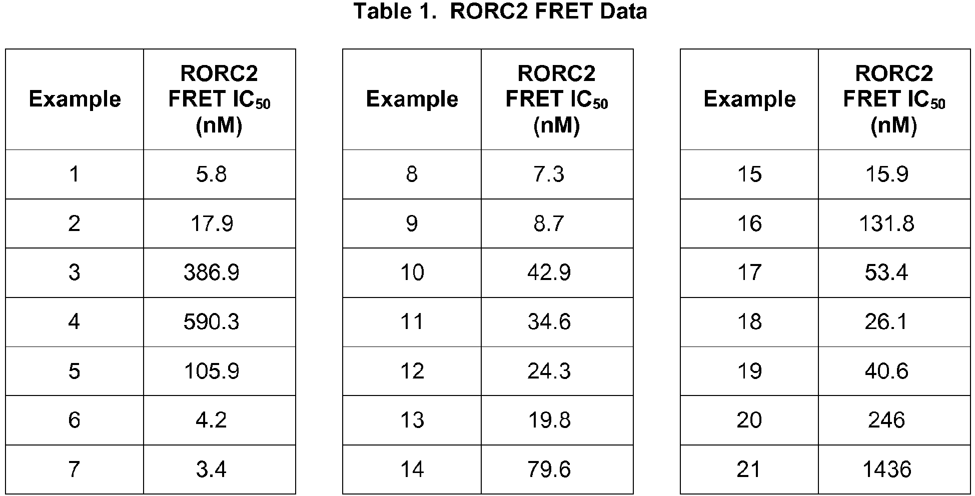

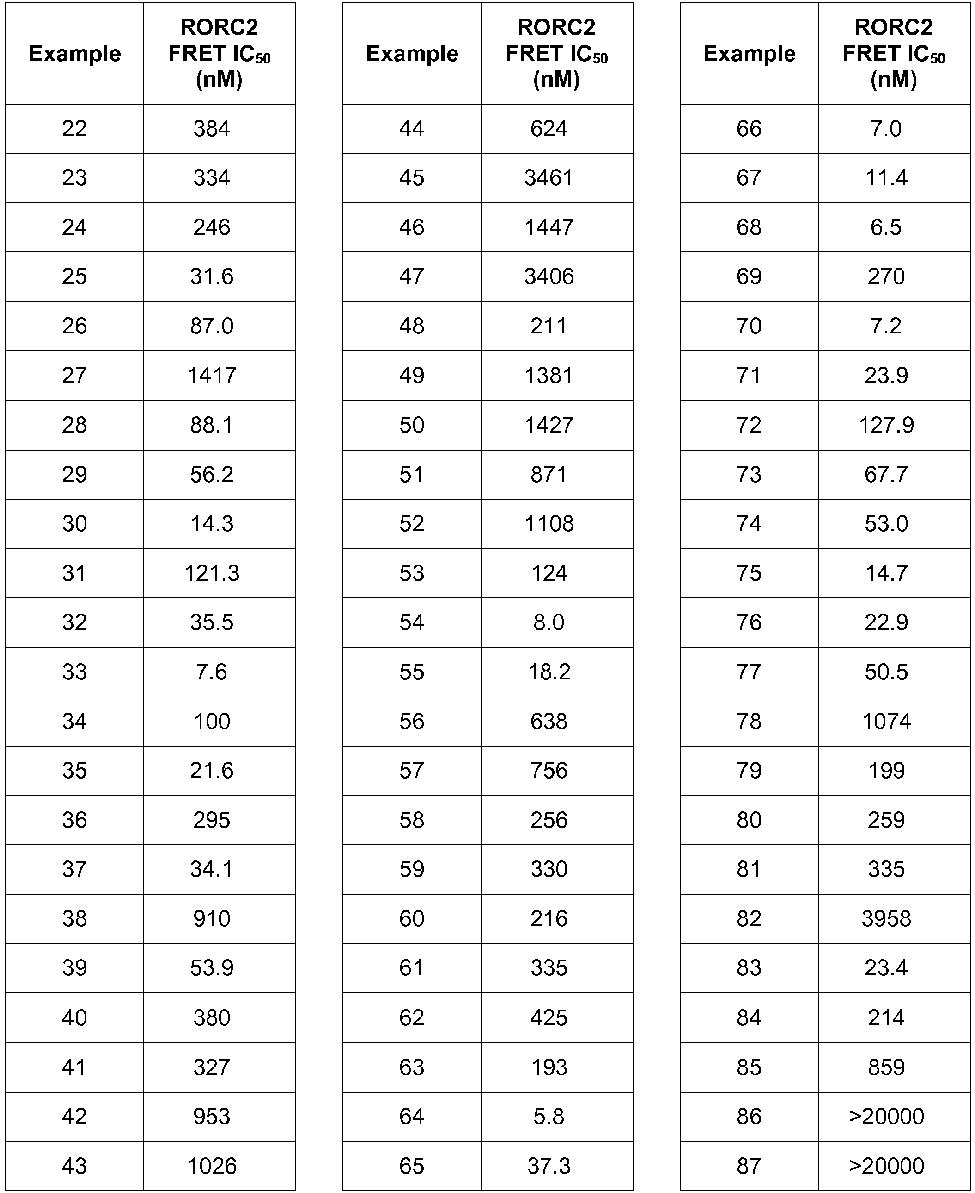

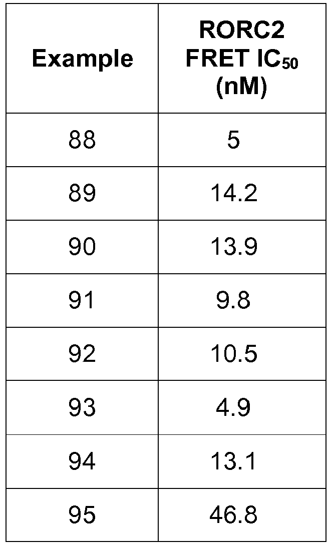

### Example 97

### Assay of Gal4-RORC2 activity by luciferase reporter

The activity of compound of the invention can be also be determined by a luciferase reporter Gal4-RORC2 activity assay. In general, Neuro2A cells (murine neuroblastoma cell line obtained from HPACC, cat #89121404) are transiently transfected with a mammalian expression vector (pM) containing Gal4-RORC2 LBD and a Gal4-responsive reporter gene containing firefly luciferase (5xGAL4UAS-Luc3). Gal4-RORC2 LBD is constitutively active in the transfected Neuro2a cells, resulting in a robust luciferase response in the absence of stimulation. Upon treatment with an RORC2 inhibitor the transcriptional response is decreased and the magnitude of the decrease in response is dose-dependently related to the intrinsic efficacy of the inhibitor.

Specifically, the growth medium was composed by MEM EBS w/o L-glutamine, 10% (v/v) FBS, 2 mM L-glutamine and 1x non-essential aminoacid (NEAA); the seeding medium was composed by MEM EBS w/o L-glutamine, w/o phenol red, 4% (v/v) FBS, 2 mM L-glutamine, 1x NEAA, 1% Penicillin (10,000 U/mL)/Streptomycin (10,000 µg/mL); and the assay medium was composed by MEM EBS w/o L-glutamine, w/o phenol red, 4% (v/v) FBS, 2 mM L-glutamine, 1x NEAA, 1% Penicillin (10 000 U/mL)/Streptomycin (10 000 µg/mL). In addition, Neuro2A cells were cultured in growth medium in humidified chambers at 37°C and 5% CO₂ using standard tissue culture procedures.

On day one of the assay, cells were seeded and transfected. Specifically, Neuro2A cells were suspended in seeding medium and mixed with plasmids and transfection reagent which was dissolved in OptiMEM I reduced serum medium (InVitrogen), and then seeded to 384-well plates (Corning, Black, Clear bottom) in 40 µL/well containing 12,500 cells, 17,25 ng Gal4-Luc3, 5,75 ng either empty pM vector ('no receptor control' wells) or pM-Gal4RORgamma-LBD, and 0,11 µL Lipofectamine2000.

On day two of the assay, the cells were treated with compounds of the invention. Specifically, the treatment was started 20-24 hours after seeding and transfection of the cells. Compounds of the invention were serially diluted in a 384-well polypropylene plate with assay medium containing 0.5% (v/v) DMSO at 5x final assay concentration. 10 µL of the compounds (or 0.5% DMSO in assay medium for 'no compound control' wells) were transferred from the dilution plate to the 384-format cell plate such that final assay volume was 50 µL and final DMSO concentration was 0.1% (v/v), followed by incubation for 20-24 hours in humidified chambers at 37°C and 5% CO₂

On day three of the assay, luminescence was measured and the results analyzed. Specifically, 10 µL of SteadyLite Plus reagent (Perkin Elmer) was added to each well. The cell plates were incubated at room temperature for 15 min in the dark before reading of luminescence on the MicroBeta Trilux (Wallac). IC₅₀ values of the compounds tested was determined by the non-linear regression analysis of dose response curves.

References which relate to the above-referenced assay include: Stehlin-Gaon et al. Nature Structural Biology 2003, 10, 820-825; Wang et al. J Biol Chem. 2010, 285(7), 5013-5025; Kumar et al. Mol Pharmacol. 2010, 77(2), 228-36.

### Example 98

### Assay of IL-17 Production from human Th17 cells

The activity of compound of the invention can be also be determined by an IL-17 production from human Th17 cells assay. In general, this assay measures blockade of IL-17 production, the signature cytokine of T helper 17 (Th17) cells, by compounds. Purified human CD4+ T cells are stimulated with anti-CD3 + anti-CD28 and incubated with a cytokine cocktail that induce their differentiation into Th17 in the absence or presence of various concentrations of compound. After 6 days, IL-17A concentration is measured in the cell culture supernatant with an ELISA kit (MSD).

*Preparation of human CD4+ T cells.* CD4+ T cells were purified from buffy coats from healthy donors (obtained from Massachusetts General Hospital) by negative selection the following procedure: Mixing 25 mL of blood with 1 mL of Rosette Sep CD4+ T cell enrichment cocktail (StemCell Technologies) followed by application of a layer of 14 mL Ficoll Paque Plus (Amersham GE Healthcare) and subsequent centrifugation at 1200 g for 20 min at room temperature. The Ficoll layer was then harvested and washed with phosphate saline buffer containing 2% (v/v) fetal bovine serum and cells were resuspended with RPMI medium containing 10 % (v/v) fetal bovine serum and 10% (v/v) DMSO, frozen and kept in LN2 until used.

On the first day of the assay, a vial containing 10⁷ CD4+ T cells is thawed rapidly in 37°C water bath, immediately transferred into 20 mL X-Vivo 15 medium (Lonza), is spun for 6 min at 300xg, the supernatant is discarded, and the resulting pellet is re-suspended at 10⁶ cells/mL in 50 mL fresh X-Vivo 15 medium, followed by storage overnight in a tissue culture vessel in a humidified chamber at 37°C and 5% CO₂. Serial dilutions of compounds of the invention are prepared at 10x final concentration in X-Vivo15 medium containing 3% (v/v) DMSO.

On the second day of the assay, a 384-well tissue culture plate was coated with 10 µg/mL anti-hCD3 (eBioscience) at 50 µL/well. After 2 hours at 37°C, the supernatant is discarded and the coated plates are kept in a sterile tissue culture hood.

Cytokine plus anti-CD28 cocktail is prepared by mixing 25 ng/mL hIL-6 (Peprotech), 5 ng/mL hTGFbeta1 (Peprotech), 12.5 ng/mL IL-1beta (Peprotech), 25 ng/mL hIL-21, 25 ng/mL hIL-23 (R&D Systems), and 1 ug/mL anti-hCD28 (eBioscience) in X-Vivo 15 medium. The cytokine plus anti-CD28 cocktail with CD4+ cells is prepared such that the cocktail is diluted 10-fold and cell density is 0.22 x 10⁶/mL. The mixture is incubated 1 hour at 37°C.

90 µL (20,000 cells) dispensed per well in the anti-hCD3 coated plate prepared as noted above.

10uL 10x compound is added per well (final DMSO=0.3%) from the compound plate that was previously prepared, followed by 6 days of incubation in a tissue culture vessel in a humidified chamber at 37°C and 5% CO₂.

On day six of the assay, production of IL-17A in 10 uL of the supernatant is determined by sandwich ELISA using 384w hIL17 MSD plates following the manufacturer's protocol. Measurement is carried out in a Sector Imager 6000 by the same manufacturer. Signal units from the instrument are converted to pg/mL using a calibration curve with known amounts of IL-17A. IC₅₀ values of test compounds are determined by the non-linear regression analysis of dose response curves.

A reference which relates to the above-referenced assay is: Yang et al. Nature 2008, 454, 350-352.

### Example 99

### Inhibition of Superantigen-induced Th17 cytokine production

Exotoxins called "superantigens" are among the most powerful T cell activators. Superantigens bind to the cell surface of major histocompatibilty complex (MHC) molecules, without intracellular processing. They stimulate T cells via the T cell receptor, irrespective of the antigen specificities. Therefore, bacterial superantigens are able to activate a large pool of CD4+ as well as CD8+ T cells in contrast to the low T cell frequency for conventional antigens. CD4+ T cells can be classified into various subsets (Th0, Th1, Th2, Th17) based on their respective cytokine secretion profiles. Th0 cells are uncommitted naive precursor cells that primarily produce IL-2 upon stimulation. Th0 cells upon activation can differentiate into Th1, Th2, or the Th17 subset depending on the local cytokine milieu. Th1 cells mainly produce Inf-y; Th2 cells, IL-4, IL-5, and IL-13, and Th17 cells, IL-17, and IL-22. During a classical immune response, the differentiation of T helper subset occurs over days, or longer. In the superantigen in-vivo model in mice injection of superantigen triggers a rapid transcription and translation of the various cytokines (i.e. IL-2, IL-4, Inf-y, IL-17) of the different Th subsets after only 6 hours. A RORyt inhibitor given to animals prior to the superantigen stimulus would impair the Th17 cytokine profile without affecting the cytokine profile of the other Th subsets (Th0, Th1, Th2). The model uses approximately 8 week old C57BL/6, Balb/c, or C3H/HeJ mice which are dosed orally with compound 1 to 2 hours prior to superantigen injection on the day of the experiment (Day 0) based on the pharmacokinetic (PK) profile of the compound. An optional dose may be given the day before superantigen injection (Day -1) to further inhibit the response if necessary. C57BL/6 and Balb/c mice will be sensitized 1 hour prior to supernatigen injection with approximately 25 mg/mouse D-Galactosamine intraperitoneally (C3H/HeJ mice do not need to be sensitized). Based on the literature superantigen is typically given at 10 µg/mouse intraperitoneally. Mice will be sacrificed at 3 hours for RNA analysis or up to 6 hours for cytokine analysis.

A reference which relates to the above-referenced assay is: Rajagopalan, G. et. al. Physiol Genomics 2009, 37, 279.

### Example 100

### Imiquimod Assay

Commercially available 5% imiquimod (IMQ) cream (3M Pharmaceuticals) is applied to the back and right ear of each experimental mouse for two consecutive days. Control mice are treated similarly with a commercially available vehicle cream. The experimental mice are then administered with RORyt inhibitors, and the control mice with vehicle, for 4 days. The ear thickness is measured on all days by digital micrometer (Mitutoyo). Tissues, such as ears and speens, are harvested on Day 5 for RNA analysis. Ear swelling and serum measurements are also made.

References describing aspects of this assay include: Van der Fits, L. et al. J. Immunol. 2009, 182(9), 5836-45; Van Belle, A.B. et al. J Immunol. 2012, 188(1), 462-9; Cai, Y. et al. Immunity 2011, 35(4), 596-610; Fanti, P.A. et al. Int. J. Dermatol. 2006, 45(12), 1464-5; Swindell, W.R. et al. PLoS One 2011, 6(4), e18266; and Roller, A. et al. J. Immunol. 2012, 189(9), 4612-20.

## Claims

1. A compound of Formula II: 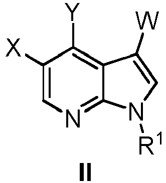 or a pharmaceutically acceptable salt, or pharmaceutically acceptable solvate thereof, wherein,
Y is hydrogen, halo, cyano, (C₁-C₃)alkyl, (C₁-C₃)hydroxyalkyl, (C₁-C₅)alkenyl, (C₁-C₃)haloalkyl, (C₁-C₃)hydroxyhaloalkyl, (C₃-C₆)cycloalkyl, (C₁-C₃)alkoxy or (C₁-C₃)haloalkoxy;
R¹ is hydrogen, (C₁-C₆)alkyl, (C₁-C₆)hydroxyalkyl or (C₁-C₆)haloalkyl;
X is 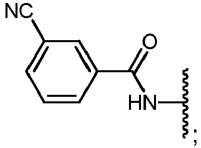 W is 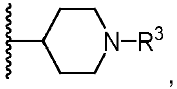 optionally substituted with one, two, three, four or five substitutents independently selected for each occurrence from the group consisting of halo, (C₁-C₆)alkyl, (C₁-C₆)hydroxyalkyl and (C₁-C₆)haloalkyl;
R³ is (C₁-C₆)alkyl, (C₃-C₈)cycloalkyl, (C₃-C₈)heterocycloalkyl, aryl, heteroaryl, -C(=O)R⁴, -C(=O)OR⁴, -C(=O)N(R⁵)₂ or -S(=O)₂R⁴, optionally substituted with one, two, three, four or five substitutents independently selected for each occurrence from the group consisting of halo, cyano, (C₁-C₄)alkyl, (C₁-C₄)haloalkyl, (C₁-C₄)cyanoalkyl, hydroxyl, (C₁-C₄)hydroxyalkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio,
(C₁-C₄)haloalkylthio, -C(=O)R, -C(=O)OR, -OC(=O)R, -C(=O)NR₂, -N(R)C(=O)R, -CH₂C (=O)R, -CH₂C(=O)OR, -CH₂OC(=O)R, -CH₂C(=O)NR₂, -CH₂N(R)C(=O)R, -S(=O)₂R, -S( =O)₂NR₂, -N(R)S(=O)₂R, -A and -CH₂A;
R⁴ is hydrogen, (C₁-C₆)alkyl, (C₃-C₈)cycloalkyl, (C₃-C₈)heterocycloalkyl, (C₃-C₈)cycloalkyl(C₁-C₆)alkyl, (C₃-C₈)heterocycloalkyl(C₁-C₆)alkyl, aryl or heteroaryl, optionally substituted with one, two, three, four or five substitutents independently selected for each occurrence from the group consisting of halo, cyano, (C₁-C₄)alkyl, (C₁-C₄)haloalkyl, (C₁-C₄)cyanoalkyl, hydroxyl, (C₁-C₄)hydroxyalkyl, (C₁-C₄)alkoxy, (C₁-C₆)alkoxy(C₁-C₆)alkyl, (C₁-C₆)haloalkoxy, -NR₂, (R₂N)(C₁-C₆)alkyl, (C₁-C₆)alkylthio, (C₁-C₆)haloalkylthio, -C(=O)R, -C(=O)OR, -OC(=O)R, -C(=O)NR₂, -N(R)C(=O)R, -CH₂C(=O) R, -CH₂C(=O)OR, -CH₂OC(=O)R, -CH₂C(=O)NR₂, -CH₂N(R)C(=O)R, -S(=O)₂R, -S(=O)₂ NR₂, -N(R)S(=O)₂R, -A and -CH₂A; and
R⁵ is independently selected for each occurrence from the group consisting of hydrogen, (C₁-C₆)alkyl, (C₃-C₈)cycloalkyl, (C₃-C₈)heterocycloalkyl, (C₃-C₈)cycloalkyl(C₁-C₆)alkyl, (C₃-C₈)heterocycloalkyl(C₁-C₆)alkyl, aryl and heteroaryl, optionally substituted with one, two, three, four or five substitutents independently selected for each occurrence from the group consisting of halo, cyano, (C₁-C₄)alkyl, (C₁-C₄)haloalkyl, (C₁-C₄)cyanoalkyl, hydroxyl, (C₁-C₄)hydroxyalkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio, (C₁-C₄)haloalkylthio, -C(=O)R, -C(=O)OR, -OC(=O)R, -C(=O)NR₂, -N(R)C(=O)R, -CH₂C(=O) R, -CH₂C(=O)OR, -CH₂OC(=O)R, -CH₂C(=O)NR₂, -CH₂N(R)C(=O)R, -S(=O)₂R, -S(=O)₂ NR₂, -N(R)S(=O)₂R, -A and -CH₂A; or where a nitrogen is substituted with two R⁵ groups may be taken together with the nitrogen atom to which they are attached to form a 4-, 5-, 6- or 7-membered saturated C₃₋₈heterocycloalkyl which, when so formed, may be optionally substituted with one, two or three substituents independently selected from the group consisting of (C₁-C₄)alkyl, halo, hydroxyl, (C₁-C₄)hydroxyalkyl, (C₁-C₄)haloalkyl and =O;
R is independently selected for each occurrence from the group consisting of hydrogen, (C₁-C₄)alkyl, (C₁-C₄)haloalkyl, (C₁-C₄)cyanoalkyl, (C₁-C₄)hydroxyalkyl, (C₃-C₈)cycloalkyl or (C₃-C₈)heterocycloalkyl; or where a nitrogen is substituted with two R groups they may be taken together with the nitrogen atom to which they are attached to form a 4-, 5-, 6- or 7-membered saturated (C₃-C₈)heterocycloalkyl which, when so formed, may be optionally substituted with one, two or three substituents independently selected from the group consisting of (C₁-C₄)alkyl, halo, hydroxyl, (C₁-C₄)hydroxyalkyl, (C₁-C₄)cyanoalkyl, (C₁-C₄)haloalkyl and =O; and
A is independently selected for each occurrence from the group consisting of (C₃-C₈)cycloalkyl, (C₃-C₈)heterocycloalkyl, aryl and heteroaryl, optionally substituted with one, two, three, four or five substitutents independently selected for each occurrence from the group consisting of halo, cyano, (C₁-C₄)alkyl, (C₁-C₄)haloalkyl, (C₁-C₄)cyanoalkyl, hydroxyl, (C₁-C₄)hydroxyalkyl, (C₁-C₄)alkoxy, (C₁-C₄)alkoxy(C₁-C₄)alkyl, (C₁-C₄)haloalkoxy, -NR₂, (R₂N)(C₁-C₄)alkyl-, (C₁-C₄)alkylthio, (C₁-C₄)haloalkylthio, -C(=O)R, -C(=O)OR, -OC(=O)R, -C(=O)NR₂, -N(R)C(=O)R, -CH₂C(=O) R, -CH₂C(=O)OR, -CH₂OC(=O)R, -CH₂C(=O)NR₂, -CH₂N(R)C(=O)R, -S(=O)₂R, -S(=O)₂ NR₂ and -N(R)S(=O)₂R.

2. The compound of claim 1, wherein R¹ is hydrogen or methyl.

3. The compound of claim 2, wherein Y is hydrogen or methyl.

4. A pharmaceutical composition comprising a compound according to any one of claims 1-3, or a pharmaceutically acceptable salt, or pharmaceutically acceptable solvate thereof, admixed with a pharmaceutically acceptable carrier, excipient or dilutant.

5. A compound or pharmaceutically acceptable salt, or pharmaceutically acceptable solvate thereof, according to any one of claims 1-3 for use in medicine.

6. A compound or pharmaceutically acceptable salt, or pharmaceutically acceptable solvate thereof according to any one of claims 1-3, or a pharmaceutical composition of claim 4, for use in treating or ameliorating an immune disorder or inflammatory disorder mediated by RORC2 and/or IL-17.

7. The compound or pharmaceutically acceptable salt or pharmaceutically acceptable solvate thereof, or pharmaceutical composition, for use according to claim 6, wherein the disorder is an inflammatory disorder.

8. The compound or pharmaceutically acceptable salt or pharmaceutically acceptable solvate thereof, or pharmaceutical composition, for use according to claim 6, wherein the disorder is an autoimmune disorder.

9. The compound or pharmaceutically acceptable salt or pharmaceutically acceptable solvate thereof, or pharmaceutical composition, for use according to claim 6, wherein the disorder is rheumatoid arthritis, psoriasis, chronic graft-versus-host disease, acute graft-versus-host disease, Crohn's disease, inflammatory bowel disease, multiple sclerosis, systemic lupus erythematosus, Celiac Sprue, idiopathic thrombocytopenic thrombotic purpura, myasthenia gravis, Sjogren's syndrome, scleroderma, ulcerative colitis, asthma, epidermal hyperplasia, cartilage inflammation, bone degradation, arthritis, juvenile arthritis, juvenile rheumatoid arthritis, pauciarticular juvenile rheumatoid arthritis, polyarticular juvenile rheumatoid arthritis, systemic onset juvenile rheumatoid arthritis, juvenile ankylosing spondylitis, juvenile enteropathic arthritis, juvenile Reter's Syndrome, SEA Syndrome, juvenile dermatomyositis, juvenile psoriatic arthritis, juvenile scleroderma, juvenile systemic lupus erythematosus, juvenile vasculitis, pauciarticular rheumatoid arthritis, polyarticular rheumatoid arthritis, systemic onset rheumatoid arthritis, ankylosing spondylitis, enteropathic arthritis, reactive arthritis, Reter's Syndrome, dermatomyositis, psoriatic arthritis, vasculitis, myolitis, polymyolitis, dermatomyolitis, osteoarthritis, polyarteritis nodossa, Wegener's granulomatosis, arteritis, polymyalgia rheumatica, sarcoidosis, sclerosis, primary biliary sclerosis, sclerosing cholangitis, dermatitis, atopic dermatitis, atherosclerosis, Still's disease, chronic obstructive pulmonary disease, Guillain-Barre disease, Type I diabetes mellitus, Graves' disease, Addison's disease, Raynaud's phenomenon, autoimmune hepatitis, psoriatic epidermal hyperplasia, plaque psoriasis, guttate psoriasis, inverse psoriasis, pustular psoriasis, erythrodermic psoriasis, an immune disorder associated with or arising from activity of pathogenic lymphocytes, plaque psoriasis, guttate psoriasis, inverse psoriasis, pustular psoriasis, erythrodermic psoriasis, noninfectious uveitis, Behcet's disease or Vogt-Koyanagi-Harada syndrome.

10. The compound or pharmaceutically acceptable salt or pharmaceutically acceptable solvate thereof, or pharmaceutical composition, for use according to claim 9, wherein the disorder is psoriasis.

11. A compound or pharmaceutically acceptable salt, or pharmaceutically acceptable solvate thereof, according to any one of claims 1-3 for use in combination with another pharmaceutical agent.

## Patentansprüche

1. Verbindung der Formel II: 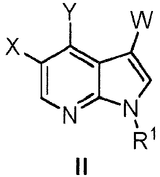 oder ein pharmazeutisch annehmbares Salz oder pharmazeutisch annehmbares Solvat davon, worin
Y Wasserstoff, Halogen, Cyano, (C₁-C₃)Alkyl, (C₁-C₃)Hydroxyalkyl, (C₁-C₅)Alkenyl, (C₁-C₃)Halogenalkyl, (C₁-C₃)Hydroxyhalogenalkyl, (C₃-C₆)Cycloalkyl, (C₁-C₃)Alkoxy oder (C₁-C₃)Halogenalkoxy ist;
R¹ Wasserstoff, (C₁-C₆)Alkyl, (C₁-C₆)Hydroxyalkyl oder (C₁-C₆)Halogenalkyl ist;
X 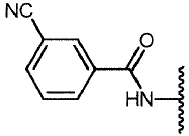 ist;
W 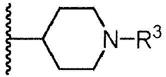 ist, das gegebenenfalls mit eins, zwei, drei, vier oder fünf Substituenten substituiert ist, die für jedes Vorkommen unabhängig ausgewählt sind aus der Gruppe, bestehend aus Halogen, (C₁-C₆)Alkyl, (C₁-C₆)Hydroxyalkyl und (C₁-C₆)Halogenalkyl;
R³ (C₁-C₆)Alkyl, (C₃-C₈)Cycloalkyl, (C₃-C₈)Heterocycloalkyl, Aryl, Heteroaryl, -C(=O)R⁴, -C(=O)OR⁴,-C(=O)N(R⁵)₂ oder -S(=O)₂R⁴, gegebenenfalls substituiert mit eins, zwei, drei, vier oder fünf Substituenten, unabhängig ausgewählt für jedes Vorkommen aus der Gruppe, bestehend aus Halogen, Cyano, (C₁-C₄)Alkyl, (C₁-C₄)Halogenalkyl, (C₁-C₄)Cyanoalkyl, Hydroxyl, (C₁-C₄)Hydroxyalkyl, (C₁-C₄)Alkoxy, (C₁-C₄)Alkoxy (C₁-C₄)alkyl, (C₁-C₄)Halogenalkoxy, -NR₂, (R₂N) (C₁-C₄)Alkyl-, (C₁-C₄)Alkylthio, (C₁-C₄)Halogenalkylthio, -C(=O)R, -C(=O)OR, -OC(=O)R, -C(=O)NR₂, -N(R)C(=O)R,-CH₂C(=O)R, -CH₂C(=O)OR, -CH₂OC (=O)R, -CH₂C(=O)NR₂,-CH₂N(R)C(=O)R, -S(=O)₂R, -S(=O)₂NR₂, -N(R)S(=O)₂R, -A und-CH₂A, ist;
R⁴ Wasserstoff, (C₁-C₆)Alkyl, (C₃-C₈)Cycloalkyl, (C₃-C₈)Heterocycloalkyl, (C₃-C₈)Cycloalkyl (C₁-C₆)alkyl, (C₃-C₈)Heterocycloalkyl(C₁-C₆)alkyl, Aryl oder Heteroaryl, gegebenenfalls substituiert mit eins, zwei, drei, vier oder fünf Substituenten, unabhängig ausgewählt für jedes Vorkommen aus der Gruppe, bestehend aus Halogen, Cyano, (C₁-C₄)Alkyl, (C₁-C₄)Halogenalkyl, (C₁-C₄)Cyanoalkyl, Hydroxyl, (C₁-C₄)Hydroxyalkyl, (C₁-C₄)Alkoxy, (C₁-C₆)Alkoxy(C₁-C₆)alkyl, (C₁-C₆)Halogenalkoxy, -NR₂, (R₂N)(C₁-C₆)Alkyl, (C₁-C₆)Alkylthio, (C₁-C₆)Halogenalkylthio, -C(=O)R, -C(=O)OR, -OC(=O)R,-C(=O)NR₂, -N(R)C(=O)R, -CH₂C(=O)R, -CH₂C(=O)OR,-CH₂OC(=O)R, -CH₂C(=O)NR₂, -CH₂N(R)C(=O)R,-S(=O)₂R, -S(=O)₂NR₂, -N(R)S(=O)₂R, -A und-CH₂A, ist; und
R⁵ unabhängig ausgewählt ist für jedes Vorkommen aus der Gruppe, bestehend aus Wasserstoff, (C₁-C₆)Alkyl, (C₃-C₈)Cycloalkyl, (C₃-C₈)Heterocycloalkyl, (C₃-C₈)Cycloalkyl (C₁-C₆)alkyl, (C₃-C₈)Heterocycloalkyl (C₁-C₆)alkyl, Aryl und Heteroaryl, gegebenenfalls substituiert mit eins, zwei, drei, vier oder fünf Substituenten, unabhängig ausgewählt für jedes Vorkommen aus der Gruppe, bestehend aus Halogen, Cyano, (C₁-C₄)Alkyl, (C₁-C₄)Halogenalkyl, (C₁-C₄)Cyanoalkyl, Hydroxyl, (C₁-C₄)Hydroxyalkyl, (C₁-C₄)Alkoxy, (C₁-C₄)Alkoxy (C₁-C₄)alkyl, (C₁-C₄)Halogenalkoxy, -NR₂, (R₂N) (C₁-C₄)Alkyl-, (C₁-C₄)Alkylthio, (C₁-C₄)Halogenalkylthio, -C(=O)R, -C(=O)OR,-OC(=O)R, -C(=O)NR₂, -N(R)C(=O)R, -CH₂C(=O)R, -CH₂C(=O)OR,-CH₂OC(=O)R, -CH₂C(=O)NR₂, -CH₂N(R)C(=O)R,-S(=O)₂R, -S(=O)₂NR₂, -N(R)S(=O)₂R, -A und-CH₂A, oder worin ein Stickstoff mit zwei R⁵-Gruppen substituiert ist und diese zusammengenommen mit dem Stickstoffatom, an das sie gebunden sind, ein 4-, 5-, 6- oder 7-gliedriges gesättigtes C₃₋₈-Heterocycloalkyl bilden, welches, wenn es so gebildet ist, gegebenenfalls mit eins, zwei oder drei Substituenten, unabhängig ausgewählt aus der Gruppe, bestehend aus (C₁-C₄)Alkyl, Halogen, Hydroxyl, (C₁-C₄)Hydroxyalkyl, (C₁-C₄)Halogenalkyl und =O, substituiert sein kann;
R unabhängig ausgewählt ist für jedes Vorkommen aus der Gruppe, bestehend aus Wasserstoff, (C₁-C₄)Alkyl, (C₁-C₄)Halogenalkyl, (C₁-C₄)Cyanoalkyl, (C₁-C₄)Hydroxyalkyl, (C₃-C₈)Cycloalkyl oder (C₃-C₈)Heterocycloalkyl, oder worin ein Stickstoff mit zwei R-Gruppen substituiert ist, welche mit dem Stickstoffatom, an das sie gebunden sind, zusammengenommen werden können, um ein 4-, 5-, 6- oder 7-gliedriges gesättigtes (C₃-C₈)Heterocycloalkyl zu bilden, welches, wenn es so gebildet ist, gegebenenfalls mit eins, zwei oder drei Substituenten, unabhängig ausgewählt aus der Gruppe, bestehend aus (C₁-C₄)Alkyl, Halogen, Hydroxyl, (C₁-C₄)Hydroxyalkyl, (C₁-C₄)Cyanoalkyl, (C₁-C₄)Halogenalkyl und =O, substituiert sein kann; und
A unabhängig ausgewählt ist für jedes Vorkommen aus der Gruppe, bestehend aus (C₃-C₈)Cycloalkyl, (C₃-C₈)Heterocycloalkyl, Aryl und Heteroaryl, gegebenenfalls substituiert mit eins, zwei, drei, vier oder fünf Substituenten, unabhängig ausgewählt für jedes Vorkommen aus der Gruppe, bestehend aus Halogen, Cyano, (C₁-C₄)Alkyl, (C₁-C₄)Halogenalkyl, (C₁-C₄)Cyanoalkyl, Hydroxyl, (C₁-C₄)Hydroxyalkyl, (C₁-C₄)Alkoxy, (C₁-C₄)Alkoxy (C₁-C₄)alkyl, (C₁-C₄)Halogenalkoxy,-NR₂, (R₂N) (C₁-C₄)Alkyl-, (C₁-C₄)Alkylthio, (C₁-C₄)Halogenalkylthio, -C(=O)R, -C(=O)OR, -OC(=O)R, -C(=O)NR₂, -N(R)C(=O)R, -CH₂C(=O)R, -CH₂C(=O)OR, -CH₂OC (=O)R,-CH₂C(=O)NR₂, -CH₂N(R)C(=O)R, -S(=O)₂R, -S(=O)₂NR₂ und -N(R)S(=O)₂R.

2. Verbindung gemäß Anspruch 1, wobei R¹ Wasserstoff oder Methyl ist.

3. Verbindung gemäß Anspruch 2, wobei Y Wasserstoff oder Methyl ist.

4. Pharmazeutische Zusammensetzung, umfassend eine Verbindung gemäß einem der Ansprüche 1 bis 3 oder ein pharmazeutisch annehmbares Salz oder pharmazeutisch annehmbares Solvat davon, vermischt mit einem pharmazeutisch annehmbaren Träger, Exzipiens oder Verdünnungsmittel.

5. Verbindung oder pharmazeutisch annehmbares Salz oder pharmazeutisch annehmbares Solvat davon gemäß einem der Ansprüche 1 bis 3 zur Verwendung in Medizin.

6. Verbindung oder pharmazeutisch annehmbares Salz oder pharmazeutisch annehmbares Solvat davon gemäß einem der Ansprüche 1 bis 3 oder pharmazeutische Zusammensetzung gemäß Anspruch 4 zur Verwendung bei der Behandlung oder Besserung einer Immunstörung oder Entzündungsstörung, vermittelt durch RORC2 und/oder IL-17.

7. Verbindung oder pharmazeutisch annehmbares Salz oder pharmazeutisch annehmbares Solvat davon oder pharmazeutische Zusammensetzung zur Verwendung gemäß Anspruch 6, wobei die Störung eine Entzündungsstörung ist.

8. Verbindung oder pharmazeutisch annehmbares Salz oder pharmazeutisch annehmbares Solvat davon oder pharmazeutische Zusammensetzung zur Verwendung gemäß Anspruch 6, wobei die Störung eine Autoimmunstörung ist.

9. Verbindung oder pharmazeutisch annehmbares Salz oder pharmazeutisch annehmbares Solvat davon oder pharmazeutische Zusammensetzung zur Verwendung gemäß Anspruch 6, wobei die Störung rheumatoide Arthritis, Psoriasis, chronische Graft-versus-Host-Krankheit, akute Graft-versus-Host-Krankheit, Crohn'sche Krankheit, inflammatorische Darmkrankheit, Multiple Sklerose, systemischer Lupus erythematodes, Zöliakie, essentielle Thrombozytopenie, Myasthenia gravis, Sjogren-Syndrom, Sklerodermie, Colitis ulcerosa, Asthma, epidermale Hyperplasie, Knorpelentzündung, Knochenabbau, Arthritis, juvenile Arthritis, juvenile rheumatoide Arthritis, nur wenige Gelenke betreffende juvenile rheumatoide Arthritis, polyartikuläre juvenile rheumatoide Arthritis, systemische rheumatoide Arthritis mit Einsetzen in der Jugend, juvenile Spondyloarthritis ancylopoetica, juvenile enteropathische Arthritis, juveniles Reiter-Sydrom, SEA-Syndrom, juvenile Dermatomyositis, juvenile psoriatische Arthritis, juvenile Sklerodermie, juveniler systemischer Lupus erythematodes, juvenile Vaskulitis, wenige Gelenke betreffende rheumatoide Arthritis, viele Gelenke betreffende rheumatoide Arthritis, rheumatoide Arthritis mit systemischem Beginn, Spondylarthritis ancylopoetica, enteropathische Arthritis, reaktive Arthritis, Reiter-Syndrom, Dermatomyositis, psoriatische Arthritis, Vaskulitis, Myolitis, Polymyolitis, Dermatomyolitis, Osteoarthritis, Polyarteritis nodosa, Wegener-Granulomatose, Arteritis, Polymyalgia rheumatica, Sarkoidose, Sklerose, primäre biliäre Sklerose, sklerosierende Cholangitis, Dermatitis, atopische Dermatitis, Atherosklerose, Still-Erkrankung, chronischobstruktive Lungenerkrankung, Guillain-Barré-Erkrankung, Typ-I-Diabetes mellitus, Morbus Basedow, Morbus Addison, Raynaud-Phänomen, autoimmune Hepatitis, psoriatische epidermale Hyperplasie, Psoriasis-Plaque, Psoriasis guttata, Psoriasis im Bereich der Körperfalten, pustulöse Psoriasis, erythroderme Psoriasis, mit der Aktivität von pathogenen Lymphozyten assoziierte oder dadurch hervorgerufene Immunstörung, Psorisasis-Plaque, Psoriasis guttata, Psoriasis im Bereich der Körperfalten, pustulöse Psoriasis, erythroderme Psoriasis, nichtinfektiöse Uveitis, Behçet-Erkrankung oder Vogt-Koyanagi-Harada-Syndrom ist.

10. Verbindung oder pharmazeutisch annehmbares Salz oder pharmazeutisch annehmbares Solvat davon oder pharmazeutische Zusammensetzung zur Verwendung gemäß Anspruch 9, wobei die Störung Psoriasis ist.

11. Verbindung oder pharmazeutisch annehmbares Salz oder pharmazeutisch annehmbares Solvat davon gemäß einem der Ansprüche 1 bis 3 zur Verwendung in Kombination mit einem anderen pharmazeutischen Mittel.

## Revendications

1. Composé selon la formule II : 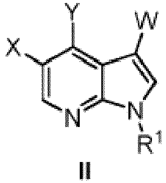 ou son sel pharmaceutiquement acceptable, ou son solvate pharmaceutiquement acceptable, dans lequel,
Y est de l'hydrogène, un groupe halo, cyano, alkyle en C₁-C₃, hydroxyalkyle en C₁-C₃, alcényle en C₁-C₅, haloalkyle en C₁-C₃, hydroxyhaloalkyle en C₁-C₃, cycloalkyle en C₃-C₆, alkoxy en C₁-C₃ ou haloalkoxy en C₁-C₃ ;
R¹ est de l'hydrogène, un groupe alkyle en C₁-C₆, hydroxyalkyle en C₁-C₆ ou haloalkyle en C₁-C₆ ;
X est 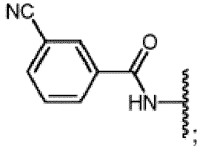 W est 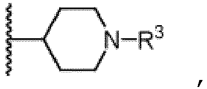 facultativement substitué par un, deux, trois, quatre ou cinq substituants indépendamment sélectionnés pour chaque occurrence parmi le groupe constitué par un groupe halo, alkyle en C₁-C₆, hydroxyalkyle en C₁-C₆ et haloalkyle en C₁-C₆ ;
R³ est un groupe alkyle en C₁-C₆, cycloalkyle en C₃-C₈, hétérocycloalkyle en C₃-C₈, aryle, hétéroaryle, -C(=O)R⁴-C(=O)OR⁴, -C(=O)N(R⁵)₂ ou -S(=O)₂R⁴, facultativement substitués par un, deux, trois, quatre ou cinq substituants indépendamment sélectionnés pour chaque occurrence parmi le groupe constitué par un groupe halo, cyano, alkyle en C₁-C₄, haloalkyle en C₁-C₄, cyanoalkyle en C₁-C₄, hydroxyle, hydroxyalkyle en C₁-C₄, alkoxy en C₁-C₄, alkoxy en C₁-C₄-alkyle en C₁-C₄, haloalkoxy en C₁-C₄, -NR₂, (R₂N)-alkyle en C₁-C₄, alkylthio en C₁-C₄, haloalkylthio en C₁-C₄, -C(=O)R,-C(=O)OR, -OC(=O)R, -C(=O)NR₂, -N(R)C(=O)R, -CH₂C(=O)R,-CH₂C(=O)OR, -CH₂OC(=O)R, -CH₂C(=O)NR₂, -CH₂N(R)C(=O)R,-S(=O)₂R, -S(=O)₂NR₂, -N(R)S(=O)₂R, -A et -CH₂A;
R⁴ est de l'hydrogène, un groupe alkyle en C₁-C₆, cycloalkyle en C₃-C₈, hétérocycloalkyle en C₃-C₈, cycloalkyle en C₃-C₈-alkyle en C₁-C₆, hétérocycloalkyle en C₃-C₈-alkyle en C₁-C₆, aryle ou hétéroaryle, facultativement substitués par un, deux, trois, quatre ou cinq substituants indépendamment sélectionnés pour chaque occurrence parmi le groupe constitué par un groupe halo, cyano, alkyle en C₁-C₄, haloalkyle en C₁-C₄, cyanoalkyle en C₁-C₄, hydroxyle, hydroxyalkyle en C₁-C₄, alkoxy en C₁-C₄, alkoxy en C₁-C₆-alkyle en C₁-C₆, haloalkoxy en C₁-C₆, -NR₂, (R₂N) -alkyle en C₁-C₆, alkylthio en C₁-C₆, haloalkylthio en C₁-C₆, -C(=O)R,-C(=O)OR, -OC(=O)R, -C(=O)NR₂, -N(R)C(=O)R, -CH₂C(=O)R,-CH₂C(=O)OR, -CH₂OC(=O)R, -CH₂C(=O)NR₂, -CH₂N(R)C(=O)R,-S(=O)₂R, -S(=O)₂NR₂, -N(R)S(=O)₂R, -A et -CH₂A; et
R⁵ est indépendamment sélectionné pour chaque occurrence parmi le groupe constitué par l'hydrogène, un groupe alkyle en C₁-C₆, cycloalkyle en C₃-C₈, hétérocycloalkyle en C₃-C₈, cycloalkyle en C₃-C₈-alkyle en C₁-C₆, hétérocycloalkyle en C₃-C₈-alkyle en C₁-C₆, aryle et hétéroaryle, facultativement substitués par un, deux, trois, quatre ou cinq substituants indépendamment sélectionnés pour chaque occurrence parmi le groupe constitué par un groupe halo, cyano, alkyle en C₁-C₄, haloalkyle en C₁-C₄, cyanoalkyle en C₁-C₄, hydroxyle, hydroxyalkyle en C₁-C₄, alkoxy en C₁-C₄, alkoxy en C₁-C₄-alkyle en C₁-C₄, haloalkoxy en C₁-C₄, -NR₂, (R₂N) -alkyle en C₁-C₄, alkylthio en C₁-C₄, haloalkylthio en C₁-C₄, -C(=O)R,-C(=O)OR, -OC(=O)R, -C(=O)NR₂, -N(R)C(=O)R, -CH₂C(=O)R,-CH₂C(=O)OR, -CH₂OC(=O)R, -CH₂C(=O)NR₂, -CH₂N(R)C(=O)R,-S(=O)₂R, -S(=O)₂NR₂, -N(R)S(=O)₂R, -A et -CH₂A; ou lorsqu'un azote est substitué par deux groupes R⁵, ils peuvent être réunis avec l'atome d'azote auxquels ils sont fixés pour former un hétérocycloalkyle en C₃-C₈ saturé à 4, 5, 6 ou 7 chaînons qui, lorsqu'il est ainsi formé, peut être facultativement substitué par un, deux ou trois substituants indépendamment sélectionnés parmi le groupe constitué par un groupe alkyle en C₁-C₄, halo, hydroxyle, hydroxyalkyle en C₁-C₄, haloalkyle en C₁-C₄ et =O;
R est indépendamment sélectionné pour chaque occurrence parmi le groupe constitué par l'hydrogène, un groupe alkyle en C₁-C₄, haloalkyle en C₁-C₄, cyanoalkyle en C₁-C₄, hydroxyalkyle en C₁-C₄, cycloalkyle en C₃-C₈ ou hétérocycloalkyle en C₃-C₈ ; ou lorsqu'un azote est substitué par deux groupes R, ils peuvent être réunis avec l'atome d'azote auxquels ils sont fixés pour former un hétérocycloalkyle en C₃-C₈ saturé à 4, 5, 6 ou 7 chaînons qui, lorsqu'il est ainsi formé, peut être facultativement substitué par un, deux ou trois substituants indépendamment sélectionnés parmi le groupe constitué par un groupe alkyle en C₁-C₄, halo, hydroxyle, hydroxyalkyle en C₁-C₄, cyanoalkyle en C₁-C₄, haloalkyle en C₁-C₄ et =O; et
A est indépendamment sélectionné pour chaque occurrence parmi le groupe constitué par un groupe cycloalkyle en C₃-C₈, hétérocycloalkyle en C₃-C₈, aryle et hétéroaryle, facultativement substitués par un, deux, trois, quatre ou cinq substituants indépendamment sélectionnés pour chaque occurrence parmi le groupe constitué par un groupe halo, cyano, alkyle en C₁-C₄, haloalkyle en C₁-C₄, cyanoalkyle en C₁-C₄, hydroxyle, hydroxyalkyle en C₁-C₄, alkoxy en C₁-C₄, alkoxy en C₁-C₄-alkyle en C₁-C₄, haloalkoxy en C₁-C₄, -NR₂, (R₂N) -alkyle en C₁-C₄, alkylthio en C₁-C₄, haloalkylthio en C₁-C₄, -C(=O)R,-C(=O)OR, -OC(=O)R, -C(=O)NR₂, -N(R)C(=O)R, -CH₂C(=O)R,-CH₂C(=O)OR, -CH₂OC(=O)R, -CH₂C(=O)NR₂, -CH₂N(R)C(=O)R,-S(=O)₂R, -S(=O)₂NR₂ et -N(R)S(=O)₂R.

2. Composé selon la revendication 1, dans lequel R¹ est de l'hydrogène ou un méthyle.

3. Composé selon la revendication 2, dans lequel Y est de l'hydrogène ou un méthyle.

4. Composition pharmaceutique comprenant un composé défini selon l'une quelconque des revendications 1 à 3, ou son sel pharmaceutiquement acceptable, ou son solvate pharmaceutiquement acceptable, mélangé avec un support, un excipient ou un diluant pharmaceutiquement acceptable.

5. Composé ou son sel pharmaceutiquement acceptable, ou son solvate pharmaceutiquement acceptable, défini selon l'une quelconque des revendications 1 à 3, destiné à être utilisé en médecine.

6. Composé ou son sel pharmaceutiquement acceptable, ou son solvate pharmaceutiquement acceptable, défini selon l'une quelconque des revendications 1 à 3, ou composition pharmaceutique selon la revendication 4, destiné(e) à être utilisé(e) pour le traitement ou l'amélioration d'un trouble immunitaire ou d'un trouble inflammatoire médié par RORC2 et/ou IL-17.

7. Composé ou son sel pharmaceutiquement acceptable ou son solvate pharmaceutiquement acceptable, ou composition pharmaceutique, destiné(e) à être utilisé(e) selon la revendication 6, dans lequel le trouble est un trouble inflammatoire.

8. Composé ou son sel pharmaceutiquement acceptable ou son solvate pharmaceutiquement acceptable, ou composition pharmaceutique, destiné(e) à être utilisé(e) selon la revendication 6, dans lequel le trouble est un trouble auto-immun.

9. Composé ou son sel pharmaceutiquement acceptable ou son solvate pharmaceutiquement acceptable, ou composition pharmaceutique, destiné(e) à être utilisé(e) selon la revendication 6, dans lequel le trouble est l'arthrite rhumatoïde, le psoriasis, la réaction de greffe contre hôte chronique, la réaction de greffe contre hôte grave, la maladie de Crohn, la maladie intestinale inflammatoire, la sclérose en plaques, le lupus érythémateux disséminé, la maladie coeliaque, le purpura thrombotique thrombocytopénique idiopathique, la myasthénie grave, le syndrome de Sjögren, la sclérodermie, la colite ulcéreuse, l'asthme, l'hyperplasie épidermique, l'inflammation du cartilage, la dégradation osseuse, l'arthrite, l'arthrite juvénile, l'arthrite rhumatoïde juvénile, l'oligo-arthrite rhumatoïde juvénile, la polyarthrite rhumatoïde juvénile, l'arthrite rhumatoïde juvénile avec début systémique, la spondylite ankylosante juvénile, l'arthrite entéropathique juvénile, le syndrome de Reiter juvénile, le syndrome SEA, la dermatomyosite juvénile, l'arthrite psoriasique juvénile, la sclérodermie juvénile, le lupus érythémateux disséminé juvénile, la vascularite juvénile, l'oligo-arthrite rhumatoïde, la polyarthrite rhumatoïde, l'arthrite rhumatoïde avec début systémique, la spondylite ankylosante, l'arthrite entéropathique, l'arthrite réactive, le syndrome de Reiter, la dermatomyosite, l'arthrite psoriasique, la vascularite, la myolite, la polymyolite, la dermatomyolite, l'arthrose, la polyartérite noueuse, la granulomatose de Wegener, l'artérite, la pseudo-polyarthrite rhyzomélique, la sarcoïdose, la sclérose, la sclérose biliaire primaire, la cholangite sclérosante, la dermatite, la dermatite atopique, l'athérosclérose, la maladie de Still, la bronchopneumopathie chronique obstructive, la maladie de Guillain-Barré, le diabète sucré de type I, la maladie de Graves, la maladie d'Addison, le phénomène de Raynaud, l'hépatite autoimmune, l'hyperplasie épidermique psoriasique, le psoriasis en plaques, le psoriasis en gouttes, le psoriasis inverse, le psoriasis pustuleux, le psoriasis érythrodermique, un trouble immunitaire associé avec ou issu de l'activité de lymphocytes pathogènes, le psoriasis en plaques, le psoriasis en gouttes, le psoriasis inverse, le psoriasis pustuleux, le psoriasis érythrodermique, l'uvéite non infectieuse, la maladie de Behçet ou le syndrome de Vogt-Koyanagi-Harada.

10. Composé ou son sel pharmaceutiquement acceptable ou son solvate pharmaceutiquement acceptable, ou composition pharmaceutique, destiné(e) à être utilisé(e) selon la revendication 9, dans lequel le trouble est le psoriasis.

11. Composé ou son sel pharmaceutiquement acceptable, ou son solvate pharmaceutiquement acceptable, défini selon l'une quelconque des revendications 1 à 3, destiné à être utilisé en combinaison avec un autre agent pharmaceutique.
